(19) Europäisches Patentamt
European Patent Office
Office européen des brevets

(11) Veröffentlichungsnummer: **0 097 822**
**B1**

# (12) EUROPÄISCHE PATENTSCHRIFT

(45) Veröffentlichungstag der Patentschrift: **15.07.87**

(21) Anmeldenummer: **83105226.1**

(22) Anmeldetag: **26.05.83**

(51) Int. Cl.⁴: **C 07 D 317/28**, C 07 D 405/06, C 07 D 413/06, A 01 N 43/48, A 01 N 43/64, A 01 N 43/84, A 01 N 43/28, A 01 N 43/56

(54) **Aminomethyldioxolane als Schädlingsbekämpfungsmittel.**

(30) Priorität: **04.06.82 DE 3221138**
**19.02.83 DE 3305769**

(43) Veröffentlichungstag der Anmeldung: **11.01.84 Patentblatt 84/2**

(45) Bekanntmachung des Hinweises auf die Patenterteilung: **15.07.87 Patentblatt 87/29**

(84) Benannte Vertragsstaaten: **AT BE CH DE FR GB IT LI NL SE**

(56) Entgegenhaltungen:
**EP - A - 0 044 276**
**DE - A - 2 200 766**
**FR - A - 2 128 713**
**FR - A - 2 163 322**
**GB - A - 601 612**
**GB - A - 2 095 236**
**US - A - 2 606 909**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Krämer, Wolfgang, Dr., Am Eckbusch 39/45, D-5600 Wuppertal 1 (DE)**
Erfinder: **Reiser, Wolf, Dr., Kiebitzweg 12a, D-5600 Wuppertal 1 (DE)**
Erfinder: **Berg, Dieter, Dr., Gellertweg 27, D-5600 Wuppertal 1 (DE)**
Erfinder: **Brandes, Wilhelm, Dr., Eichendorffstrasse 3, D-5653 Leichlingen (DE)**
Erfinder: **Reinecke, Paul, Dr., Lessingstrasse 11, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft die Verwendung von teilweise bekannten substituierten Aminoketalen als Schädlingsbekämpfungsmittel.

Es ist bereits bekannt, dass bestimmte Aminoketale, wie z.B. 2-Methyl-2-(3,4,5-trimethoxyphenyl)-4-(1-pyrrolidinylmethyl)-1,3-dioxolan, 2-Cyclohexyl-2-phenyl-4-(1-morpholinylmethyl)-1,3-dioxolan, 2-Phenyl-2-chlormethyl-4-(1-morpholinylmethyl)-1,3-dioxolan und 2-Methyl-2-[1-(4-methyl-buten(3)yl)]-4-(1-piperidinylmethyl)-1,3-dioxolan, auf dem Pharmasektor, z.B. als Diuretika oder Hypotensiva, eine Wirkung aufweisen [vergleiche z.B. FR-Patentschrift 2 163 322; Japan. Patentanmeldung 52-33 608; Belgische Patentschrift 776 421; Niederld. Patentschrift 6 609 579; US-Patentschrift 3 337 408 und SU-Patentschrift 722 910; ausserdem A.R. Patel u.a. «J. Pharm. Sci. 52 (1963), Nr. 6, S. 588–592; J. Wolinski u.a. «Acta Pol. Pharm. 1980, 37 (1), S. 15–24 (Pol.), 1978, 35 (6), S. 621–627 (Pol.), 1978, 35 (3), S. 265–272 (Pol.), 1977, 34 (2), S. 143–147 (Pol.)]

Weiterhin sind Aminoketale, wie z.B. 2,2-Dimethyl-4-(1-morpholinylmethyl)-1,3-dioxolan, als Zwischenprodukte oder Ausgangsprodukte zur Herstellung anderer pharma-aktiver Verbindungen bekannt [vergleiche z.B. N.D. Harris «J. Org. Chem. 28 (1963), Nr. 3, S. 745–748 und Karpysow «Bio-Org. Khim» 1979, 5 (2), S. 238–241 (Russ.)]. Bei keinem der bekannten Aminoketale ist eine Wirkung auf dem Pflanzenschutzgebiet bekannt.

Weiterhin ist bereits bekannt geworden, dass Aminopropiophenon-Derivate, wie zum Beispiel (4-tert.-Butylphenyl)-(1-dimethylamino-prop-2-yl)-keton (vgl. DE-OS-3 019 497), und auch organische Schwefelverbindungen, wie z.B. Zink-ethylen-1,2-bis-(dithiocarbamat) (vgl. R. Wegler, «Chemie der Pflanzenschutz- und Schädlingsbekämpfungsmittel», Springer Verlag Berlin 1970, Band 2, Seite 65f), fungizide Eigenschaften aufweisen. Die Wirkung dieser Verbindungen ist jedoch insbesondere bei niedrigen Aufwandmengen und -konzentrationen in einigen Anwendungsbereichen nicht immer voll befriedigend.

Es wurde gefunden, dass die teilweise bekannten substituierten Aminoketale der Formel (I)

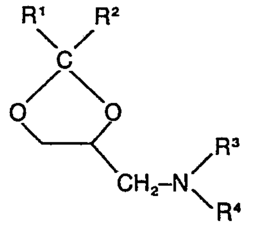

(I)

in welcher

$R^1$ für geradkettiges oder verzweigtes Alkyl mit 1 bis 18 Kohlenstoffatomen; für substituiertes Alkyl, wie geradkettiges oder verzweigtes Halogenalkyl mit 1 bis 12 Kohlenstoffatomen und 1 bis 5 Halogenatomen, geradkettiges oder verzweigtes Cyanalkyl mit 1 bis 4 Kohlenstoffatomen im Alkylteil, gegebenenfalls ein- oder mehrfach, gleich oder verschieden substituiertes Arylalkyl, Aryloxyalkyl, Aralkyloxyalkyl, Arylthioalkyl, Arylsulfinylalkyl, Arylsulfonylalkyl mit jeweils 1 bis 6 Kohlenstoffatomen in jedem Alkylteil und 6 bis 10 Kohlenstoffatomen im Arylteil, wobei als Arylsubstituenten jeweils in Frage kommen:

Halogen, Cyano, Nitro; Alkyl, Alkoxy, Alkylthio mit jeweils 1 bis 4 Kohlenstoffatomen; Halogenalkyl, Halogenalkoxy und Halogenalkylthio mit jeweils 1 oder 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy;

und für Cycloalkylalkyl mit 1 bis 4 Kohlenstoffatomen im Alkylteil und 3 bis 7 Kohlenstoffatomen im gegebenenfalls ein- oder mehrfach durch Alkyl mit 1 bis 4 Kohlenstoffatomen substituierten Cycloalkylteil, ferner für geradkettiges oder verzweigtes Alkenyl oder Alkinyl mit jeweils 2 bis 6 Kohlenstoffatomen, für gegebenenfalls einfach oder mehrfach, gleich oder verschieden durch Alkyl mit 1 bis 4 Kohlenstoffatomen substituiertes Cycloalkyl mit 3 bis 7 Kohlenstoffatomen, oder für jeweils gegebenenfalls einfach oder mehrfach, gleich oder verschieden substituiertes Aryl mit 6 bis 10 Kohlenstoffatomen, sowie Arylalkenyl mit 2 bis 6 Kohlenstoffatomen im Alkenylteil und 6 bis 10 Kohlenstoffatomen im Arylteil steht, wobei als Arylsubstituenten jeweils in Frage kommen:
Halogen, Cyano, Nitro; Alkyl, Alkoxy, Alkylthio mit jeweils 1 bis 4 Kohlenstoffatomen; Halogenalkyl, Halogenalkoxy, Halogenalkylthio mit jeweils 1 bis 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy;

$R^2$ dieselbe Bedeutung hat wie $R^1$, von diesem gleich oder verschieden sein kann, und ausserdem für Wasserstoff steht,

$R^3$ für geradkettiges oder verzweigtes Alkyl mit 1 bis 12 Kohlenstoffatomen, für geradkettiges oder verzweigtes Alkenyl mit 3 bis 12 Kohlenstoffatomen, für geradkettiges oder verzweigtes Alkinyl mit 3 bis 7 Kohlenstoffatomen, für gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen sowie für jeweils gegebenenfalls einfach oder mehrfach, gleich oder verschieden substituiertes Aryl mit 6 bis 10 Kohlenstoffatomen oder Aralkyl mit 1 oder 2 Kohlenstoffatomen im Arylteil steht, wobei als Arylsubstituenten jeweils in Frage kommen:

Halogen, Alkyl mit 1 bis 4 Kohlenstoffatomen sowie Halogenalkyl mit 1 oder 2 Kohlenstoffatomen und 1 bis 5 Halogenatomen;

$R^4$ für geradkettiges oder verzweigtes Alkyl mit 1 bis 12 Kohlenstoffatomen, für geradkettiges oder verzweigtes Alkenyl mit 3 bis 12 Kohlenstoffatomen, für geradkettiges oder verzweigtes Alkinyl

mit 3 bis 7 Kohlenstoffatomen, für gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen sowie für jeweils gegebenenfalls einfach oder mehrfach, gleich oder verschieden substituiertes Aryl mit 6 bis 10 Kohlenstoffatomen oder Aralkyl mit 1 bis 2 Kohlenstoffatomen im Alkylteil und 6 bis 10 Kohlenstoffatomen im Arylteil steht, wobei als Arylsubstituenten jeweils in Frage kommen;
Halogen, Alkyl mit 1 bis 4 Kohlenstoffatomen, sowie Halogenalkyl mit 1 oder 2 Kohlenstoffatomen und 1 bis 5 Halogenatomen; oder

$R^3$ und $R^4$ zusammen mit dem Stickstoffatom, an das sie gebunden sind, für einen gegebenenfalls einfach oder mehrfach, gleich oder verschieden substituierten 5- bis 7-gliedrigen, gesättigten oder ungesättigten Heterocyclus mit 1 bis 3 Heteroatomen, vorzugsweise Stickstoff oder Sauerstoff stehen, wobei als Substituenten vorzugsweise genannt seien: Alkyl mit 1 bis 4 Kohlenstoffatomen, Alkoxycarbonyl mit 1 bis 4 Kohlenstoffatomen im Alkylteil und Phenyl
und deren physiologisch verträglichen Säureadditionssalze und Metallsalzkomplexe, fungizide Eigenschaften aufweisen.

Die Verbindungen der Formel (I) können als geometrische Isomere oder Isomerengemische unterschiedlicher Zusammensetzung anfallen. Sowohl die reinen Isomeren als auch die Isomerengemische werden erfindungsgemäss beansprucht.

Überraschenderweise zeigen die erfindungsgemäss zu verwendenden substituierten Aminoketale der Formel (I) eine höhere fungizide Wirkung als die nach dem Stand der Technik bekannten Verbindungen (4-tert.-Butylphenyl)-(1-dimethylaminoprop-2-yl)-keton und Zink-ethylen-1,2-bis-(dithiocarbamat), welche Verbindungen gleicher Wirkungsrichtung sind. Die erfindungsgemässen Stoffe stellen somit eine Bereicherung der Technik dar.

Die erfindungsgemäss zu verwendenden substituierten Aminoketale sind durch die Formel (I) allgemein definiert. Bevorzugt sind diejenigen Verbindungen, in denen

$R^1$ für geradkettiges oder verzweigtes Alkyl mit 1 bis 12 Kohlenstoffatomen, Cyclopropyl, Cyclopentyl, Cyclohexyl, für geradkettiges oder verzweigtes Halogenalkyl mit 1 bis 6 Kohlenstoffatomen und 1 bis 5 Halogenatomen oder für jeweils gegebenenfalls ein- bis dreifach, gleich oder verschieden substituiertes Phenyl, Phenylalkyl, Phenoxyalkyl, Benzyloxyalkyl, Phenylthioalkyl, Phenylsulfinylalkyl und Phenylsulfonylalkyl mit jeweils 1 bis 5 Kohlenstoffatomen im Alkylteil, sowie gegebenenfalls ein- bis dreifach, gleich oder verschieden substituiertes Phenylalkenyl mit 2 bis 4 Kohlenstoffatomen im Alkenylteil steht, wobei als Phenylsubstituenten besonders bevorzugt sind:
Fluor, Chlor, Brom, Cyano, Nitro, Methyl, Methoxy, Methylthio, Ethyl, Ethoxy, Ethylthio, n- und i-Propyl, Isopropoxy, n-, iso-, sec.- und t-Butyl, Trifluormethyl, Trifluormethoxy, Trifluormethylthio, Cyclohexyl, Methoxycarbonyl, Ethoxycarbonyl und gegebenenfalls durch Fluor, Chlor oder Methyl substituiertes Phenyl oder Phenoxy;

$R^2$ für geradkettiges oder verzweigtes Alkyl mit 1 bis 10 Kohlenstoffatomen, für gegebenenfalls ein- bis dreifach, gleich oder verschieden substituiertes Phenyl und Benzyl, wobei als Phenyl- bzw. Benzylsubstituenten jeweils in Frage kommen:
Fluor, Chlor, Brom, Cyano, Nitro, Trifluormethyl, Trifluormethoxy, Trifluormethylthio, Methoxycarbonyl und Ethoxycarbonyl, ferner für Cyclopropyl, Cyclopentyl und Cyclohexyl,

$R^3$ für Methyl, Ethyl, n- und i-Propyl, n-, i-, Sec.- und t-Butyl, n-Pentyl, n-Hexyl, n-Heptyl, n-Octyl, n-Nonyl, n-Decyl, n-Dodecyl, Allyl, Propargyl, 2-Butenyl, Cyclopentyl, Cyclohexyl sowie für jeweils gegebenenfalls ein- bis zweifach, gleich oder verschieden durch Fluor, Chlor, Methyl oder Trifluormethyl substituiertes Phenyl oder Benzyl steht und

$R^4$ für n- und i-Propyl, n-, i-, sec.- und t-Butyl, n-Pentyl, n-Hexyl, n-Heptyl, n-Octyl, n-Nonyl, n-Decyl, Allyl, Propargyl, 2-Butenyl, Cyclopentyl, Cyclohexyl sowie für jeweils gegebenenfalls ein- bis zweifach, gleich oder verschieden durch Fluor, Chlor, Methyl oder Trifluormethyl substituiertes Phenyl oder Benzyl steht; oder

$R^3$ und $R^4$ zusammen mit dem Stickstoffatom, an das sie gebunden sind, für jeweils gegebenenfalls ein- bis dreifach, gleich oder verschieden substituiertes 1-Pyrrolidinyl, 1-Piperidinyl, 1-Piperazinyl, 4-Morpholinyl, 1-Perhydroazepinyl, 1-Imidazolyl, 1-(1,2,4-Triazolyl) oder 1-Pyrazolyl stehen, wobei als Substituenten besonders bevorzugt sind:
Methyl, Ethyl, n- und i-Propyl, Phenyl, Methoxycarbonyl und Ethoxycarbonyl.

Ganz besonders bevorzugt sind diejenigen substituierten Aminoketale der Formel (I), in denen

$R^1$ für geradkettiges oder verzweigtes Alkyl mit 1 bis 5 Kohlenstoffatomen sowie für jeweils gegebenenfalls ein- bis dreifach, gleich oder verschieden substituiertes Phenylalkenyl, Phenylalkyl, Phenoxyalkyl, Benzyloxyalkyl, Phenylthioalkyl, Phenylsulfinylalkyl und Phenylsulfonylalkyl mit jeweils bis zu 5 Kohlenstoffatomen im Alkylteil bzw. Alkenylteil steht, wobei als Phenylsubstituenten genannt seien:
Fluor, Chlor, Brom, Methyl, Ethyl, n- und i-Propyl, t-Butyl, Methoxy, Ethoxy, Isopropoxy, Cyclohexyl, Trifluormethyl, Trifluormethoxy, Trifluormethylthio, Methoxycarbonyl, Ethoxycarbonyl, Nitro, Cyano, Phenyl und Phenoxy;

$R^2$ für geradkettiges oder verzweigtes Alkyl mit 1 bis 4 Kohlenstoffatomen oder Wasserstoff steht;

$R^3$ für Methyl, Ethyl, n- und i-Propyl oder n-, i- und t-Butyl steht;

$R^4$ für Methyl, Ethyl, n- und i-Propyl, n- und i-Butyl, Pentyl oder Hexyl steht oder

$R^3$ und $R^4$ zusammen mit dem Stickstoffatom, an das sie gebunden sind, für jeweils gegebenenfalls ein- bis dreifach, gleich oder verschieden substituiertes 1-Piperidinyl, 1-Perhydroazepinyl, 1-Pyrrolidinyl, 4-Morpholinyl, 1-Piperazinyl, 1-Imidazolyl und 1-(1,2,4-Triazolyl) stehen, wobei als Substituenten genannt seien: Alkyl mit 1 bis 3 Kohlenstoffatomen, Methoxycarbonyl, Ethoxycarbonyl und Phenyl.

Insbesondere seien diejenigen substituierten Aminoketale der Formel (I) genannt, in denen

$R^1$ für die Gruppierungen

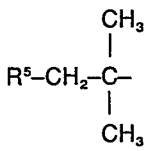 , 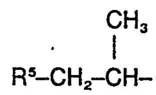 , 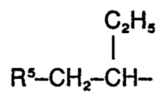 ,

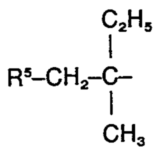 und 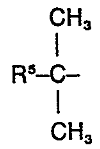 steht, wobei

$R^5$ für jeweils gegebenenfalls einfach bis dreifach, gleich oder verschieden substituiertes Phenyl, Phenoxy, Phenylthio, Phenylsulfinyl oder Phenylsulfonyl steht, wobei als Substituenten genannt seien: Fluor, Chlor, Brom, Nitro, Cyclohexyl, geradkettiges oder verzweigtes Alkyl mit 1 bis 4 Kohlenstoffatomen, geradkettiges oder verzweigtes Alkoxy mit 1 bis 3 Kohlenstoffatomen und Alkoxycarbonyl mit 1 oder 2 Kohlenstoffatomen im Alkoxyteil,

$R^2$ für geradkettiges oder verzweigtes Alkyl mit 1 bis 4 Kohlenstoffatomen steht,

$R^3$ für geradkettiges oder verzweigtes Alkyl mit 1 bis 4 Kohlenstoffatomen oder für geradkettiges oder verzweigtes Alkenyl mit 3 bis 5 Kohlenstoffatomen steht,

$R^4$ für geradkettiges oder verzweigtes Alkyl mit 1 bis 4 Kohlenstoffatomen steht und

$R^3$ und $R^4$ zusammen mit dem Stickstoffatom, an das sie gebunden sind, für einen jeweils gegebenenfalls einfach bis dreifach, gleich oder verschieden durch Alkyl mit 1 bis 3 Kohlenstoffatomen substituierten Piperidin-, Morpholin- und Pyrrolidinrest stehen,

ausserdem diejenigen Aminoketale der Formel (I), in denen

$R^1$ für die Gruppierungen

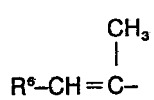 und 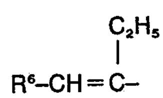

steht, wobei

$R^6$ für jeweils gegebenenfalls einfach bis dreifach, gleich oder verschieden substituiertes Phenyl steht, wobei als Substituenten genannt seien: Fluor, Chlor, Brom, Nitro, Cyclohexyl, geradkettiges oder verzweigtes Alkyl oder Alkoxy mit je 1 bis 4 Kohlenstoffatomen und Alkoxycarbonyl mit 1 oder 2 Kohlenstoffatomen im Alkoxyteil,

$R^2$ für geradkettiges oder verzweigtes Alkyl mit 1 bis 4 Kohlenstoffatomen steht,

$R^3$ für geradkettiges oder verzweigtes Alkyl mit 1 bis 4 Kohlenstoffatomen oder für geradkettiges oder verzweigtes Alkenyl mit 3 bis 5 Kohlenstoffatomen steht,

$R^4$ für geradkettiges oder verzweigtes Alkyl mit 1 bis 4 Kohlenstoffatomen steht und

$R^3$ und $R^4$ zusammen mit dem Stickstoffatom, an das sie gebunden sind, für einen jeweils gegebenenfalls einfach bis dreifach, gleich oder verschieden durch Alkyl mit 1 bis 3 Kohlenstoffatomen substituierten Piperidin-, Morpholin- oder Pyrrolidinrest stehen.

Erfindungsgemäss verwendbare Verbindungen sind auch die Additionsprodukte aus Säuren und den substituierten Aminoketalen der Formel (I) und Metallsalzkomplexe der Verbindungen der Formel (I).

Zur Herstellung von physiologisch verträglichen Säureadditionssalzen der Verbindungen der Formel (I) kommen vorzugsweise folgende Säuren in Frage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten organischen Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Die erfindungsgemäss zu verwendenden substituierten Aminoketale der Formel (I) sind teilweise bekannt. Die bekannten Verbindungen können nach literaturbekannten Verfahren hergestellt werden, ebenso wie die neuen substituierten Aminoketale der Formel (IA)

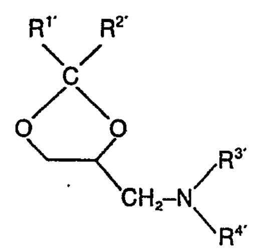 (IA)

in welcher

$R^{1'}$, $R^{2'}$, $R^{3'}$ und $R^{4'}$ die weiter unten angegebene Bedeutung haben,

indem man substituierte Dioxolane der allgemeinen Formel (II)

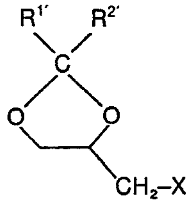

(II)

in welcher

$R^{1'}$ und $R^{2'}$ die weiter unten angegebene Bedeutung haben und

X für Halogen, gegebenenfalls substituiertes Alkylsulfonyloxy oder Arylsulfonyloxy steht,

mit Aminen der allgemeinen Formel (III)

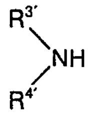

(III)

in welcher

$R^{3'}$ und $R^{4'}$ die weiter unten angegebene Bedeutung haben,

gegebenenfalls in Gegenwart eines Katalysators sowie gegebenenfalls in Gegenwart einer Base und gegebenenfalls in Gegenwart eines Verdünnungsmittels umsetzt.

In der Formel (IA) haben die Substituenten die nachfolgende Bedeutung:

$R^{1'}$ für geradkettiges oder verzweigtes Alkyl mit 6 bis 18 Kohlenstoffatomen; für substituiertes Alkyl, wie geradkettiges oder verzweigtes Halogenalkyl mit 2 bis 12 Kohlenstoffatomen und 1 bis 5 Halogenatomen, geradkettiges oder verzweigtes Cyanalkyl mit 1 bis 4 Kohlenstoffatomen im Alkylteil, ein- oder mehrfach, gleich oder verschieden substituiertes Benzyl, gegebenenfalls ein- oder mehrfach, gleich oder verschieden substituiertes Arylalkyl mit 2 bis 6 Kohlenstoffatomen im Alkylteil und 6 bis 10 Kohlenstoffatomen im Arylteil, Aryloxyalkyl, Aralkyloxyalkyl, Arylthioalkyl, Arylsulfinylalkyl, Arylsulfonylalkyl mit jeweils 1 bis 6 Kohlenstoffatomen in jedem Alkylteil und 6 bis 10 Kohlenstoffatomen im Arylteil, wobei als Aryl- bzw. Benzylsubstituenten jeweils in Frage kommen:

Halogen, Cyano, Nitro; Alkyl, Alkoxy und Alkylthio mit jeweils 1 bis 4 Kohlenstoffatomen; Halogenalkyl, Halogenalkoxy und Halogenalkylthio mit jeweils 1 oder 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy; und für Cycloalkylalkyl mit 1 bis 4 Kohlenstoffatomen im Alkylteil und 3 bis 7 Kohlenstoffatomen im gegebenenfalls ein- oder mehrfach durch Alkyl mit 1 bis 4 Kohlenstoffatomen substituierten Cycloalkylteil; ferner für geradkettiges oder verzweigtes Alkenyl mit 2 bis 5 Kohlenstoffatomen, für geradkettiges oder verzweigtes Alkinyl mit 2 bis 6 Kohlenstoffatomen; für ein- bis mehrfach, gleich oder verschieden durch Alkyl mit 1 bis 4 Kohlenstoffatomen substituiertes Cycloalkyl mit 3 bis 7 Kohlenstoffatomen und für Arylalkenyl mit 2 bis 6 Kohlenstoffatomen im Alkenylteil und 6 bis 10 Kohlenstoffatomen im Arylteil steht, wobei als Arylsubstituenten jeweils in Frage kommen:

Halogen, Cyano, Nitro; Alkyl, Alkoxy, Alkylthio mit jeweils 1 bis 4 Kohlenstoffatomen; Halogenalkyl, Halogenalkoxy und Halogenalkylthio mit jeweils 1 oder 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy,

$R^{2'}$ für Wasserstoff, für geradkettiges oder verzweigtes Alkyl mit 1 bis 18 Kohlenstoffatomen; für geradkettiges oder verzweigtes Halogenalkyl mit 1 bis 12 Kohlenstoffatomen und 1 bis 5 Halogenatomen, geradkettiges oder verzweigtes Cyanalkyl mit 1 bis 4 Kohlenstoffatomen im Alkylteil, gegebenenfalls ein- oder mehrfach, gleich oder verschieden substituiertes Arylalkyl, Aryloxyalkyl, Aralkyloxyalkyl, Arylthioalkyl, Arylsulfinylalkyl, Arylsulfonylalkyl mit jeweils 1 bis 6 Kohlenstoffatomen in jedem Alkylteil und 6 bis 10 Kohlenstoffatomen im Arylteil steht, wobei als Arylsubstituenten jeweils in Frage kommen:

Halogen, Cyano, Nitro; Alkyl, Alkoxy, Alkylthio mit jeweils 1 bis 4 Kohlenstoffatomen; Halogenalkyl, Halogenalkoxy und Halogenalkylthio mit jeweils 1 oder 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy; und Cycloalkylalkyl mit 1 bis 4 Kohlenstoffatomen im Alkylteil und 3 bis 7 Kohlenstoffatomen im gegebenenfalls ein- oder mehrfach durch Alkyl mit 1 bis 4 Kohlenstoffatomen substituierten Cycloalkylteil, ferner für geradkettiges oder verzweigtes Alkenyl oder Alkinyl mit jeweils 2 bis 6 Kohlenstoffatomen, für gegebenenfalls einfach oder mehrfach, gleich oder verschieden durch Alkyl mit 1 bis 4 Kohlenstoffatomen substituiertes Cycloalkyl mit 3 bis 7 Kohlenstoffatomen, oder für jeweils gegebenenfalls einfach oder mehrfach, gleich oder verschieden substituiertes Aryl mit 6 bis 10 Kohlenstoffatomen, sowie Arylalkenyl mit 2 bis 6 Kohlenstoffatomen im Alkenylteil und 6 bis 10 Kohlenstoffatomen im Arylteil steht, wobei als Arylsubstituenten jeweils in Frage kommen:

Halogen, Cyano, Nitro; Alkyl, Alkoxy, Alkylthio mit jeweils 1 bis 4 Kohlenstoffatomen; Halogenalkyl, Halogenalkoxy und Halogenalkylthio mit jeweils 1 bis 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy;

$R^{3'}$ für geradkettiges oder verzweigtes Alkyl mit 1 bis 12 Kohlenstoffatomen, für geradkettiges oder verzweigtes Alkenyl mit 3 bis 12 Kohlenstoffatomen, für geradkettiges oder verzweigtes Alkinyl mit 3 bis 7 Kohlenstoffatomen, für gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen sowie für jeweils gegebenenfalls einfach oder mehrfach, gleich oder verschieden substituiertes Aryl mit 6 bis 10 Kohlenstoffatomen und Aralkyl mit 1 oder 2 Kohlenstoffatomen im Alkylteil und 6 bis 10 Kohlenstoffatomen im Arylteil steht, wobei als Arylsubstituenten jeweils in Frage kommen: Halogen, Alkyl mit 1 bis 4 Kohlenstoffatomen sowie Halogenalkyl mit 1 oder 2 Kohlenstoffatomen und 1 bis 5 Halogenatomen;

$R^{4'}$ für geradkettiges oder verzweigtes Alkyl mit 1 bis 12 Kohlenstoffatomen, für geradkettiges oder verzweigtes Alkenyl mit 3 bis 12 Kohlenstoffatomen, für geradkettiges oder verzweigtes Alkinyl mit 3 bis 7 Kohlenstoffatomen, für gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen sowie für jeweils gegebenenfalls einfach oder mehrfach, gleich oder verschieden substituiertes Aryl mit 6 bis 10 Kohlenstoffatomen oder Aralkyl mit 1 bis 2 Kohlenstoffatomen im Alkylteil und 6 bis 10 Kohlenstoffatomen im Arylteil steht, wobei als Arylsubstituenten jeweils in Frage kommen: Halogen, Alkyl mit 1 bis 4 Kohlenstoffatomen, sowie Halogenalkyl mit 1 oder 2 Kohlenstoffatomen und 1 bis 5 Halogenatomen; oder

$R^{3'}$ und $R^{4'}$ zusammen mit dem Stickstoffatom an das sie gebunden sind, für einen gegebenenfalls einfach oder mehrfach, gleich oder verschieden substituierten 5- bis 7-gliedrigen, gesättigten oder ungesättigten Heterocyclus mit 1 bis 3 Heteroatomen, vorzugsweise Stickstoff oder Sauerstoff stehen, wobei als Substituenten vorzugsweise genannt seien: Alkyl mit 1 bis 4 Kohlenstoffatomen, Alkoxycarbonyl mit 1 bis 4 Kohlenstoffatomen im Alkylteil und Phenyl, ausgenommen Verbindungen, in welchen
$R^{1'}$ für Ethylbutylcarbinol (3-Heptyl), $R^{2'}$ für Phenyl und $R^{3'}$ und $R^{4'}$ für Ethyl stehen und
$R^{1'}$ für Methyl, $R^{2'}$ für $n{-}C_9H_{19}{-}$ und $R^{3'}$ und $R^{4'}$ für Ethyl stehen.

Besonders bevorzugt sind diejenigen substituierten Aminoketale der Formel (IA), in denen

$R^{1'}$ für geradkettiges oder verzweigtes Alkyl mit 6 bis 12 Kohlenstoffatomen; für geradkettiges oder verzweigtes Halogenalkyl mit 2 bis 6 Kohlenstoffatomen und 1 bis 5 Halogenatomen, für Cyanmethyl oder Cyanethyl, für ein- bis dreifach, gleich oder verschieden substituiertes Benzyl, gegebenenfalls ein- bis dreifach, gleich oder verschieden substituiertes Phenylalkyl mit 2 bis 6 Kohlenstoffatomen im Alkylteil, für Phenyloxyalkyl, Phenylalkyloxyalkyl, Phenylthioalkyl, Phenylsulfinylalkyl, Phenylsulfonylalkyl mit jeweils 1 bis 4 Kohlenstoffatomen in jedem Alkylteil steht, wobei als Phenyl- bzw. Benzylsubstituenten jeweils in Frage kommen:

Fluor, Chlor, Brom, Cyano, Nitro, Methyl, Methoxy, Methylthio, Ethyl, Ethoxy, Ethylthio, n- und i-Propyl, Isopropoxy, n-, iso-, sec.- und t-Butyl, Trifluormethyl, Trifluormethoxy, Trifluormethylthio, Cyclohexyl, Methoxycarbonyl, Ethoxycarbonyl und gegebenenfalls durch Fluor, Chlor oder Methyl substituiertes Phenyl oder Phenoxy; und ferner für Cycloalkylalkyl mit 1 bis 4 Kohlenstoffatomen im Alkylteil und 5 bis 7 Kohlenstoffatomen im gegebenenfalls ein- oder mehrfach durch Alkyl mit 1 bis 4 Kohlenstoffatomen substituierten Cycloalkylteil; ferner für geradkettiges oder verzweigtes Alkenyl mit 3 bis 5 Kohlenstoffatomen, für geradkettiges oder verzweigtes Alkinyl mit 3 bis 6 Kohlenstoffatomen; für ein- bis dreifach, gleich oder verschieden durch Methyl, Ethyl oder Propyl substituiertes Cycloalkyl mit 5 bis 7 Kohlenstoffatomen und für Phenylalkenyl mit 2 bis 5 Kohlenstoffatomen im Alkenylteil steht, wobei als Phenylsubstituenten jeweils in Frage kommen:

Fluor, Chlor, Brom, Cyano, Nitro, Methyl, Methoxy, Methylthio, Ethyl, Ethoxy, Ethylthio, n- und i-Propyl, Isopropoxy, n-, iso-, sec.- und t-Butyl, Trifluormethyl, Trifluormethoxy, Trifluormethylthio, Cyclohexyl, Methoxycarbonyl, Ethoxycarbonyl und gegebenenfalls durch Fluor, Chlor oder Methyl substituiertes Phenyl oder Phenoxy;

$R^{2'}$ für Wasserstoff, für geradkettiges oder verzweigtes Alkyl mit 1 bis 10 Kohlenstoffatomen; für gegebenenfalls ein- bis dreifach, gleich oder verschieden substituiertes Phenyl oder Benzyl steht, wobei als Phenyl- bzw. Benzylsubstituenten jeweils in Frage kommen:

Fluor, Chlor, Brom, Cyano, Nitro, Trifluormethyl, Trifluormethoxy, Trifluormethylthio, Methoxycarbonyl und Ethoxycarbonyl,

$R^{3'}$ für Methyl, Ethyl, n- und i-Propyl, n-, i-, sec.- und t-Butyl, n-Pentyl, n-Hexyl, n-Heptyl, n-Octyl, n-Nonyl, n-Decyl, n-Dodecyl, Allyl, Propargyl, 2-Butenyl, Cyclopentyl, Cyclohexyl sowie für jeweils gegebenenfalls ein- bis zweifach, gleich oder verschieden durch Fluor, Chlor, Methyl oder Trifluormethyl substituiertes Phenyl oder Benzyl steht und

$R^{4'}$ für n- und i-Propyl, n-, i-, sec.- und t-Butyl, n-Pentyl, n-Hexyl, n-Heptyl, n-Octyl, n-Nonyl, n-Decyl, Allyl, Propargyl, 2-Butenyl, Cyclopentyl, Cyclohexyl sowie für jeweils gegebenenfalls einbis zweifach, gleich oder verschieden durch Fluor, Chlor, Methyl oder Trifluormethyl substituiertes Phenyl oder Benzyl steht; oder

$R^{3'}$ und $R^{4'}$ zusammen mit dem Stickstoffatom, an das sie gebunden sind, für jeweils gegebenenfalls ein- bis dreifach, gleich oder verschieden substituiertes 1-Pyrrolidinyl, 1-Piperidinyl, 1-Piperazinyl, 4-Morpholinyl, 1-Perhydroazepinyl, 1-Imidazolyl, 1-(1,2,4-Triazolyl) oder 1-Pyrazolyl stehen, wobei als Substituenten besonders bevorzugt sind:

Methyl, Ethyl, n- und i-Propyl, Phenyl, Methoxycarbonyl und Ethoxycarbonyl.

Insbesondere seien diejenigen substituierten Aminoketale der Formel (IA) genant, in denen

$R^{1'}$ für die Gruppierungen

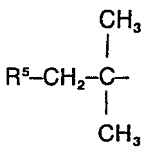 , 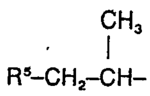 , 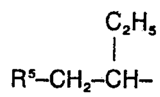 ,

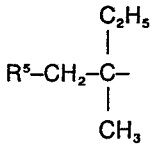 und 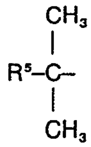 steht, wobei

$R^5$ für jeweils gegebenenfalls einfach bis dreifach, gleich oder verschieden substituiertes Phenyl, Phenoxy, Phenylthio, Phenylsulfinyl oder Phenylsulfonyl steht, wobei als Substituenten genannt seien:
Fluor, Chlor, Brom, Nitro, Cyclohexyl, geradkettiges oder verzweigtes Alkyl mit 1 bis 4 Kohlenstoffatomen, geradkettiges oder verzweigtes Alkoxy mit 1 bis 3 Kohlenstoffatomen und Alkoxycarbonyl mit 1 oder 2 Kohlenstoffatomen im Alkoxyteil,

$R^{2'}$ für geradkettiges oder verzweigtes Alkyl mit 1 bis 4 Kohlenstoffatomen steht,

$R^{3'}$ für geradkettiges oder verzweigtes Alkyl mit 1 bis 4 Kohlenstoffatomen oder für geradkettiges oder verzweigtes Alkenyl mit 3 bis 5 Kohlenstoffatomen steht,

$R^{4'}$ für geradkettiges oder verzweigtes Alkyl mit 1 bis 4 Kohlenstoffatomen steht und

$R^{3'}$ und $R^{4'}$ zusammen mit dem Stickstoffatom, an das sie gebunden sind, für jeweils gegebenenfalls einfach bis dreifach, gleich oder verschieden durch Alkyl mit 1 bis 3 Kohlenstoffatomen substituierten Piperidin-, Morpholin- oder Pyrrolidinrest stehen,
ausserdem diejenigen Aminoketale der Formel (IA), in denen

$R^{1'}$ für die Gruppierungen

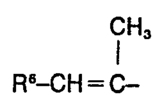 und 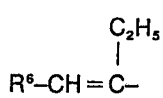 steht,

wobei

$R^6$ für jeweils gegebenenfalls einfach bis dreifach, gleich oder verschieden substituiertes Phenyl steht, wobei als Substituenten genannt seien: Fluor, Chlor, Brom, Nitro, Cyclohexyl, geradkettiges oder verzweigtes Alkyl oder Alkoxy mit je 1 bis 4 Kohlenstoffatomen und Alkoxycarbonyl mit 1 oder 2 Kohlenstoffatomen im Alkoxyteil,

$R^{2'}$ für geradkettiges oder verzweigtes Alkyl mit 1 bis 4 Kohlenstoffatomen steht,

$R^{3'}$ für geradkettiges oder verzweigtes Alkyl mit 1 bis 4 Kohlenstoffatomen oder für geradkettiges oder verzweigtes Alkenyl mit 3 bis 5 Kohlenstoffatomen steht,

$R^{4'}$ für geradkettiges oder verzweigtes Alkyl mit 1 bis 4 Kohlenstoffatomen steht und

$R^{3'}$ und $R^{4'}$ zusammen mit dem Stickstoffatom, an das sie gebunden sind, für jeweils gegebenenfalls einfach bis dreifach, gleich oder verschieden durch Alkyl mit 1 bis 3 Kohlenstoffatomen substituierten Piperidin-, Morpholin- oder Pyrrolidinrest stehen.

Die bei der Durchführung des angegebenen Verfahrens als Ausgangsstoffe benötigten substituierten Dioxolane sind durch die Formel (II) allgemein definiert. In dieser Formel haben $R^{1'}$ und $R^{2'}$ vorzugsweise diejenigen Bedeutungen, die bereits im Zusammenhang mit der Beschreibung der erfindungsgemässen Stoffe der Formel (IA) vorzugsweise für diese Reste genannt wurden. X steht vorzugsweise für Chlor, Brom, Methansulfonyloxy, p-Toluolsulfonyloxy oder Trifluormethansulfonyloxy.

Die substituierten Dioxolane der Formel (II) sind teilweise bekannt [vgl. z.B.: Rec. Trav. Chim. Pays-Bas 91, 989–1001 (1972), Farm. Ed. Sci. 29, 167–174 (1974); J. med. Chem. 6 (1963), 3, 325–328] oder können nach den dort angegebenen Verfahren in analoger Weise erhalten werden, indem man zum Beispiel Aldehyde oder Ketone der Formel

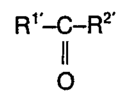 (IV)

in welcher

$R^{1'}$ und $R^{2'}$ die oben angegebene Bedeutung haben,

in üblicher Weise mit substituierten 1,2-Diolen der Formel

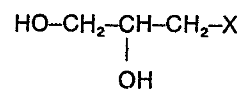 (V)

in welcher

X die oben angegebene Bedeutung hat,

in Gegenwart eines inerten organischen Lösungsmittels, wie z.B. Toluol, und in Gegenwart eines Katalysators, wie z.B. p-Toluolsulfonsäure, bei Temperaturen zwischen 50 °C und 120 °C umsetzt.

Die ausserdem für das angegebene Verfahren als Ausgangsstoffe benötigten Amine sind durch die Formel (III) allgemein definiert. In dieser Formel stehen $R^{3'}$ und $R^{4'}$ vorzugsweise für diejenigen Reste, die bereits im Zusammenhang mit der Beschreibung der erfindungsgemässen Stoffe der Formel (IA) vorzugsweise für diese Substituenten genannt wurden.

Die Amine der Formel (III) sind allgemein bekannte Verbindungen der organischen Chemie.

Als Verdünnungsmittel kommen für das Verfahren organische Lösungsmittel in Frage. Hierzu gehören vorzugsweise aromatische Kohlenwasserstoffe, wie Benzol, Toluol oder Xylol; halogenierte Kohlenwasserstoffe, wie Tetrachlorkohlenstoff oder Chlorbenzol; Formamide, wie Dimethylformamid; Nitrile, wie Acetonitril oder Propionitril; Alkohole, wie Propanol oder Butanol; Amine wie Triethylamin oder Piperidin; sowie die hochpolaren Lösungsmittel Dimethylsulfoxid oder Hexamethylphosphorsäuretriamid.

Das Verfahren wird gegebenenfalls in Gegenwart einer Base als Säurebindemittel durchgeführt. Hierbei können alle üblichen organischen und insbesondere anorganischen Basen eingesetzt werden. Hierzu gehören vorzugsweise Alkalimetallhydroxide oder Carbonate, wie beispielsweise Natriumhydroxid, Natriumcarbonat oder Kaliumcarbonat; ferner Triethylamin und Pyridin. Gegebenenfalls können auch die als Ausgangsstoffe eingesetzten Amine der Formel (III) als Lösungsmittel und als Säurebindemittel verwendet werden.

Das Verfahren wird gegebenenfalls in Gegenwart eines Katalysators durchgeführt. Vorzugsweise verwendet man Alkalimetalljodide, wie zum Beispiel Kaliumjodid.

Die Reaktionstemperaturen können bei dem Verfahren in einem grösseren Bereich variiert werden. Im allgemeinen arbeitet man zwischen 50 °C und 250 °C, vorzugsweise zwischen 80 °C und 200 °C.

Das Verfahren kann bei Normaldruck, aber auch bei erhöhtem Druck durchgeführt werden. Im allgemeinen arbeitet man bei Drucken zwischen etwa 1,5 atü und 5 atü, vorzugsweise zwischen 1,5 atü und 3 atü.

Bei der Durchführung des Verfahrens setzt man auf 1 Mol substituiertes Dioxolan der Formel (II) vorzugsweise 1 bis 30 Mol Amin der Formel (III) ein, je nachdem ob das Amin auch als Verdünnungsmittel und/oder Säurebindemittel verwendet wird. Die Isolierung der Endprodukte erfolgt nach üblichen Methoden.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) bzw. (IA) können in einfacher Weise nach üblichen Verfahren erhalten werden, so z.B. durch Lösen des Metallsalzes in Alkohol, z.B. Ethanol und Hinzufügen zur Verbindung der Formel (I) bzw. (IA). Man kann Metallsalz-Komplexe in bekannter Weise, z.B. durch Abfiltrieren, Isolieren und gegebenenfalls durch Umkristallisation reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemässen Wirkstoffe mit besonders gutem Erfolg zur Bekämpfung von Getreidekrankheiten, wie z.B. gegen den Erreger des Gerstenmehltaus (Erysiphe graminis) oder den Erreger der Streifenkrankheit (Drechslera graminea), von Reiskrankheiten, wie z.B. Pyricularia oryzae oder von Kartoffelkrankheiten, wie z.B. gegen den Erreger der Kartoffelkraut- und -knollenfäule (Phytophtora infestans) eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweisshydrolysate; als

Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungizide, Insektizide, Akarizide, Herbizide, Bakteriziden, Nematiziden, Schutzstoffe gegen Vogelfrass, Wuchsstoffen, Pflanzennährstoffen und Bodenstrukturverbesserungsmitteln.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Giessen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Herstellungsbeispiele

Beispiel 1

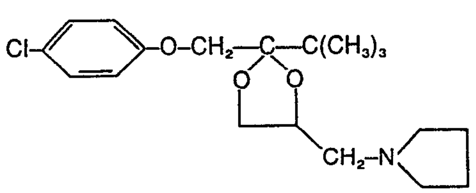

17,3 g (0,05 Mol) 2-(t-Butyl)-2-(4-chlorphenoxymethyl)-4-methansulfonyloxymethyl-1,3-dioxolan und 7,1 g (0,1 Mol) Pyrrolidin werden 15 Stunden auf 120 °C erhitzt. Nach dem Abkühlen wird überschüssiges Pyrrolidin im Vakuum abdestilliert und der Rückstand in Ether aufgenommen. Die Etherlösung wird zweimal mit Wasser gewaschen, über Natriumsulfat getrocknet und im Vakuum eingedampft.

Man erhält 12,1 g (65,7% der Theorie) 2-(t-Butyl)-2-(4-chlorphenoxymethyl)-4-(1-pyrrolidinylmethyl)-1,3-dioxolan als Öl vom Brechungsindex $n^{20}_D$ 1.5099.

Herstellung des Ausgangsproduktes:

a) 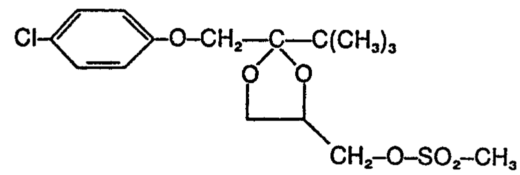

142 g (0,5 Mol) 2-(t-Butyl)-2-(4-chlorphenoxymethyl)-4-hydroxymethyl-1,3-dioxolan werden in 150 ml Pyridin aufgenommen und bei 0 °C tropfenweise mit 57 g (0,5 Mol) Methansulfonsäurechlorid versetzt. Man rührt 17 Stunden bei 20 °C–25 °C und destilliert dann das Lösungsmittel im Vakuum ab. Der Rückstand wird in Ether aufgenommen, die Etherlösung mit Wasser gewaschen, über Natriumsulfat getrocknet und im Vakuum eingedampft.

Man erhält 171 g (98% der Theorie) 2-(t-Butyl)-2-(4-chlorphenoxymethyl)-4-methansulfonyloxymethyl-1,3-dioxolan als Öl.

$^1$H-NMR ($CDCl_3$): δ (ppm) = 7,2 (d, 2H)
6,8 (d, 2H)
4,6–3,6 (m, 7H)
3,0 (d, 3H)
1,05 (s, 9H)

b) 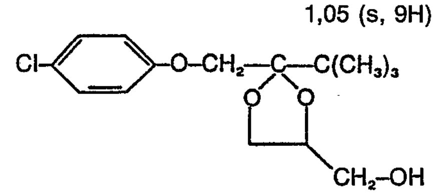

226 g (1 Mol) 1-(4-Chlorphenoxy)-3,3-dimethylbutan-2-on (aus Natrium-4-chlorphenolat und α-Brompinakolon; vergleiche hierzu DE-PS 2 201 063) werden zusammen mit 120 g (1,07 Mol) Glycerin, 19 g (0,1 Mol) p-Toluolsulfonsäuremonohydrat und 30 ml Butanol in 1000 ml Toluol gelöst und 16 Stunden am Wasserabscheider unter Rückfluss erhitzt. Nach dem Abkühlen wird die Toluolphase 4mal mit jeweils 250 ml Wasser gewaschen, über Magnesiumsulfat getrocknet, filtriert und im Vakuum eingedampft. Durch Destillation erhält man 186 g (65,4% der Theorie) 2-(t-Butyl)-2-(4-chlorphenoxymethyl)-4-hydroxymethyl-1,3-dioxolan vom Siedepunkt $KP_{0,1}$: 161 °C–163 °C.

Beispiel 2

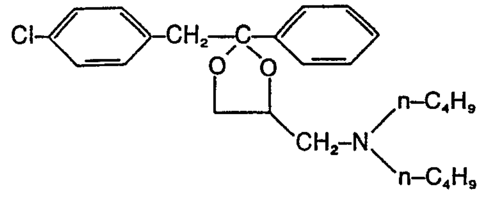

16,1 g (0,05 Mol) 4-Chlormethyl-2-(4-chlorphenylmethyl)-2-phenyl-1,3-dioxolan, 100 ml Di-n-butylamin und 13,8 g (0,1 Mol) Kaliumcarbonat werden zusammen mit einer Spatelspitze Kaliumjodid 8 Stunden bei 180 °C und 2 bar im Autoklaven gerührt.

Zur Aufarbeitung entfernt man das überschüssige Di-n-butylamin im Wasserstrahlvakuum bei 40 °C–100 °C Badtemperatur. Der erkaltete Rückstand wird in 50 ml Toluol aufgenommen, filtriert und im Vakuum eingedampft.

Man erhält 9,4 g (47% der Theorie) 2-(4-Chlorphenylmethyl)-4-(N,N-di-n-butylaminomethyl)-2-phenyl-1,3-dioxolan ls Öl vom Brechungsindex $n^{20}_D = 1.572$.

Herstellung des Ausgangsproduktes:

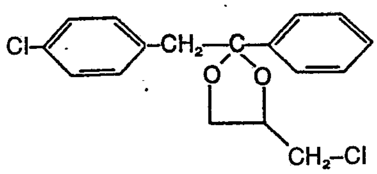

69 g (0,3 Mol) Phenyl-(4-chlorphenylmethyl)-keton, 66,3 g (0,6 Mol) 3-Chlor-1,2-propandiol und 5,2 g (0,03 Mol) p-Toluolsulfonsäure werden in 600 ml Toluol drei Tage am Wasserabscheider refluxiert. Die erkaltete Reaktionsmischung wird mit Natriumbicarbonatlösung extrahiert, über Natriumsulfat getrocknet und im Vakuum eingedampft.

Man erhält 95 g (98% der Theorie) 4-Chlormethyl-2-(4-chlorphenylmethyl)-2-phenyl-1,3-dioxolan vom Schmelzpunkt 65 °C.

Beispiel 3

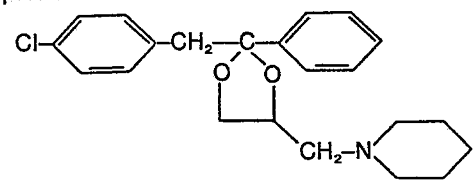

16,1 g (0,05 Mol) 4-Chlormethyl-2-(4-chlorphenylmethyl)-2-phenyl-1,3-dioxolan (Herstellung vergleiche Beispiel 2), 100 g (1,18 Mol) Piperidin, 1,38 g (0,1 Mol) Kaliumcarbonat und eine Spatelspitze Kaliumjodid werden zusammen 16 Stunden am Rückfluss gekocht. Die Reaktionsmischung wird im Vakuum eingeengt, in 150 ml 0,4 n Salzsäure aufgenommen und dreimal mit 50 ml Ligroin gewaschen. Die salzsaure Lösung wird mit Natriumchlorid gesättigt und 3mal mit 60 ml Essigester extrahiert. Die Essigesterphase wird mit Natriumbicarbonat- und Natriumchloridlösung gewaschen, über Natriumsulfat getrocknet und bei 40 °C Badtemperatur im Vakuum vom Lösungsmittel befreit.

Man erhält 10 g (53,8% der Theorie) 2-(4-Chlorphenylmethyl)-2-phenyl-4-(1-piperidinylmethyl)-1,3-dioxolan als wachsartig festen Rückstand vom Schmelzpunkt 58 °C.

In entsprechender Weise und gemäss dem angegebenen Verfahren wurden die folgenden Verbindungen der Formel (I)

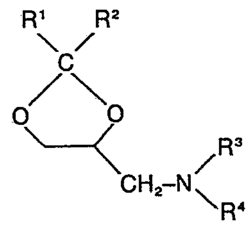

(I)

erhalten:

| Beispiel Nr. | $R^1$ | $R^2$ | 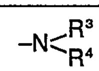 | Schmp. (°C) oder Brechungsindex ($n^{20}_D$) |
|---|---|---|---|---|
| 4 | $C_2H_5$ | $CH_3$ | 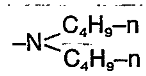 | 1.5027 |
| 5 | $C_2H_5$ | $CH_3$ | 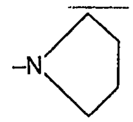 | 1.5036 |
| 6 | $C_2H_5$ | $CH_3$ | 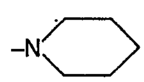 | 1.5012 |
| 7 | $C_2H_5$ | $CH_3$ | 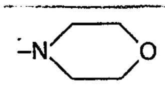 | 1.5047 |
| 8 | $C_2H_5$ | $CH_3$ | 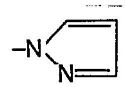 | 1.4682 |
| 9 | $i-C_4H_9$ | $CH_3$ | 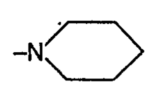 | 1.5039 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 10 | $i-C_4H_9$ | $CH_3$ | 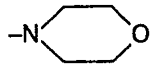 | 1.5041 |
| 11 | $i-C_4H_9$ | $CH_3$ | 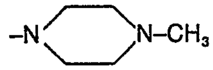 | 1.5035 |
| 12 | $n-C_9H_{19}$ | $CH_3$ | 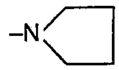 | 1.4570 |
| 13 | $n-C_9H_{19}$ | $CH_3$ | 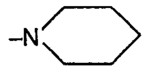 | 1.4535 |
| 14 | $n-C_9H_{19}$ | $CH_3$ | 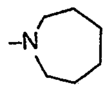 | 1.4549 |
| 15 | $n-C_9H_{19}$ | $CH_3$ | 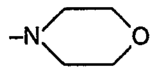 | 1.4489 |
| 16 | $n-C_9H_{19}$ | $CH_3$ | 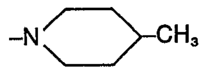 | 1.4510 |
| 17 | $n-C_9H_{19}$ | $CH_3$ | 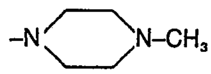 | 1.4551 |
| 18 | $n-C_9H_{19}$ | $n-C_9H_{19}$ | 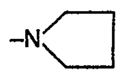 | 1.4510 |
| 19 | $n-C_9H_{19}$ | $n-C_9H_{19}$ | 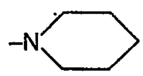 | 1.4588 |
| 20 | $n-C_9H_{19}$ | $n-C_9H_{19}$ | 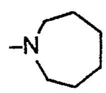 | 1.4578 |
| 21 | $n-C_9H_{19}$ | $n-C_9H_{19}$ | 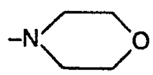 | 1.4539 |
| 22 | $n-C_9H_{19}$ | $n-C_9H_{19}$ | 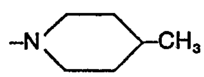 | 1.4534 |
| 23 | $n-C_9H_{19}$ | $n-C_9H_{19}$ | 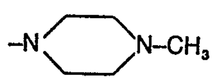 | 1.4555 |
| 24 | 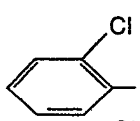 | $CH_3$ | 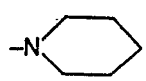 | 1.5301 |
| 25 | 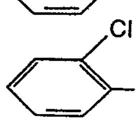 | $CH_3$ | 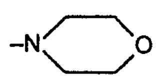 | 1.5326 |
| 26 | 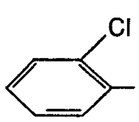 | $CH_3$ | 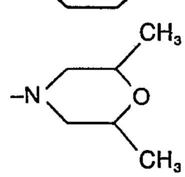 | 1.5084 |
| 27 | 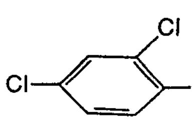 | $CH_3$ | 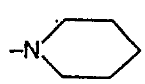 | 1.5372 |

| Beispiel Nr. | $R^1$ | $R^2$ | $-N{<}\genfrac{}{}{0pt}{}{R^3}{R^4}$ | Schmp. (°C) oder Brechungsindex ($n_D^{20}$) |
|---|---|---|---|---|
| 28 | 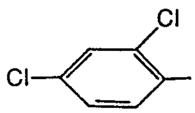 | $CH_3$ | 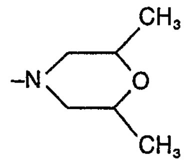 | 1.5219 |
| 29 | 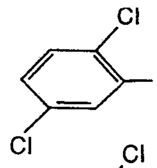 | $CH_3$ | 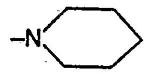 | 1.5383 |
| 30 | 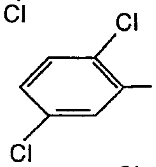 | $CH_3$ | 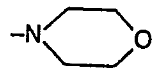 | 1.5384 |
| 31 | 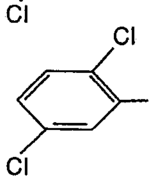 | $CH_3$ | 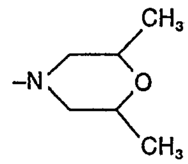 | 1.5181 |
| 32 | 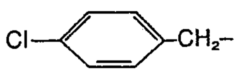 | $C_6H_5$ | 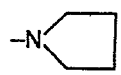 | 61 |
| 33 | 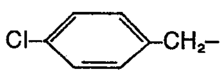 | $C_6H_5$ | 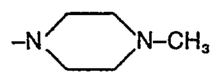 | 71 |
| 34 | 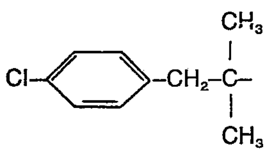 | $CH_3$ | 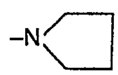 | 1.5261 |
| 35 | 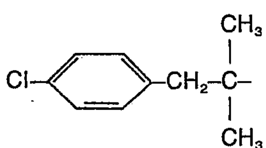 | $CH_3$ | 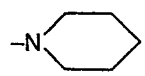 | 1.5244 |
| 36 | 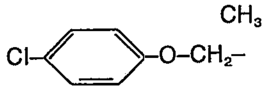 | $CH_3$ | 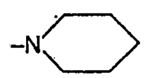 | 1.5282 |
| 37 | 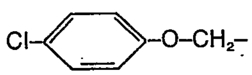 | t-$C_4H_9$ | $-N{<}\genfrac{}{}{0pt}{}{C_3H_7\text{–n}}{C_3H_7\text{–n}}$ | 1.4889 |
| 38 | 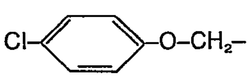 | t-$C_4H_9$ | $-N{<}\genfrac{}{}{0pt}{}{C_4H_9\text{–i}}{C_4H_9\text{–i}}$ | 1.5003 |
| 39 | 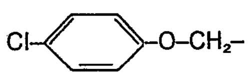 | t-$C_4H_9$ | 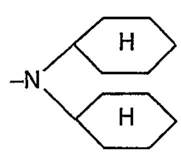 | 98 |
| 40 | 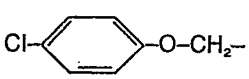 | t-$C_4H_9$ | $-N{<}\genfrac{}{}{0pt}{}{CH_2\text{–}C_6H_5}{CH_2\text{–}C_6H_5}$ | 121 |
| 41 | 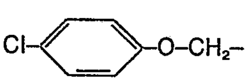 | t-$C_4H_9$ | $-N{<}\genfrac{}{}{0pt}{}{CH_3}{CH_2\text{–}C_6H_5}$ | 1.5049 |
| 42 | 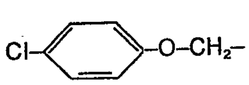 | t-$C_4H_9$ | 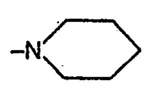 | 1.5173 |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 43 | 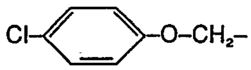 | t-$C_4H_9$ | 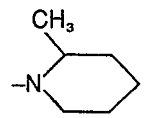 | 1.5068 |
| 44 | 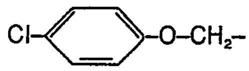 | t-$C_4H_9$ | 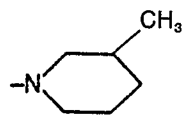 | 1.5026 |
| 45 | 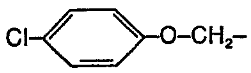 | t-$C_4H_9$ | 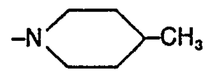 | 1.5032 |
| 46 | 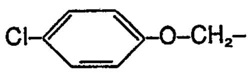 | t-$C_4H_9$ | 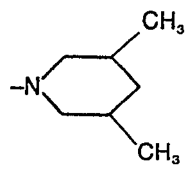 | 1.5012 |
| 47 | 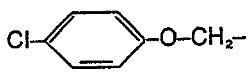 | t-$C_4H_9$ | 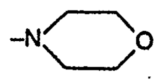 | 40 |
| 48 | 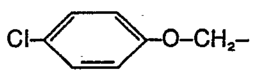 | t-$C_4H_9$ | 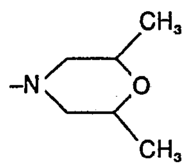 | 1.5082 |
| 49 | 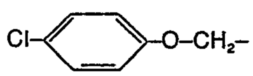 | t-$C_4H_9$ | 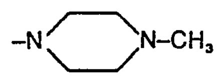 | 1.5187 |
| 50 | 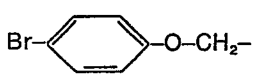 | t-$C_4H_9$ | 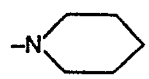 | 1.5271 |
| 51 | 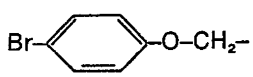 | t-$C_4H_9$ | 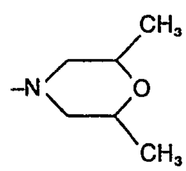 | 1.5126 |
| 52 | 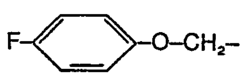 | t-$C_4H_9$ | 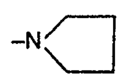 | 1.4917 |
| 53 | 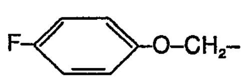 | t-$C_4H_9$ | 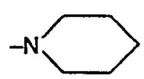 | 1.5026 |
| 54 | 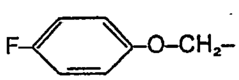 | t-$C_4H_9$ | 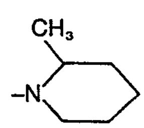 | 1.4992 |
| 55 | 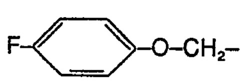 | t-$C_4H_9$ | 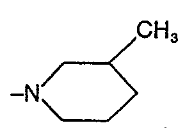 | 1.5023 |
| 56 | 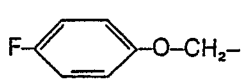 | t-$C_4H_9$ | 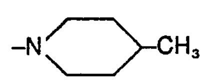 | 1.5045 |

| Beispiel Nr. | $R^1$ | $R^2$ | $-N<{R^3 \atop R^4}$ | Schmp. (°C) oder Brechungsindex ($n_D^{20}$) |
|---|---|---|---|---|
| 57 | 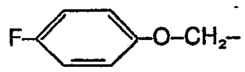 | $t-C_4H_9$ | 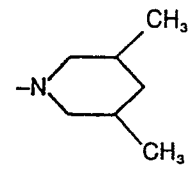 | 1.5008 |
| 58 | 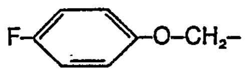 | $t-C_4H_9$ | 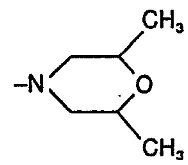 | 1.4938 |
| 59 | 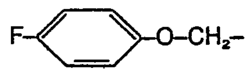 | $t-C_4H_9$ | 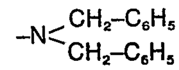 | 134 |
| 60 | 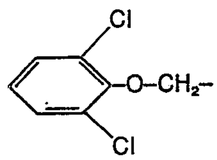 | $t-C_4H_9$ | 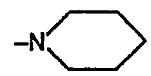 | 1.5109 |
| 61 | 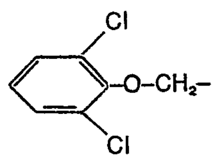 | $t-C_4H_9$ | 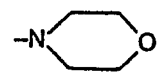 | 1.5226 |
| 62 | 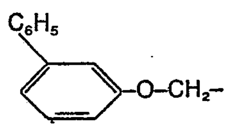 | $t-C_4H_9$ | 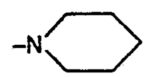 | 1.5577 |
| 63 | 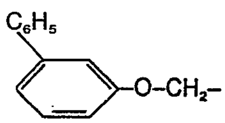 | $t-C_4H_9$ | 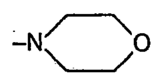 | 1.5557 |
| 64 | 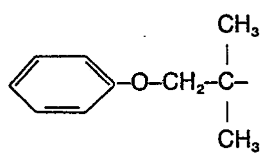 | $CH_3$ | 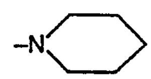 | 1.5011 |
| 65 | 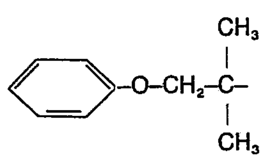 | $CH_3$ | 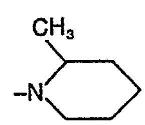 | 1.4998 |
| 66 | 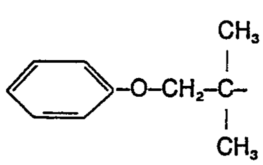 | $CH_3$ | 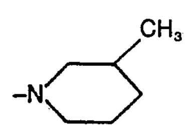 | 1.4973 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 67 | 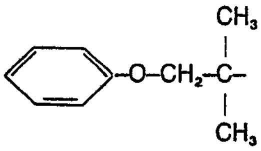 | $CH_3$ | 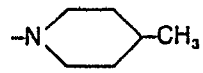 | 1.4939 |
| 68 | 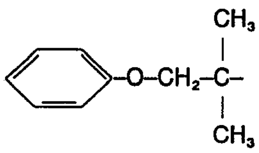 | $CH_3$ | 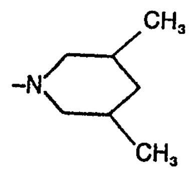 | 1.4974 |
| 69 | 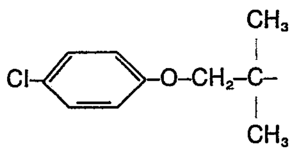 | $CH_3$ | 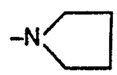 | 1.5200 |
| 70 | 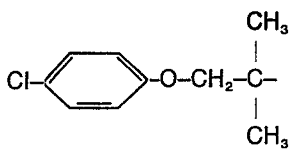 | $CH_3$ | 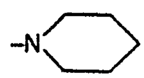 | 1.5121 |
| 71 | 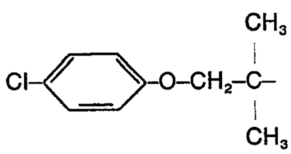 | $CH_3$ | 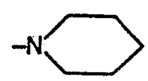 (× HCl) | 153–155 |
| 72 | 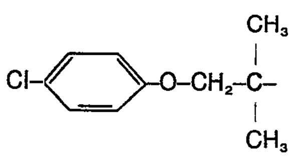 | $CH_3$ | 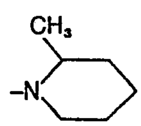 | 1.5102 |
| 73 | 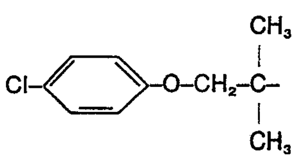 | $CH_3$ | 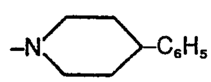 | 1.5431 |
| 74 | 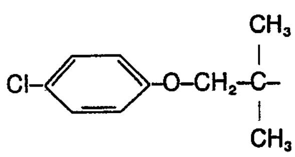 | $CH_3$ | 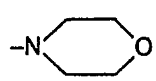 | 1.5191 |
| 75 | 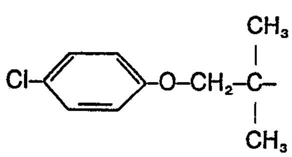 | $CH_3$ | 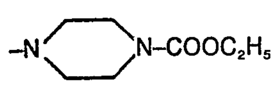 | 1.4993 |
| 76 | 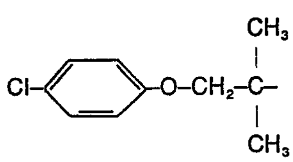 | $CH_3$ | $-N<^{C_4H_9-n}_{C_4H_9-n}$ | 1.5377 |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 77 | 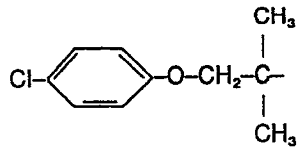 | $CH_3$ | 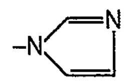 | 1.5150 |
| 78 | 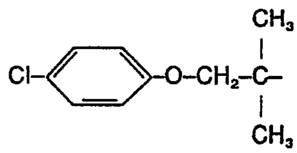 | $CH_3$ | 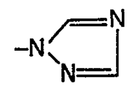 | 100 (× HCl) |
| 79 | 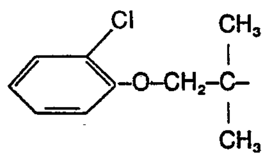 | $CH_3$ | 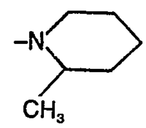 | 1.5047 |
| 80 | 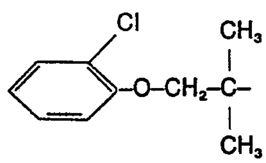 | $CH_3$ | 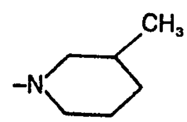 | 1.5051 |
| 81 | 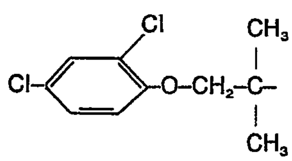 | $CH_3$ | 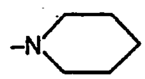 | 1.5006 |
| 82 | 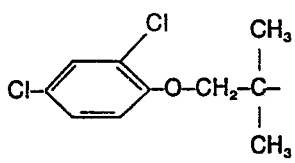 | $CH_3$ | 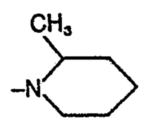 | 1.4941 |
| 83 | 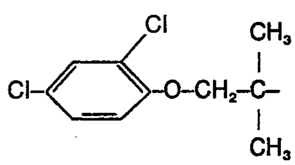 | $CH_3$ | 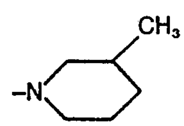 | 1.5034 |
| 84 | 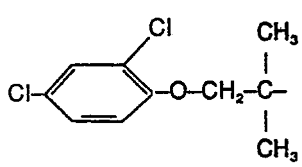 | $CH_3$ | 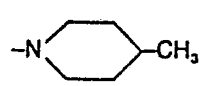 | 1.4987 |
| 85 | 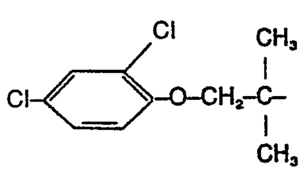 | $CH_3$ | 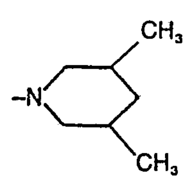 | 1.4980 |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 86 | 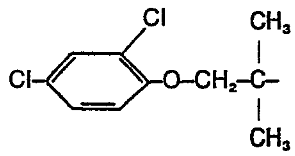 | $CH_3$ | 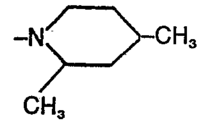 | 1.5021 |
| 87 | 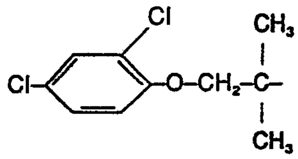 | $CH_3$ | 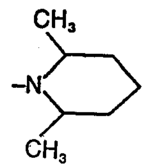 | 1.4980 |
| 88 | 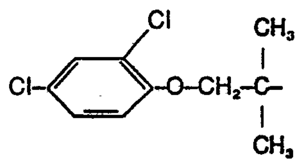 | $CH_3$ | 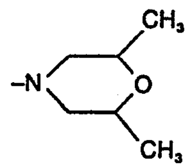 | 1.5034 |
| 89 | 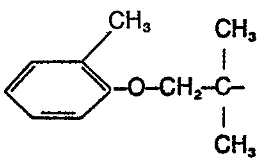 | $CH_3$ | 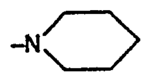 | 1.4952 |
| 90 | 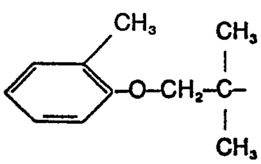 | $CH_3$ | 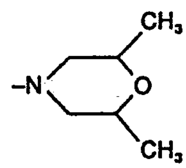 | 1.4874 |
| 91 | 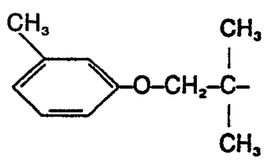 | $CH_3$ | 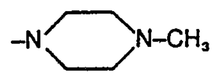 | 1.5023 |
| 92 | 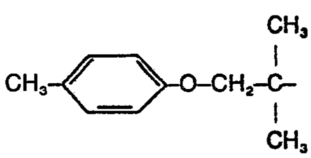 | $CH_3$ | $-N{<}^{C_4H_9-n}_{C_4H_9-n}$ | 1.5132 |
| 93 | 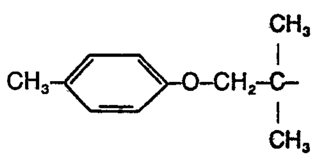 | $CH_3$ | 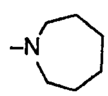 | 1.5118 |
| 94 | 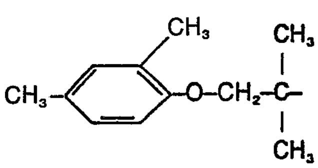 | $CH_3$ | 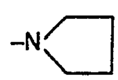 | 1.5081 |
| 95 | 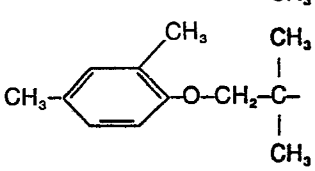 | $CH_3$ | 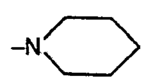 | 1.5064 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 96 | 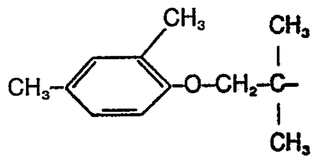 | $CH_3$ | 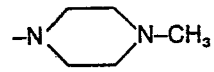 | 1.5086 |
| 97 | 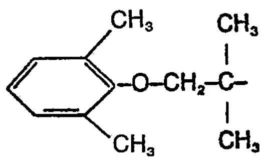 | $CH_3$ | 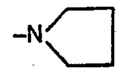 | 1.5087 |
| 98 | 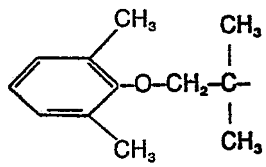 | $CH_3$ | 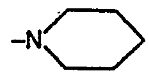 | 1.5074 |
| 99 | 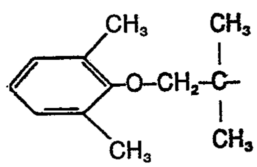 | $CH_3$ | 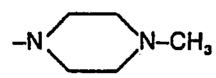 | 1.5067 |
| 100 | 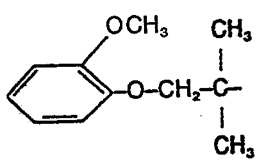 | $CH_3$ | $-N<^{C_4H_9-n}_{C_4H_9-n}$ | 1.5063 |
| 101 | 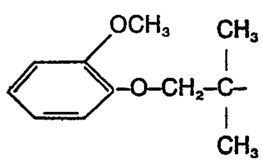 | $CH_3$ | 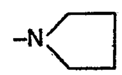 | 1.5057 |
| 102 | 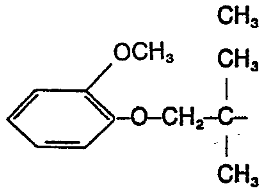 | $CH_3$ | 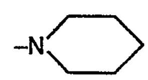 | 1.5070 |
| 103 | 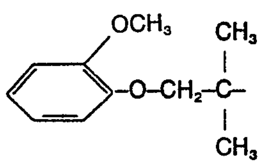 | $CH_3$ | 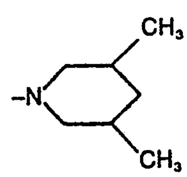 | 1.4944 |
| 104 | 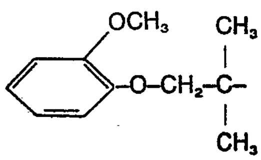 | $CH_3$ | 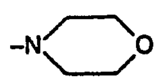 | 1.5067 |
| 105 | 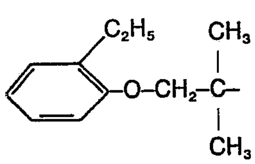 | $CH_3$ | 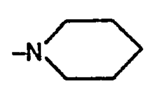 | 1.5087 |

| Beispiel Nr. | $R^1$ | $R^2$ | $-N\begin{smallmatrix}R^3\\R^4\end{smallmatrix}$ | Schmp. (°C) oder Brechungsindex ($n_D^{20}$) |
|---|---|---|---|---|
| 106 | 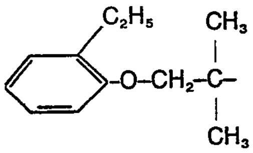 | $CH_3$ | 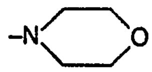 | 1.5076 |
| 107 | 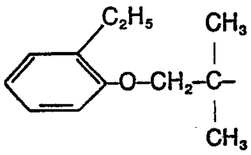 | $CH_3$ | 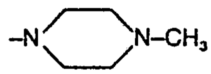 | 1.5046 |
| 108 | 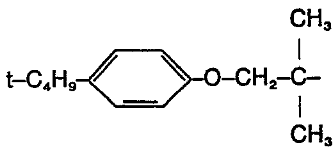 | $CH_3$ | 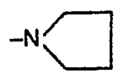 | 1.4948 |
| 109 | 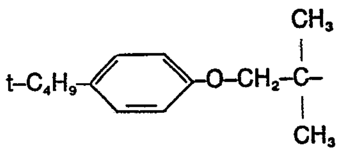 | $CH_3$ | 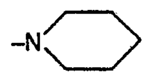 | 1.4954 |
| 110 | 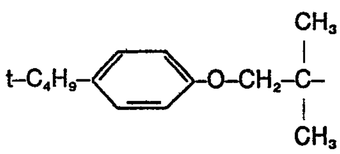 | $CH_3$ | 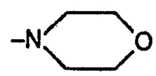 | 1.4962 |
| 111 | 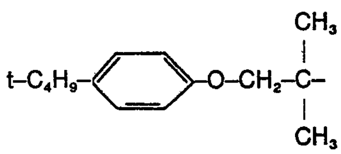 | $CH_3$ | 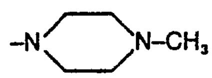 | 1.4975 |
| 112 | 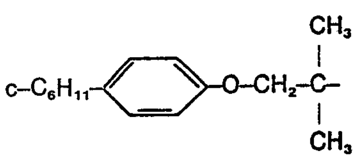 | $CH_3$ | 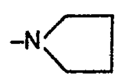 | 1.5110 |
| 113 | 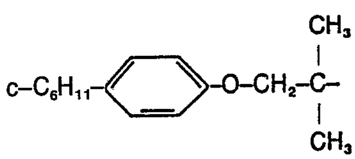 | $CH_3$ | 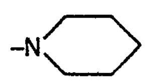 | 1.5098 |
| 114 | 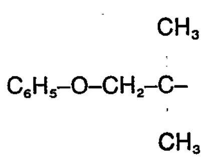 | $CH_3$ | 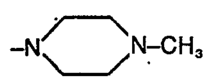 | 1.5031 |
| 115 | 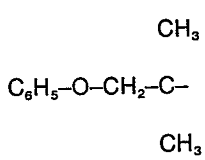 | $CH_3$ | 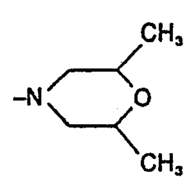 | 1.4925 |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 116 | 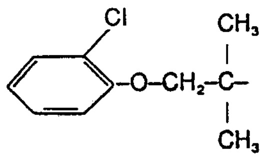 | $CH_3$ | 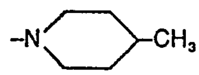 | 1.5044 |
| 117 | 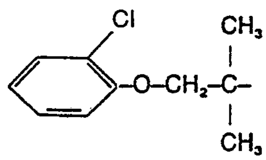 | $CH_3$ | 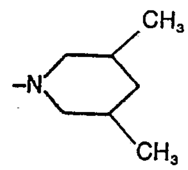 | 1.5047 |
| 118 | 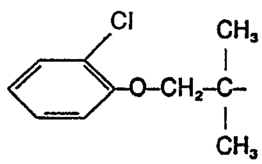 | $CH_3$ | 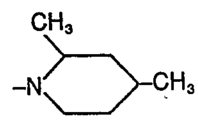 | 1.5040 |
| 119 | 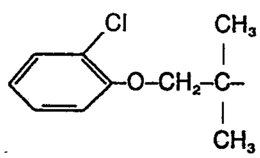 | $CH_3$ | 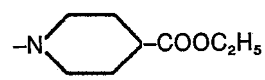 | 1.5044 |
| 120 | 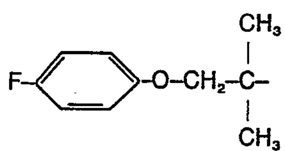 | $CH_3$ | 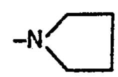 | 1.4912 |
| 121 | 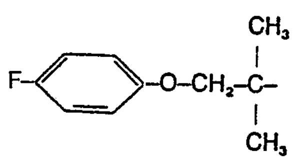 | $CH_3$ | 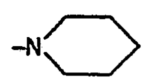 | 1.4907 |
| 122 | 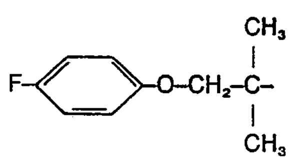 | $CH_3$ | 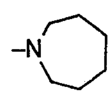 | 1.4930 |
| 123 | 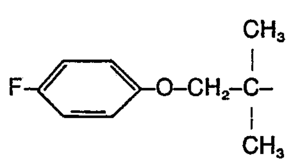 | $CH_3$ | 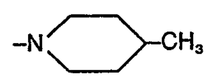 | 1.4868 |
| 124 | 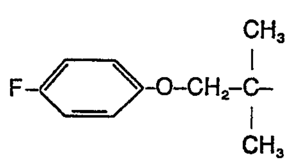 | $CH_3$ | 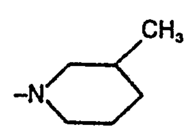 | 1.4873 |
| 125 | 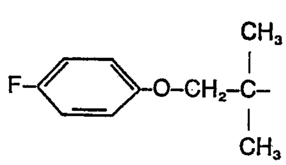 | $CH_3$ | 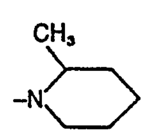 | 1.4897 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 126 | 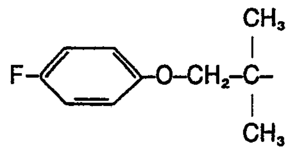 | $CH_3$ | 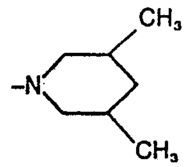 | 1.4841 |
| 127 | 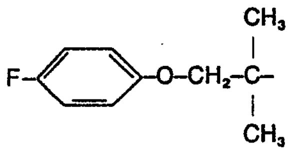 | $CH_3$ | 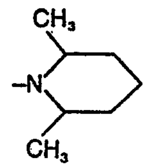 | 1.4893 |
| 128 | 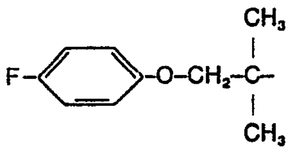 | $CH_3$ | 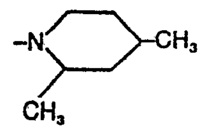 | 1.4864 |
| 129 | 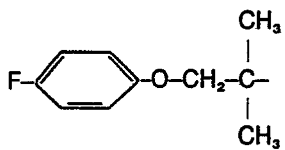 | $CH_3$ | 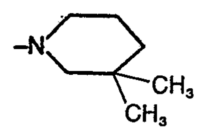 | 1.4825 |
| 130 | 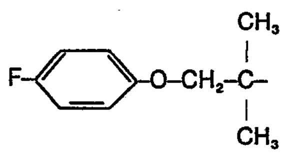 | $CH_3$ | 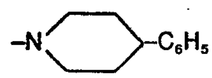 | 1.5118 |
| 131 | 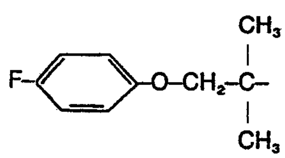 | $CH_3$ | 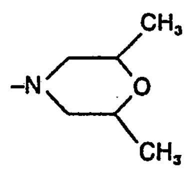 | 1.4839 |
| 132 | 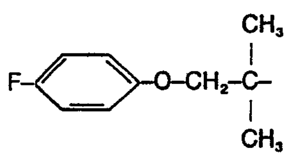 | $CH_3$ | 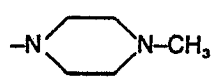 | 1.4919 |
| 133 | 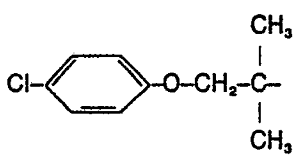 | $CH_3$ | 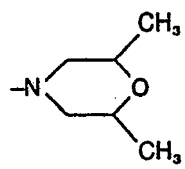 | 1.5361 |
| 134 | 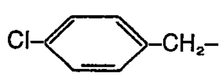 | 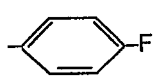 | $-N<^{C_4H_9-n}_{C_4H_9-n}$ | 1.5214 |
| 135 | 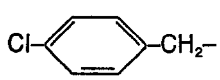 | 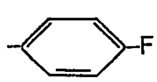 | $-N<^{C_3H_7-n}_{C_3H_7-n}$ | 1.5148 |
| 136 | 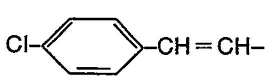 | $C(CH_3)_3$ | 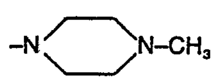 | 1.5298 |
| 137 | 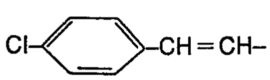 | $C(CH_3)_3$ | 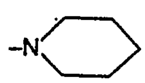 | 74 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 138 | 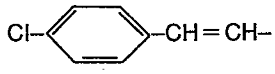 | $C(CH_3)_3$ | 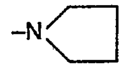 | 86–87 |
| 139 | 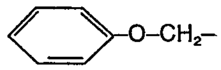 | H | 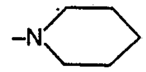 | 1.5222 |
| 140 | 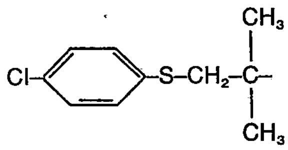 | $CH_3$ | 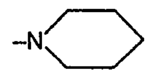 | 1.5491 |
| 141 | 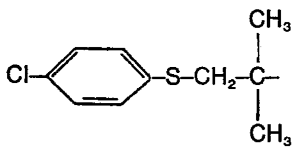 | $CH_3$ | 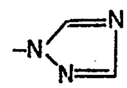 | 1.5626 |
| 142 | 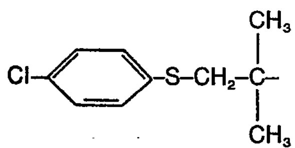 | $CH_3$ | 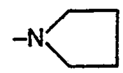 | 1.5538 |
| 143 | 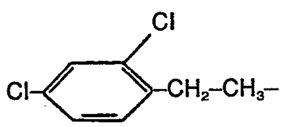 | $C(CH_3)_3$ | 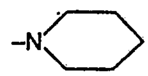 | 1.5171 |
| 144 | 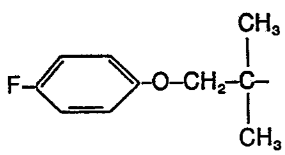 | $CH_3$ | 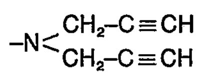 | 1.4968 |
| 145 | 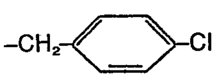 | 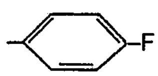 | 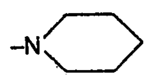 | 1.5498 |
| 146 | 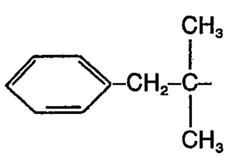 | $CH_3$ | 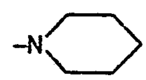 | 1.5091 |
| 147 | 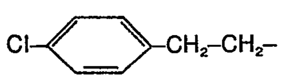 | $-C(CH_3)_3$ | 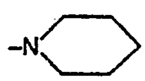 | 1.5179 |
| 148 | 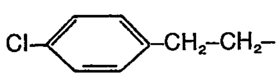 | $-C(CH_3)_3$ | 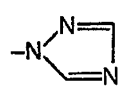 | 117 |
| 149 | 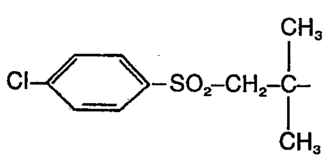 | $CH_3$ | 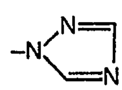 | 1.5187 |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 150 | 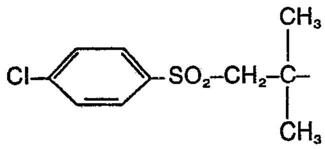 | $CH_3$ | 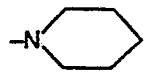 | 1.5231 |
| 151 | 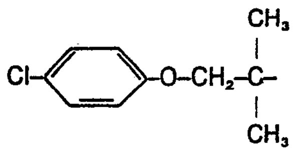 | 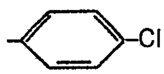 | 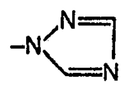 | 1.5518 |
| 152 | 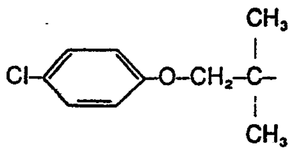 | 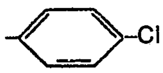 | 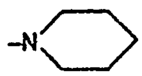 | 1.5518 |
| 153 | 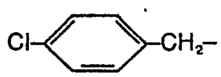 | 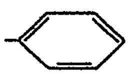 | 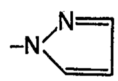 | 1.5641 |
| 154 | 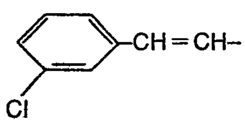 | $CH_3$ | 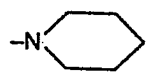 | 1.5366 |
| 155 | 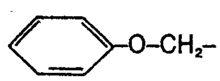 | H | 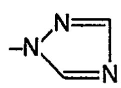 | 1.5409 |
| 156 | 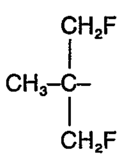 | $CH_3$ | 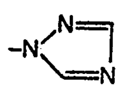 | 30 |
| 157 | 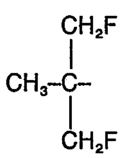 | $CH_3$ | 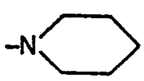 | 1.4599 |
| 158 | 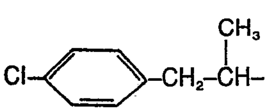 | $CH_3$ | 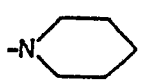 | 1.5195 |
| 159 | 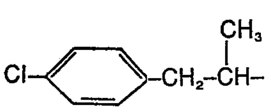 | $CH_3$ | 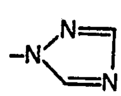 | 1.5361 |
| 160 | 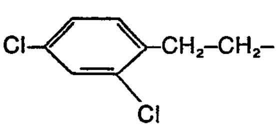 | $t-C_4H_9$ | 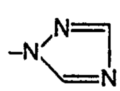 | 1.5366 |
| 161 | 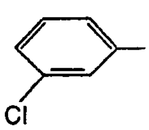 | $i-C_4-H_9$ | 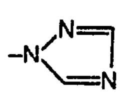 | 1.5263 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 162 | 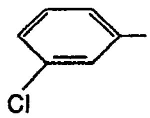 | $i-C_4H_9$ | 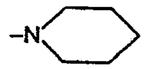 | 1.5179 |
| 163 | 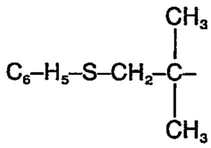 | $CH_3$ | 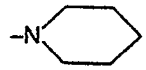 | 1.5445 |
| 164 | 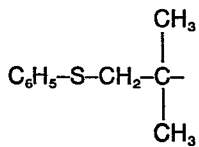 | $CH_3$ | 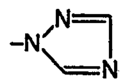 | 1.5568 |
| 165 | $CH_3-(CH_2)_8-$ | $CH_3$ | $-N<^{(CH_2)_3-CH_3}_{(CH_2)_3-CH_3}$ | 1.4461 |
| 166 | $CH_3-(CH_2)_8-$ | $CH_3-(CH_2)_8-$ | $-N<^{(CH_2)_3-CH_3}_{(CH_2)_3-CH_3}$ | Öl |
| 167 | 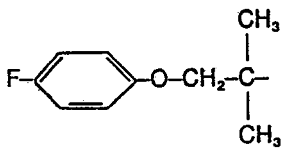 | $CH_3$ | $-N<^{(CH_2)_3-CH_3}_{(CH_2)_3-CH_3}$ | 1.4702 |
| 168 | 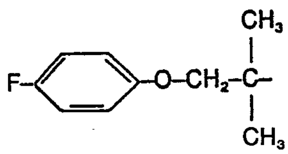 | $CH_3$ | 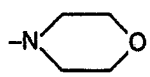 | 1.4921 |
| 169 | 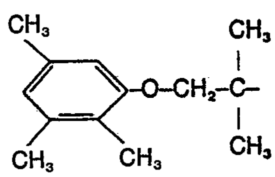 | $CH_3$ | 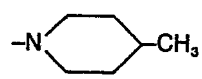 | 1.5019 |
| 170 | 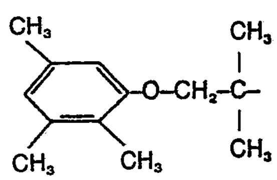 | $CH_3$ | 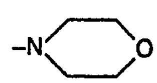 | 1.5034 |
| 171 | 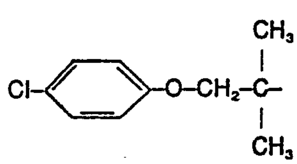 | $CH_3$ | 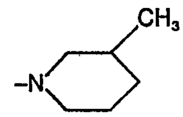 | 1.5045 |
| 172 | 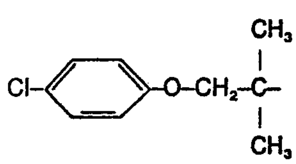 | $CH_3$ | 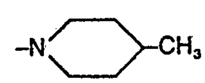 | 1.5040 |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 173 | 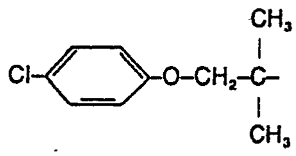 | $CH_3$ | 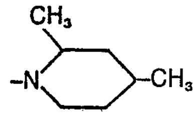 | 1.5031 |
| 174 | 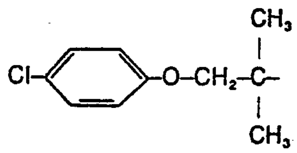 | $CH_3$ | 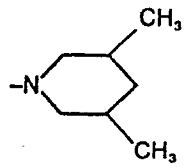 | 1.5000 |
| 175 | 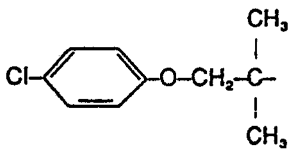 | $CH_3$ | 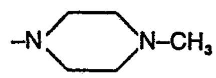 | 1.5082 |
| 176 | 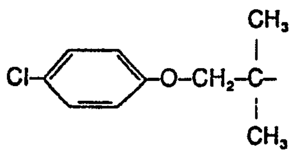 | $CH_3$ | 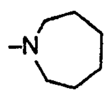 | 1.5079 |
| 177 | 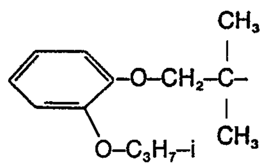 | $CH_3$ | 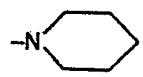 | 1.5087 |
| 178 | 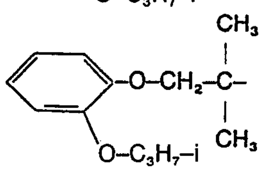 | $CH_3$ | 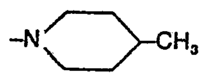 | 1.5095 |
| 179 | 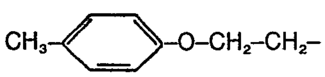 | $CH_3$ | 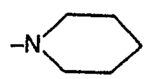 | 1.5117 |
| 180 | 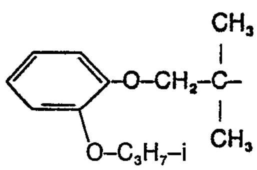 | $CH_3$ | 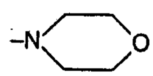 | 1.5060 |
| 181 | 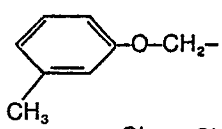 | t-$C_4H_9$ | 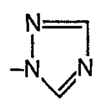 | 1.5072 |
| 182 | 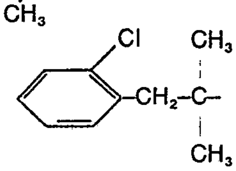 | $CH_3$ | 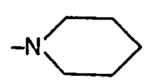 | 1.5239 |
| 183 | 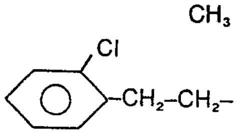 | $CH_3$ | 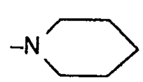 | 1.5212 |
| 184 | 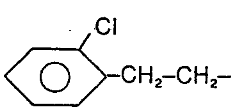 | $CH_3$ | 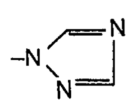 | 1.5299 |

| Beispiel Nr. | $R^1$ | $R^2$ | 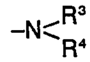 | Schmp. (°C) oder Brechungsindex ($n_D^{20}$) |
|---|---|---|---|---|
| 185 | 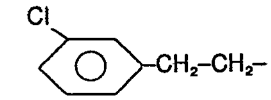 | $CH_3$ | 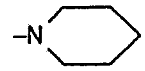 | 1.5282 |
| 186 | 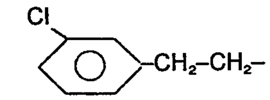 | $CH_3$ | 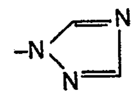 | 1.5386 |
| 187 | 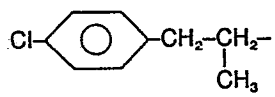 | $CH_3$ | 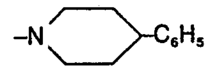 | 1.5506 |
| 188 | 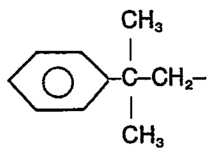 | $CH_3$ | 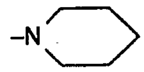 | 1.5149 |
| 189 | 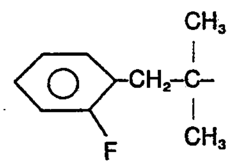 | $CH_3$ | 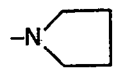 | 1.5086 |
| 190 | 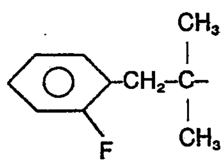 | $CH_3$ | 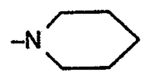 | 1.5077 |
| 191 | 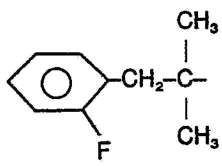 | $CH_3$ | 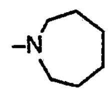 | 1.5108 |
| 192 | 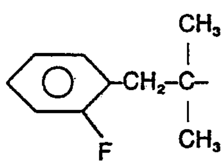 | $CH_3$ | 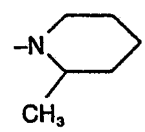 | 1.5070 |
| 193 | 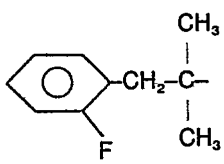 | $CH_3$ | 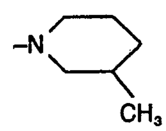 | 1.5028 |
| 194 | 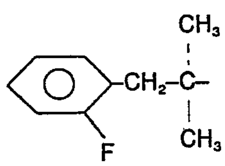 | $CH_3$ | 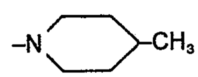 | 1.5035 |
| 195 | 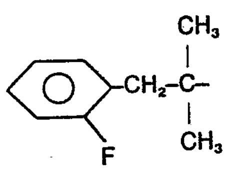 | $CH_3$ | 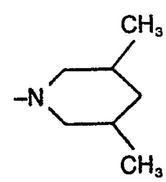 | 1.4978 |

| Beispiel Nr. | $R^1$ | $R^2$ | $-N<{R^3 \atop R^4}$ | Schmp. (°C) oder Brechungsindex ($n_D^{20}$) |
|---|---|---|---|---|
| 196 | 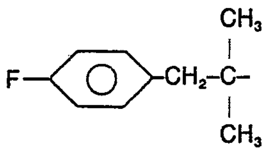 | $CH_3$ | 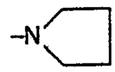 | 1.5069 |
| 197 | 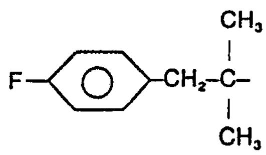 | $CH_3$ | 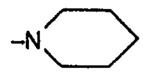 | 1.5061 |
| 198 | 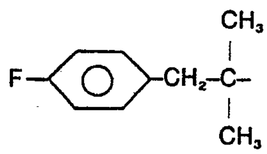 | $CH_3$ | 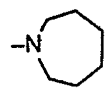 | 1.5086 |
| 199 | 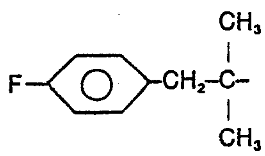 | $CH_3$ | 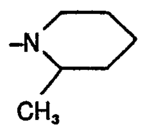 | 1.4929 |
| 200 | 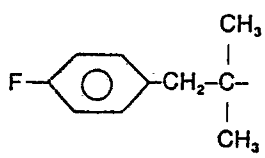 | $CH_3$ | 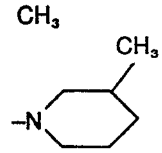 | 1.5018 |
| 201 | 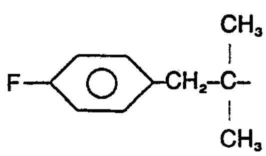 | $CH_3$ | 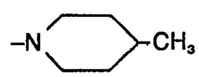 | 1.5003 |
| 202 | 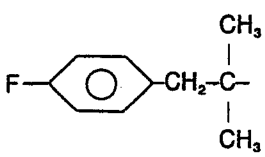 | $CH_3$ | 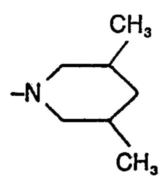 | 1.4952 |
| 203 | 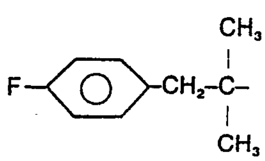 | $CH_3$ | 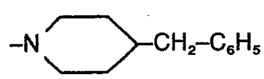 | 1.5356 |
| 204 | 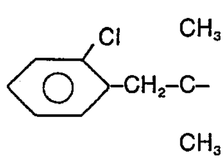 | $CH_3$ | 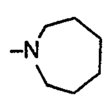 | 1.5272 |
| 205 | 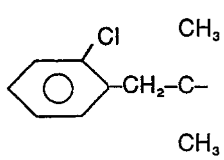 | $CH_3$ | 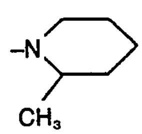 | 1.5241 |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 206 | 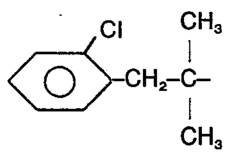 | $CH_3$ | 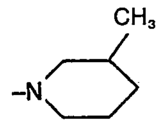 | 130 |
| 207 | 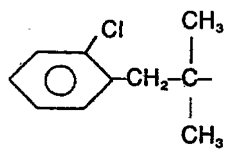 | $CH_3$ | 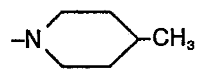 | 1.5193 |
| 208 | 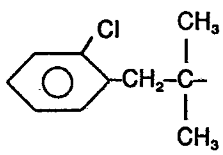 | $CH_3$ | 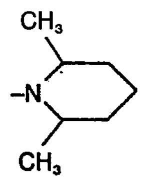 | 1.5375 |
| 209 | 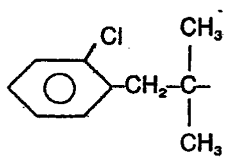 | $CH_3$ | 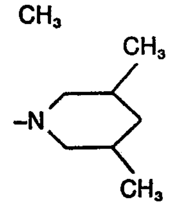 | 1.5162 |
| 210 | 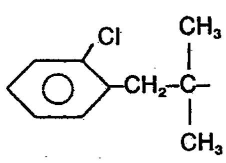 | $CH_3$ | 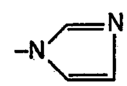 | 1.5321 |
| 211 | 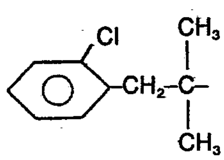 | $CH_3$ | 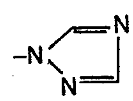 | 1.5257 |
| 212 | 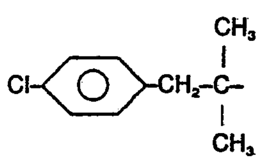 | $CH_3$ | 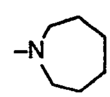 | 1.5269 |
| 213 | 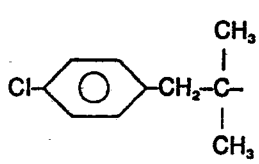 | $CH_3$ | 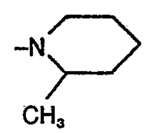 | 1.5209 |
| 214 | 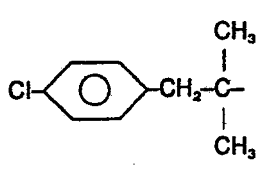 | $CH_3$ | 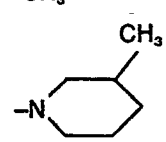 | 1.5185 |
| 215 | 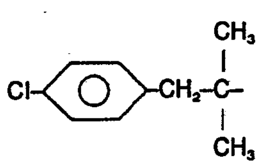 | $CH_3$ | 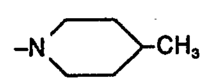 | 1.5170 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 216 | 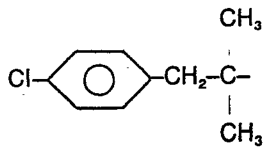 | $CH_3$ | 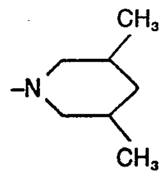 | 1.5034 |
| 217 | 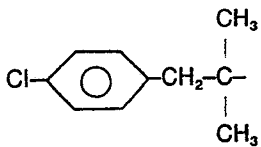 | $CH_3$ | 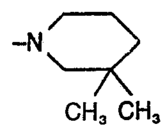 | 1.5154 |
| 218 | 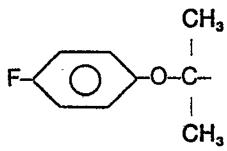 | $CH_3$ | 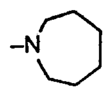 | |
| 219 | 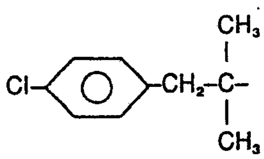 | $CH_3$ | 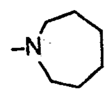 | 1.5341 |
| 220 | 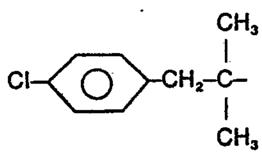 | $CH_3$ | 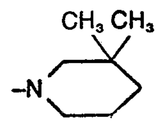 | 1.5249 |
| 221 | 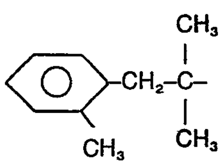 | $CH_3$ | 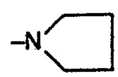 | 1.5189 |
| 222 | 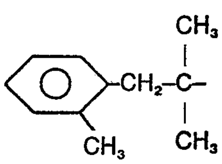 | $CH_3$ | 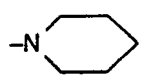 | 1.5177 |
| 223 | 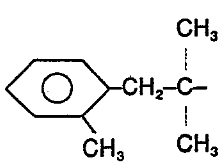 | $CH_3$ | 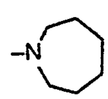 | 1.5189 |
| 224 | 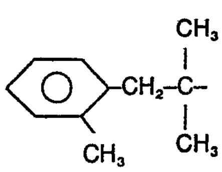 | $CH_3$ | 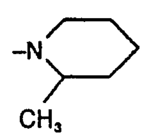 | 1.5177 |
| 225 | 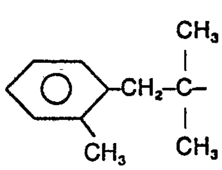 | $CH_3$ | 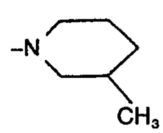 | 1.5178 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 226 | 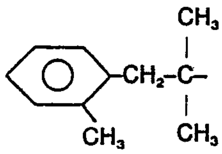 | $CH_3$ | 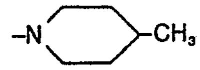 | 1.5126 |
| 227 | 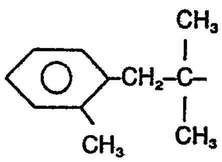 | $CH_3$ | 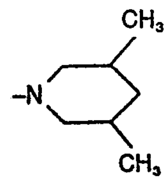 | 1.5091 |
| 228 | 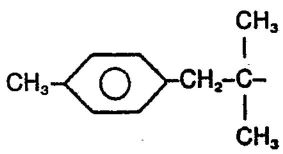 | $CH_3$ | 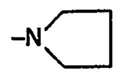 | 170 |
| 229 | 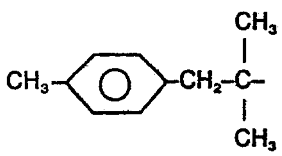 | $CH_3$ | 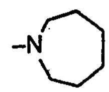 | 1.5170 |
| 230 | 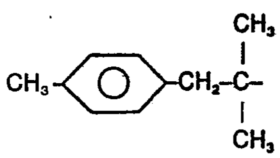 | $CH_3$ | 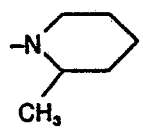 | 1.5032 |
| 231 | 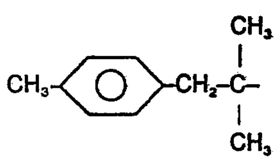 | $CH_3$ | 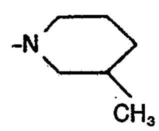 | 1.5111 |
| 232 | 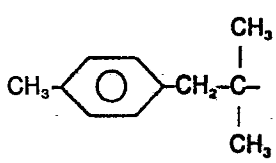 | $CH_3$ | 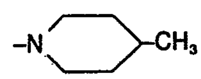 | 1.5105 |
| 233 | 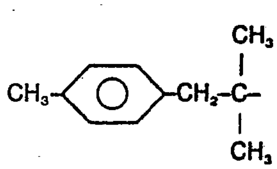 | $CH_3$ | 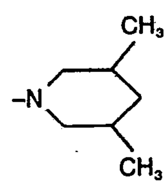 | 1.5072 |
| 234 | 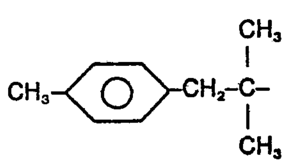 | $CH_3$ | 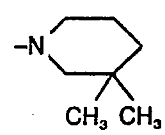 | 1.5049 |
| 235 | 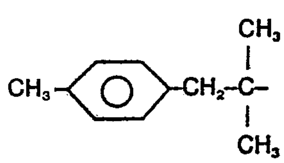 | $CH_3$ | 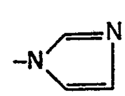 | Kp. 160°C/0,1 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 236 | 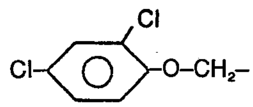 | H | 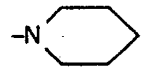 | 1.5433 |
| 237 | 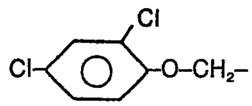 | H | 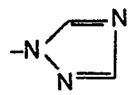 | 1.5601 |
| 238 | 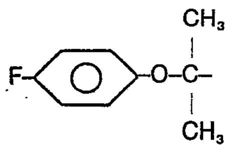 | $CH_3$ | 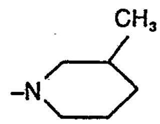 | Kp. 200°C/0,3 |
| 239 | 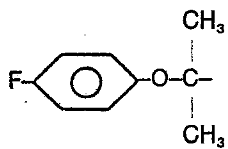 | $CH_3$ | 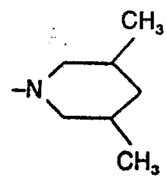 | Kp. 190°C/0,2 |
| 240 | 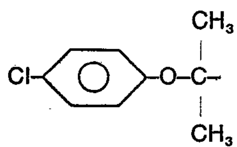 | $CH_3$ | 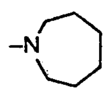 | 1.5092 |
| 241 | 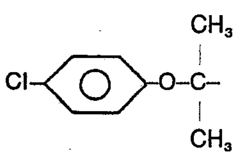 | $CH_3$ | 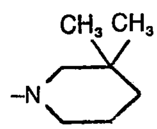 | 1.5062 |
| 242 | 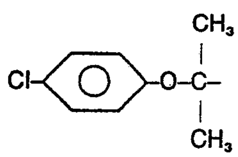 | $CH_3$ | 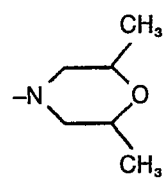 | 1.5076 |
| 243 | 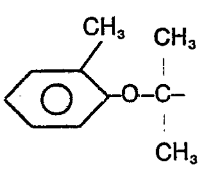 | $CH_3$ | 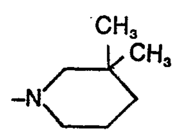 | 1.5039 |
| 244 | 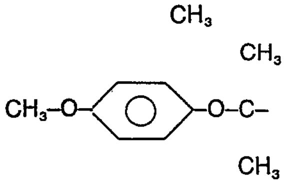 | $CH_3$ | 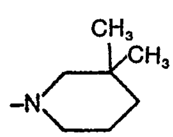 | 1.5047 |
| 245 | 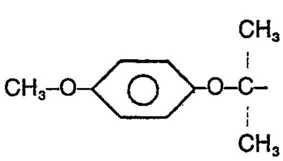 | $CH_3$ | 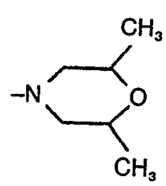 | 1.5067 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 246 | 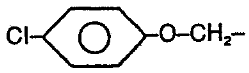 | $C(CH_3)_3$ | $-N<^{CH_3}_{CH_3}$ | 1.5042 |
| 247 | 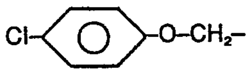 | $C(CH_3)_3$ | $-N<^{C_2H_5}_{C_2H_5}$ | 1.4987 |
| 248 | 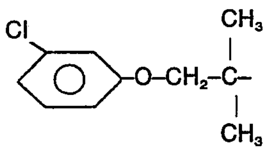 | $CH_3$ | 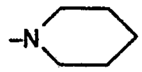 | 1.5073 |
| 249 | 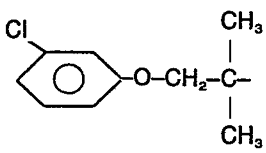 | $CH_3$ | 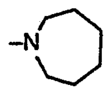 | 1.4995 |
| 250 | 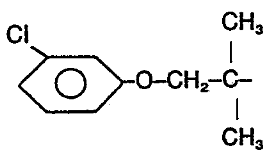 | $CH_3$ | 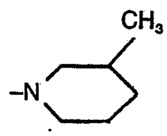 | 1.5073 |
| 251 | 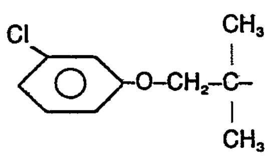 | $CH_3$ | 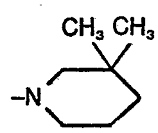 | 1.4995 |
| 252 | 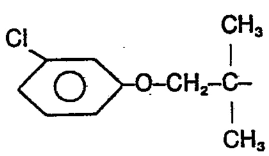 | $CH_3$ | 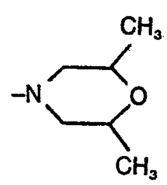 | 1.4970 |
| 253 | 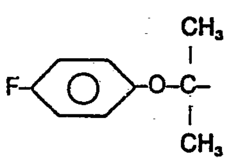 | $CH_3$ | 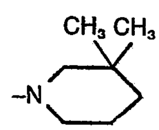 | |
| 254 | 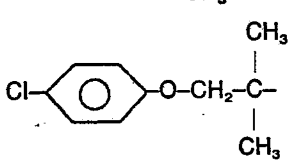 | $CH_3$ | 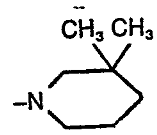 | 1.5105 |
| 255 | 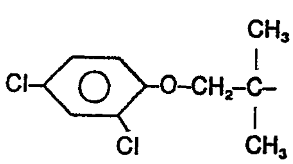 | $CH_3$ | 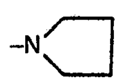 | 1.5016 |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 256 | 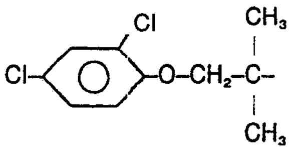 | $CH_3$ | 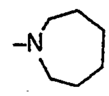 | 1.5076 |
| 257 | 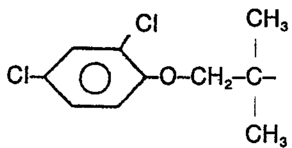 | $CH_3$ | 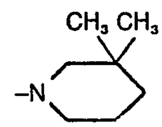 | 1.5044 |
| 258 | 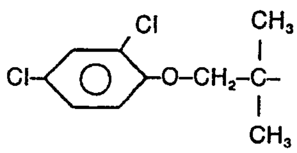 | $CH_3$ | 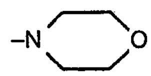 | 1.5064 |
| 259 | 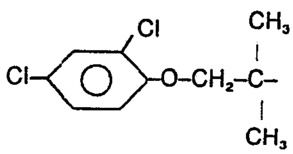 | $CH_3$ | 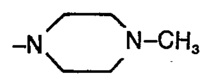 | 1.5023 |
| 260 | 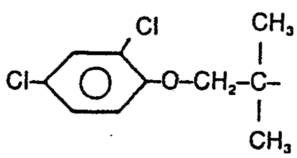 | $CH_3$ | 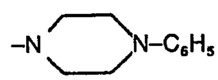 | 1.5092 |
| 261 | 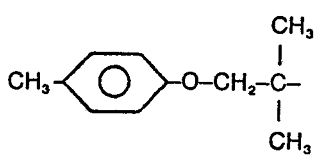 | $CH_3$ | 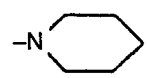 | Öl |
| 262 | 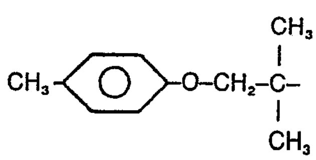 | $CH_3$ | 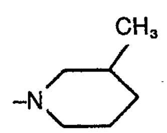 | Öl |
| 263 | 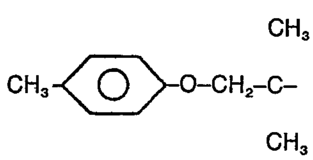 | $CH_3$ | 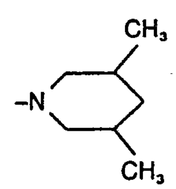 | Öl |
| 264 | 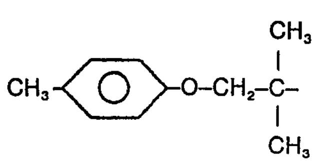 | $CH_3$ | 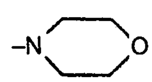 | Öl |
| 265 | 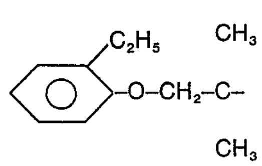 | $CH_3$ | 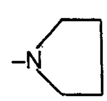 | 1.5110 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 266 | 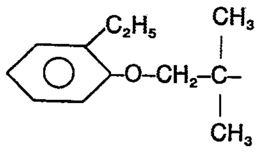 | $CH_3$ | 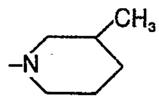 | 1.5042 |
| 267 | 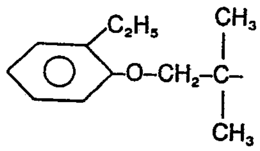 | $CH_3$ | 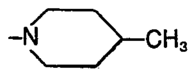 | 1.5067 |
| 268 | 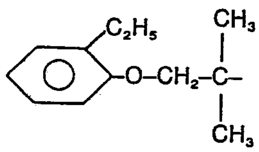 | $CH_3$ | 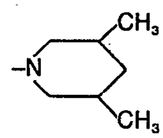 | 1.5047 |
| 269 | 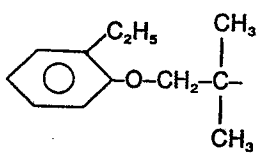 | $CH_3$ | 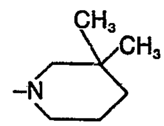 | 1.5071 |
| 270 | 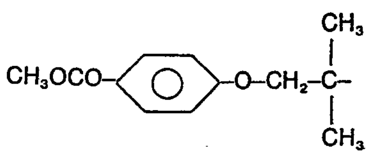 | $CH_3$ | 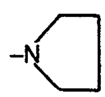 | 1.5085 |
| 271 | 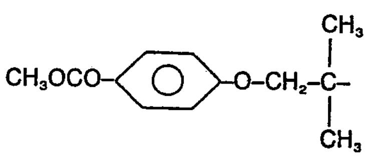 | $CH_3$ | 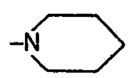 | 1.5043 |
| 272 | 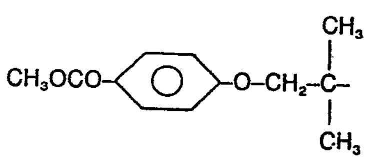 | $CH_3$ | 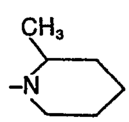 | 1.5061 |
| 273 | 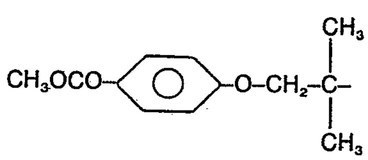 | $CH_3$ | 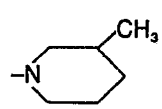 | 1.5039 |
| 274 | 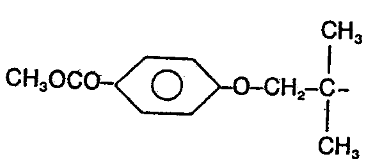 | $CH_3$ | 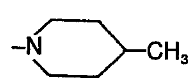 | 1.5049 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 275 | 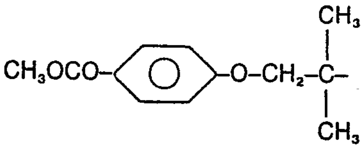 | $CH_3$ | 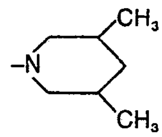 | 1.5064 |
| 276 | 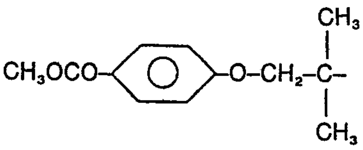 | $CH_3$ | 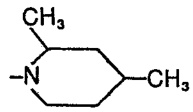 | 1.5051 |
| 277 | 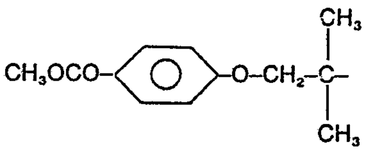 | $CH_3$ | 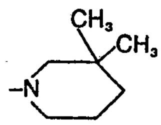 | 1.5047 |
| 278 | 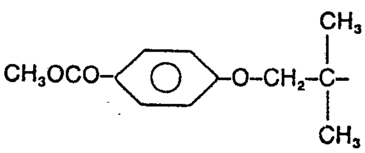 | $CH_3$ | 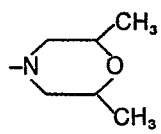 | 1.5067 |
| 279 | 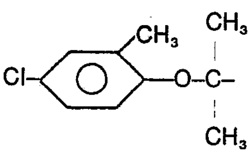 | $CH_3$ | 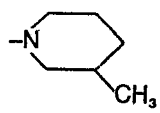 | Kp. 210°C/0,1 |
| 280 | 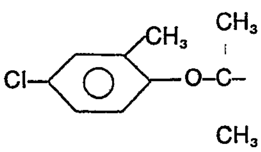 | $CH_3$ | 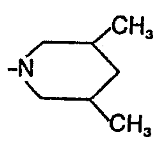 | Kp. 210°C/0,2 |
| 281 | 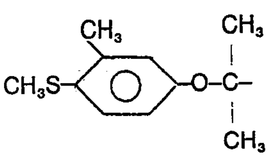 | $CH_3$ | 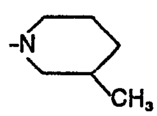 | 1.5027 |
| 282 | 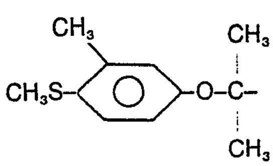 | $CH_3$ | 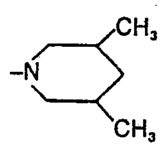 | 1.5041 |
| 283 | 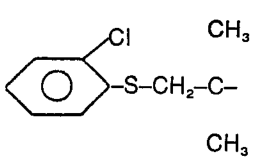 | $CH_3$ | 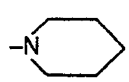 | 1.5422 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 284 | 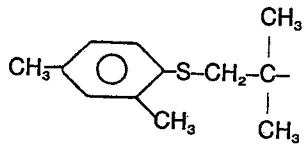 | $CH_3$ | 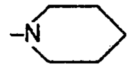 | 1.5457 |
| 285 | 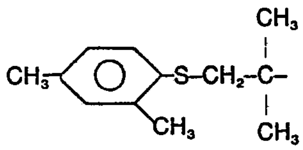 | $CH_3$ | 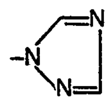 | 1.5305 |
| 286 | 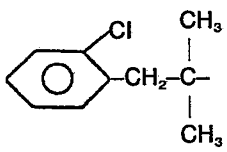 | $CH_3$ | 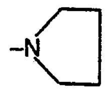 | 1.5269 |
| 287 | 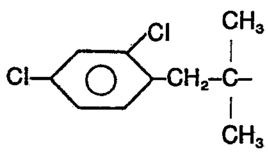 | $CH_3$ | 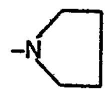 | 1.5346 |
| 288 | 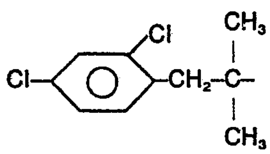 | $CH_3$ | 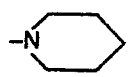 | 1.5340 |
| 289 | 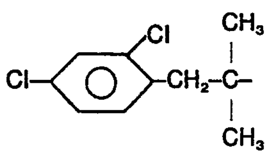 | $CH_3$ | 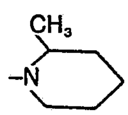 | Kp. 220° C/0,2 |
| 290 | 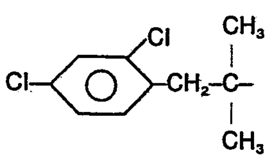 | $CH_3$ | 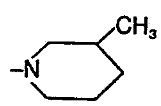 | 1.5293 |
| 291 | 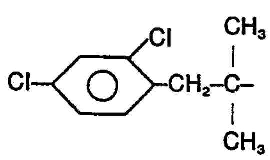 | $CH_3$ | 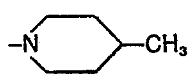 | 1.5286 |
| 292 | 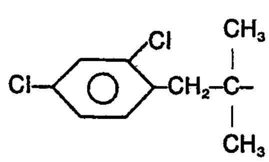 | $CH_3$ | 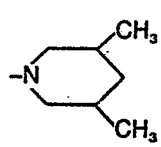 | 1.5251 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 293 | 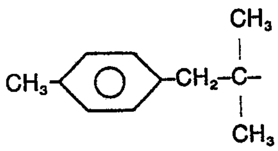 | $CH_3$ | 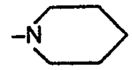 | 1.5150 |
| 294 | 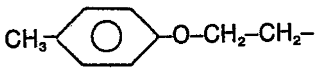 | $CH_3$ | 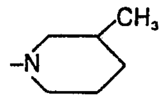 | 1.5133 |
| 295 | 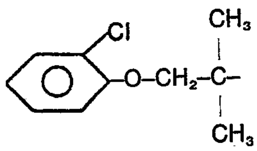 | $CH_3$ | 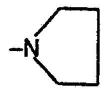 | 1.5064 |
| 296 | 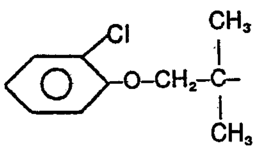 | $CH_3$ | 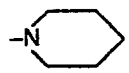 | 1.5082 |
| 297 | 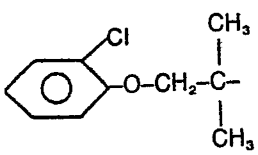 | $CH_3$ | 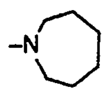 | 1.5090 |
| 298 | 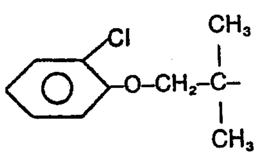 | $CH_3$ | 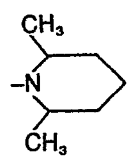 | 1.5076 |
| 299 | 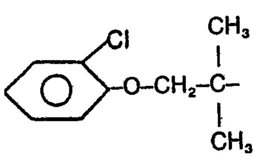 | $CH_3$ | 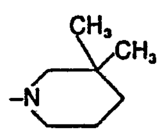 | 1.5045 |
| 300 | 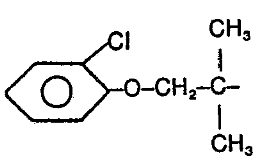 | $CH_3$ | 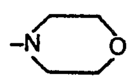 | 1.5089 |
| 301 | 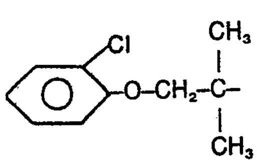 | $CH_3$ | 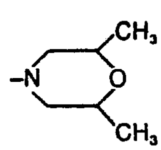 | 1.5062 |

| Beispiel Nr. | $R^1$ | $R^2$ | $-N<\begin{smallmatrix}R^3\\R^4\end{smallmatrix}$ | Schmp. (°C) oder Brechungsindex ($n_D^{20}$) |
|---|---|---|---|---|
| 302 | 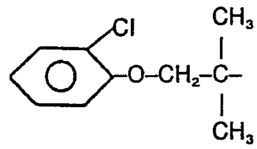 | $CH_3$ | 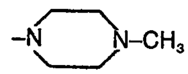 | 1.5021 |
| 303 | 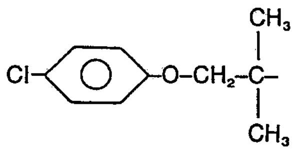 | $CH_3$ | 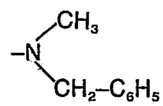 | 1.5171 |
| 304 | 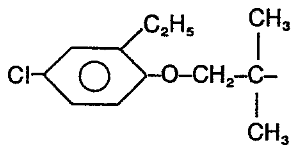 | $CH_3$ | 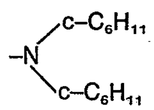 | 1.5085 |
| 305 | 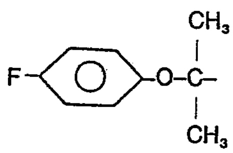 | $CH_3$ | 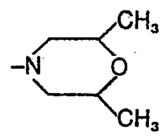 | |
| 306 | 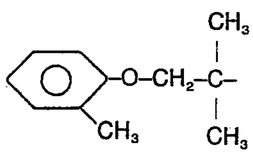 | $CH_3$ | 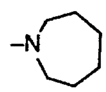 | 1.4995 |
| 307 | 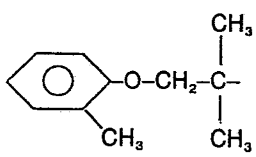 | $CH_3$ | 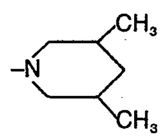 | 1.4909 |
| 308 | 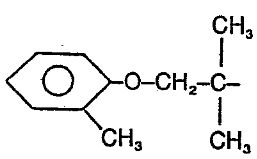 | $CH_3$ | 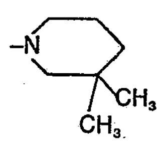 | 1.4908 |
| 309 | 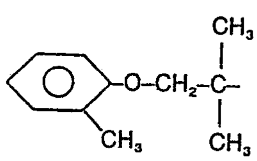 | $CH_3$ | 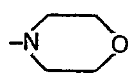 | 1.5002 |
| 310 | 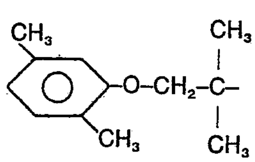 | $CH_3$ | 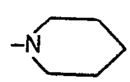 | 1.4980 |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 311 | 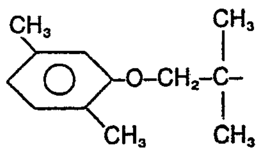 | $CH_3$ | 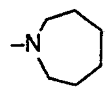 | 1.4941 |
| 312 | 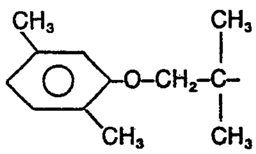 | $CH_3$ | 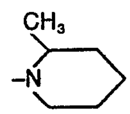 | 1.4953 |
| 313 | 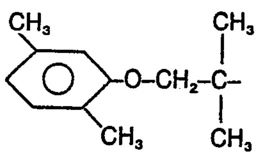 | $CH_3$ | 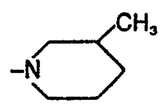 | 1.4939 |
| 314 | 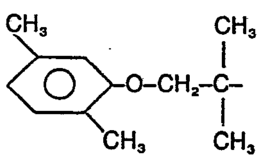 | $CH_3$ | 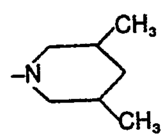 | 1.4902 |
| 315 | 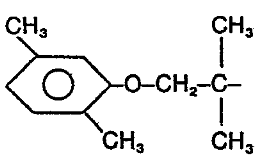 | $CH_3$ | 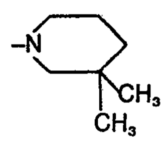 | 1.4900 |
| 316 | 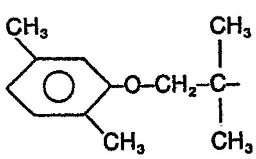 | $CH_3$ | 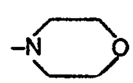 | 1.4979 |
| 317 | 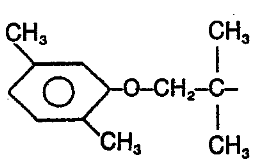 | $CH_3$ | 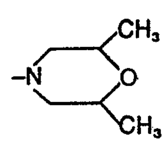 | 1.4905 |
| 318 | 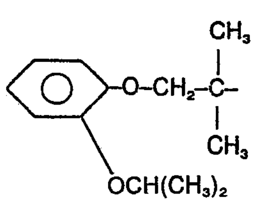 | $CH_3$ | 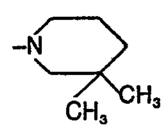 | 1.5041 |
| 319 | 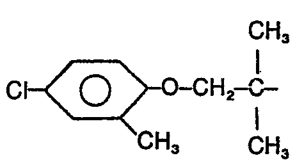 | $CH_3$ | 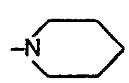 | 1.5058 |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 320 | 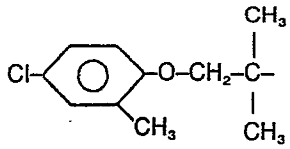 | $CH_3$ | 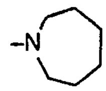 | 1.5068 |
| 321 | 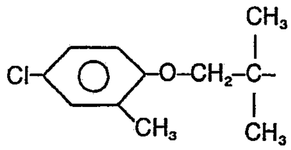 | $CH_3$ | 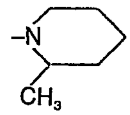 | 1.5031 |
| 322 | 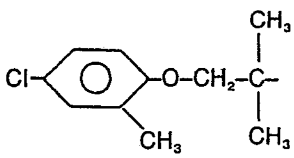 | $CH_3$ | 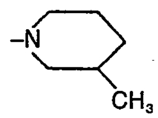 | 1.5021 |
| 323 | 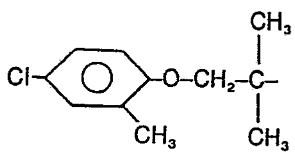 | $CH_3$ | 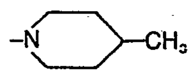 | 1.5012 |
| 324 | 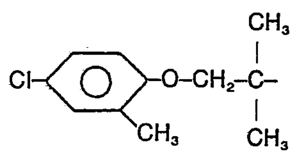 | $CH_3$ | 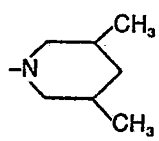 | 1.4984 |
| 325 | 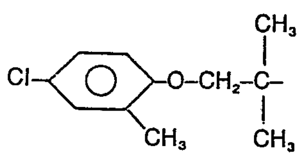 | $CH_3$ | 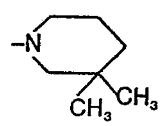 | 1.5069 |
| 326 | 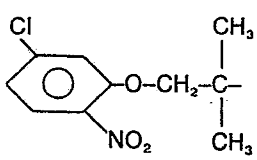 | $CH_3$ | 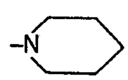 | Öl |
| 327 | 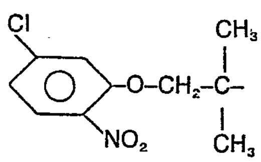 | $CH_3$ | 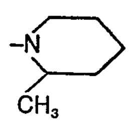 | Öl |
| 328 | 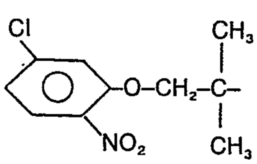 | $CH_3$ | 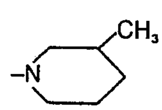 | Öl |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 329 | 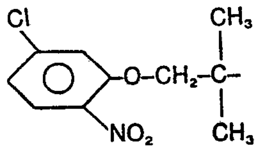 | $CH_3$ | 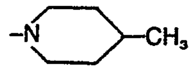 | Öl |
| 330 | 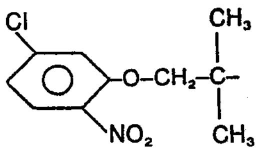 | $CH_3$ | 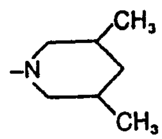 | Öl |
| 331 | 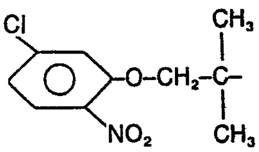 | $CH_3$ | 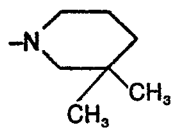 | Öl |
| 332 | 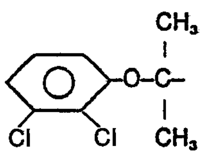 | $CH_3$ | 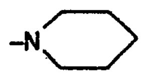 | |
| 333 | 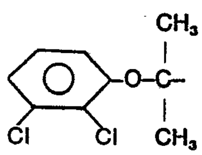 | $CH_3$ | 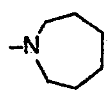 | |
| 334 | 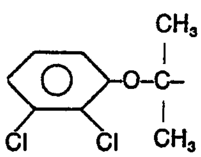 | $CH_3$ | 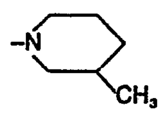 | |
| 335 | 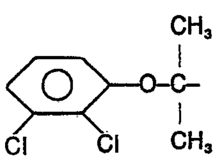 | $CH_3$ | 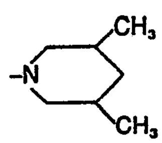 | |
| 336 | 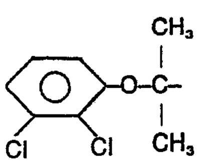 | $CH_3$ | 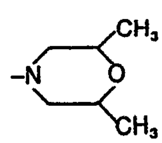 | |
| 337 | 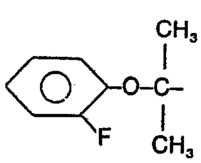 | $CH_3$ | 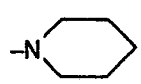 | |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 338 | 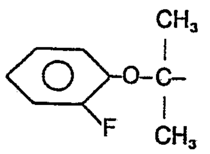 | $CH_3$ | 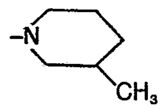 | |
| 339 | 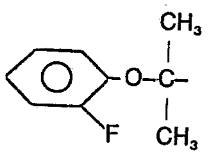 | $CH_3$ | 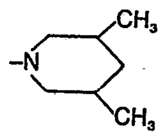 | |
| 340 | 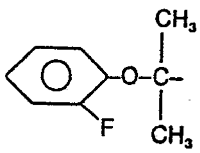 | $CH_3$ | 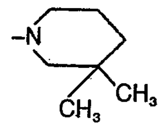 | |
| 341 | 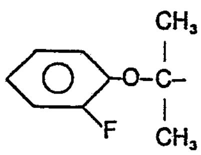 | $CH_3$ | 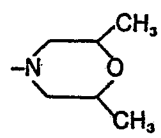 | |
| 342 | 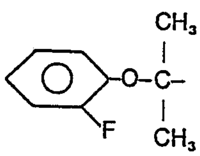 | $CH_3$ | 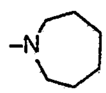 | |
| 343 | 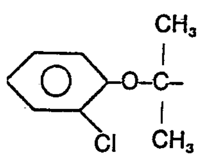 | $CH_3$ | 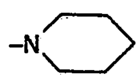 | |
| 344 | 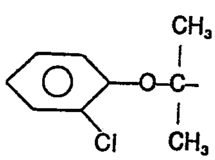 | $CH_3$ | 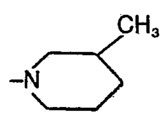 | |
| 345 | 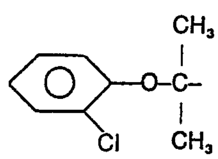 | $CH_3$ | 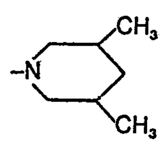 | |
| 346 | 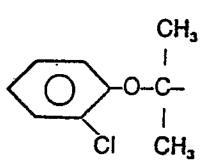 | $CH_3$ | 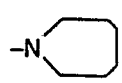 | |

| Beispiel Nr. | $R^1$ | $R^2$ | $-N<\genfrac{}{}{0pt}{}{R^3}{R^4}$ | Schmp. (°C) oder Brechungsindex ($n_D^{20}$) |
|---|---|---|---|---|
| 347 | 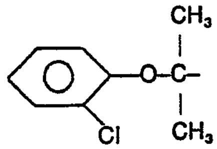 | $CH_3$ | 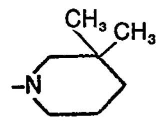 | |
| 348 | 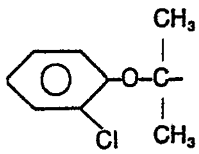 | $CH_3$ | 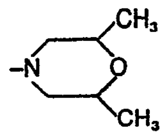 | |
| 349 | 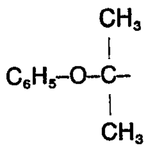 | $CH_3$ | 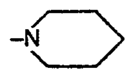 | |
| 350 | 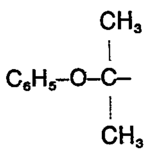 | $CH_3$ | 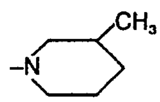 | |
| 351 | 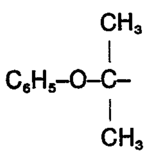 | $CH_3$ | 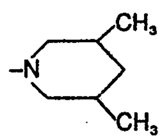 | |
| 352 | 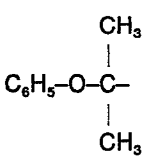 | $CH_3$ | 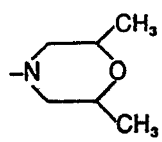 | |
| 353 | 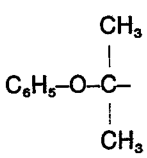 | $CH_3$ | 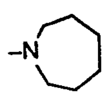 | |
| 354 | 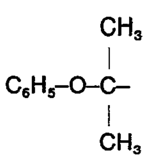 | $CH_3$ | 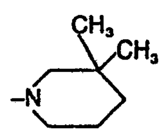 | |
| 355 | 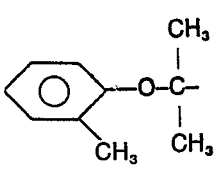 | $CH_3$ | 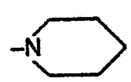 | |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 356 | 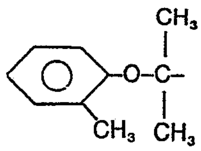 | $CH_3$ | 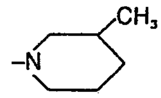 | |
| 357 | 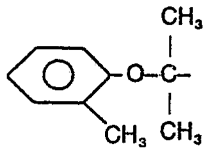 | $CH_3$ | 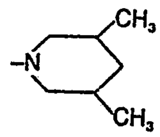 | |
| 358 | 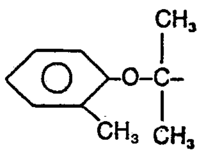 | $CH_3$ | 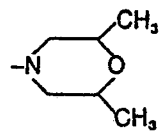 | |
| 359 | 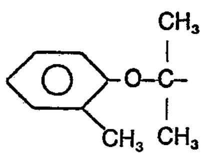 | $CH_3$ | 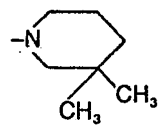 | |
| 360 | 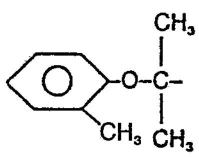 | $CH_3$ | 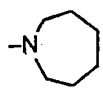 | |
| 361 | 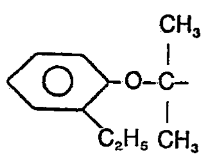 | $CH_3$ | 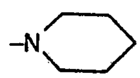 | |
| 362 | 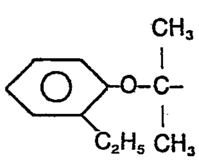 | $CH_3$ | 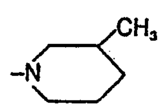 | |
| 363 | 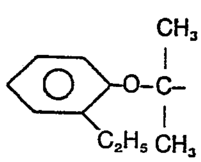 | $CH_3$ | 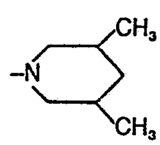 | |
| 364 | 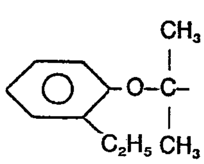 | $CH_3$ | 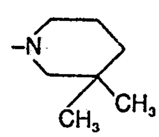 | |

| Beispiel Nr. | $R^1$ | $R^2$ | $-N<{R^3 \atop R^4}$ | Schmp. (°C) oder Brechungsindex ($n_D^{20}$) |
|---|---|---|---|---|
| 365 | 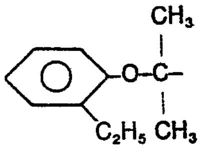 | $CH_3$ | 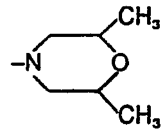 | |
| 366 | 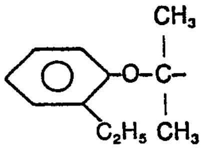 | $CH_3$ | 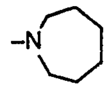 | |
| 367 | 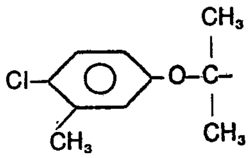 | $CH_3$ | 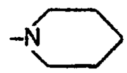 | |
| 368 | 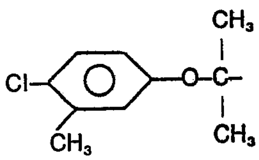 | $CH_3$ | 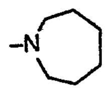 | |
| 369 | 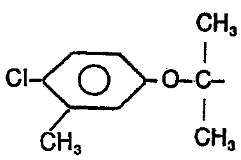 | $CH_3$ | 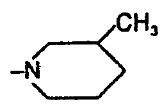 | |
| 370 | 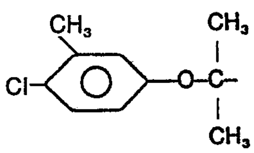 | $CH_3$ | 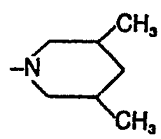 | |
| 371 | 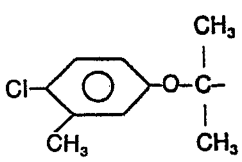 | $CH_3$ | 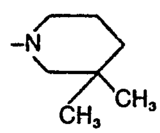 | |
| 372 | 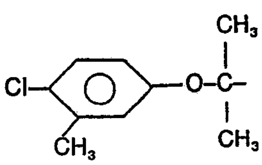 | $CH_3$ | 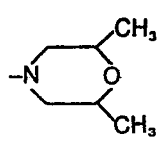 | |
| 373 | 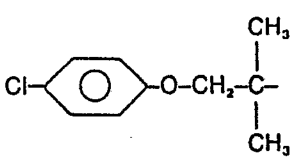 | $CH_3$ | 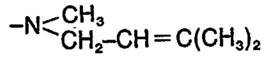 | |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 374 | 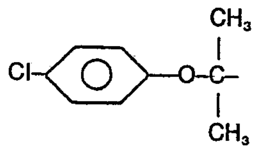 | $CH_3$ | 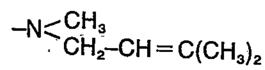 | |
| 375 | 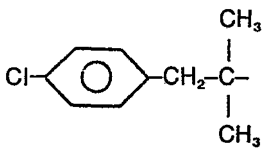 | $CH_3$ | 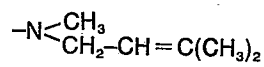 | |
| 376 | 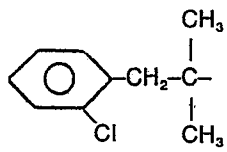 | $CH_3$ | 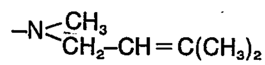 | |
| 377 | 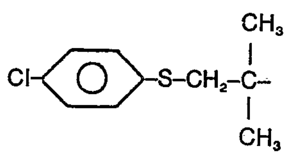 | $CH_3$ | 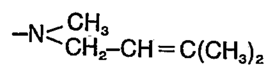 | |
| 378 | 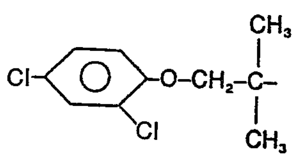 | $CH_3$ | 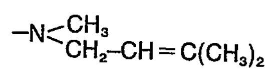 | |
| 379 | 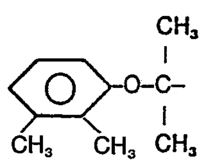 | $CH_3$ | 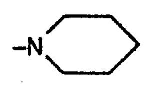 | |
| 380 | 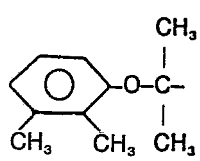 | $CH_3$ | 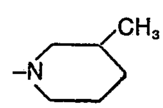 | |
| 381 | 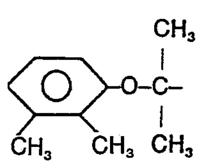 | $CH_3$ | 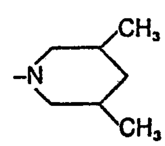 | |
| 382 | 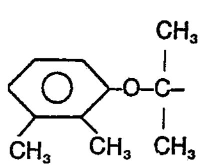 | $CH_3$ | 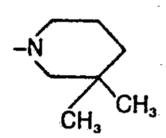 | |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 383 | 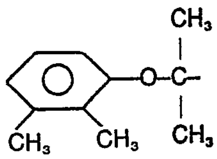 | $CH_3$ | 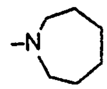 | |
| 384 | 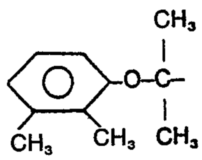 | $CH_3$ | 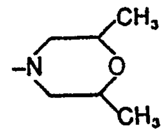 | |
| 385 | 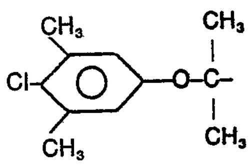 | $CH_3$ | 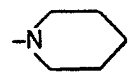 | |
| 386 | 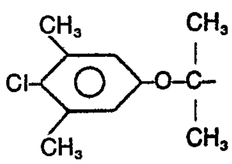 | $CH_3$ | 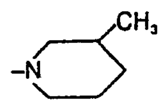 | |
| 387 | 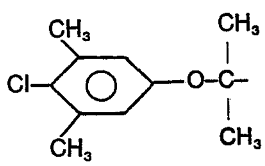 | $CH_3$ | 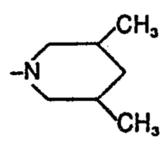 | |
| 388 | 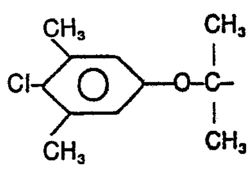 | $CH_3$ | 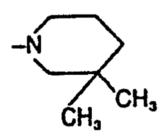 | |
| 389 | 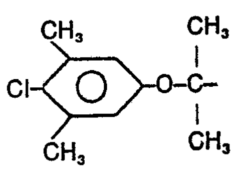 | $CH_3$ | 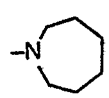 | |
| 390 | 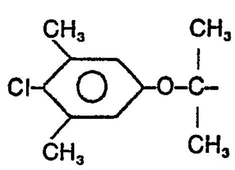 | $CH_3$ | 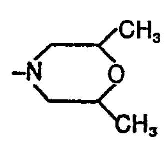 | |
| 391 | 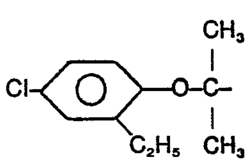 | $CH_3$ | 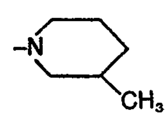 | |

| Beispiel Nr. | $R^1$ | $R^2$ | $-N<^{R^3}_{R^4}$ | Schmp. (°C) oder Brechungsindex ($n_D^{20}$) |
|---|---|---|---|---|
| 392 | 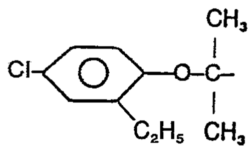 | $CH_3$ | 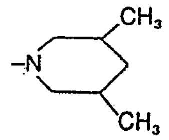 | |
| 393 | 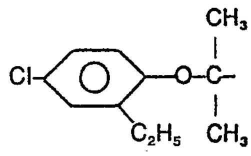 | $CH_3$ | 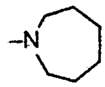 | |
| 394 | 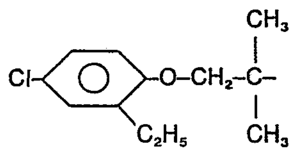 | $CH_3$ | 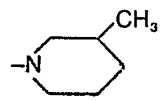 | |
| 395 | 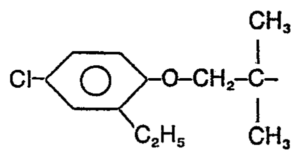 | $CH_3$ | 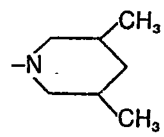 | |
| 396 | 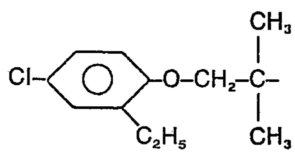 | $CH_3$ | 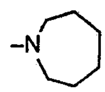 | |
| 397 | 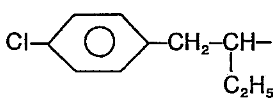 | $CH_3$ | 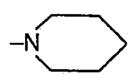 | |
| 398 | 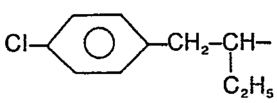 | $CH_3$ | 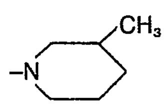 | |
| 399 | 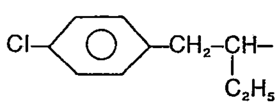 | $CH_3$ | 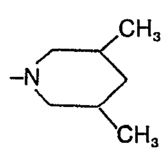 | |
| 400 | 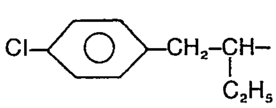 | $CH_3$ | 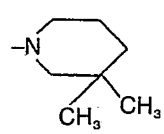 | |

| Beispiel Nr. | $R^1$ | $R^2$ | $-N<{R^3 \atop R^4}$ | Schmp. (°C) oder Brechungsindex ($n_D^{20}$) |
|---|---|---|---|---|
| 401 | 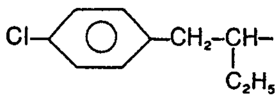 | $CH_3$ | 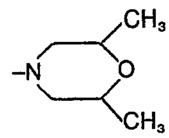 | |
| 402 | 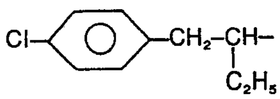 | $CH_3$ | 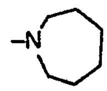 | |
| 403 | 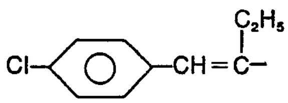 | $CH_3$ | 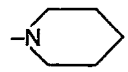 | |
| 404 | 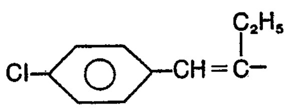 | $CH_3$ | 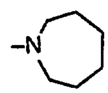 | |
| 405 | 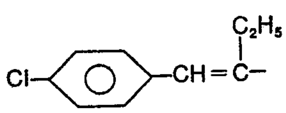 | $CH_3$ | 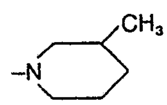 | |
| 406 | 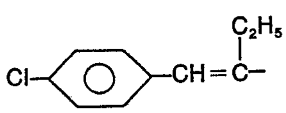 | $CH_3$ | 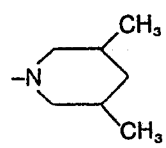 | |
| 407 | 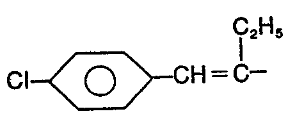 | $CH_3$ | 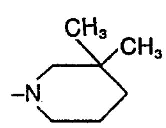 | |
| 408 | 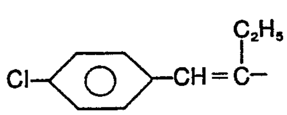 | $CH_3$ | 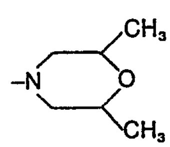 | |
| 409 | 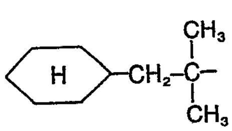 | $CH_3$ | 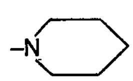 | |

| Beispiel Nr. | $R^1$ | $R^2$ | $-N\genfrac{}{}{0pt}{}{R^3}{R^4}$ | Schmp. (°C) oder Brechungsindex ($n_D^{20}$) |
|---|---|---|---|---|
| 410 | 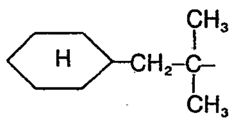 | $CH_3$ | 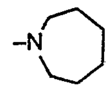 | |
| 411 | 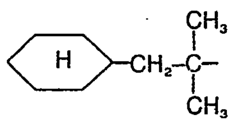 | $CH_3$ | 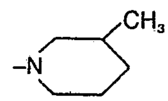 | |
| 412 | 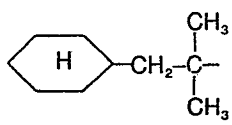 | $CH_3$ | 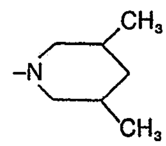 | |
| 413 | 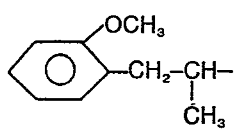 | $CH_3$ | 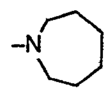 | |
| 414 | 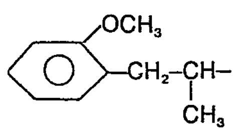 | $CH_3$ | 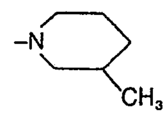 | |
| 415 | 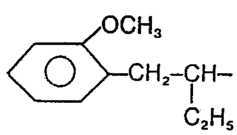 | $CH_3$ | 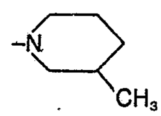 | |
| 416 | 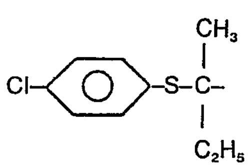 | $CH_3$ | 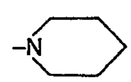 | |
| 417 | 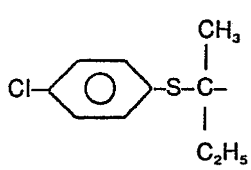 | $CH_3$ | 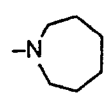 | |
| 418 | 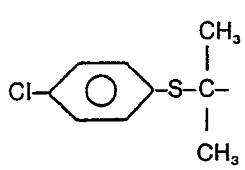 | $CH_3$ | 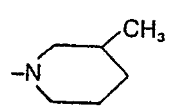 | |

| Beispiel Nr. | $R^1$ | $R^2$ | $-N{<}^{R^3}_{R^4}$ | Schmp. (°C) oder Brechungsindex ($n_D^{20}$) |
|---|---|---|---|---|
| 419 | 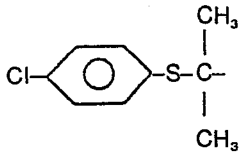 | $CH_3$ | 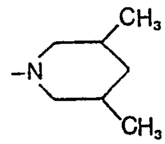 | |
| 420 | 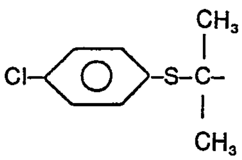 | $CH_3$ | 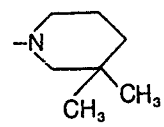 | |
| 421 | 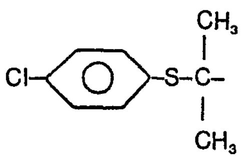 | $CH_3$ | 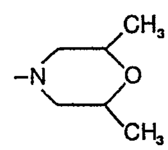 | |
| 422 | 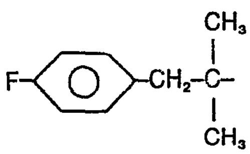 | $CH_3$ | 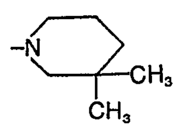 | 1.4986 |
| 423 | 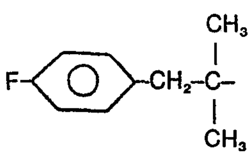 | $CH_3$ | 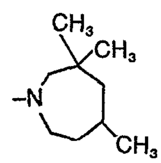 | 1.5002 |

Anwendungsbeispiele:

In den folgenden fungiziden Anwendungsbeispielen werden die nachstehend angegebenen Verbindungen als Vergleichssubstanzen eingesetzt.

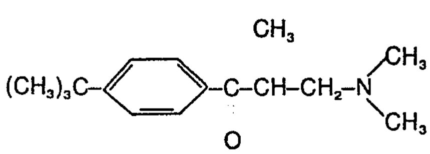 (A)

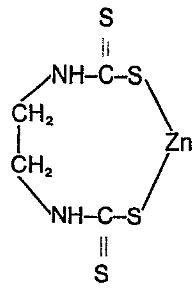 (B)

Beispiel A

Phytophthora-Test (Tomate) / protektiv

Lösungsmittel: 4,7 Gewichtsteile Aceton
Emulgator: 0,3 Gewichtsteile Alkyl-aryl-polyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Phytophthora infestans inokuliert.

Die Pflanzen werden in einer Inkubationskabine mit 100% relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 71, 78.

Tabelle A

Phytophthora-Test (Tomate)/protektiv

| Wirkstoff | Befall in % bei einer Wirkstoffkonzentration von 0,005% |
|---|---|
| 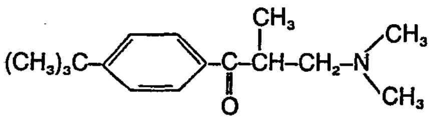 (A) (bekannt) | 60 |
| 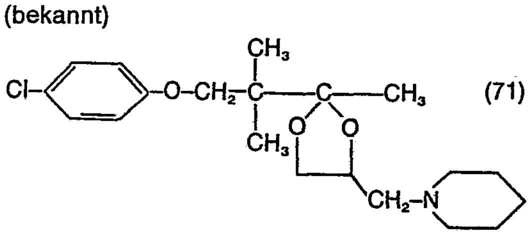 (71) × HCl | 16 |
| 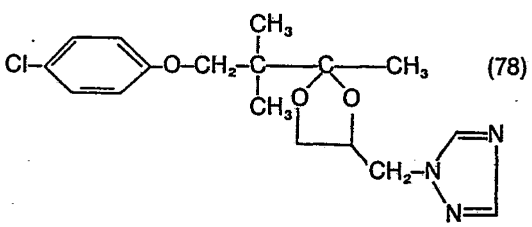 (78) | 21 |

Beispiel B

Drechslera graminea-Test (Gerste) /Saatgutbehandlung (syn. Helminthosporium gramineum)

Die Anwendung der Wirkstoffe erfolgt als Trokkenbeizmittel. Sie werden zubereitet durch Abstrecken des jeweiligen Wirkstoffes mit Gesteinsmehl zu einer feinpulvrigen Mischung, die eine gleichmässige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4 °C aus. Dabei wird die Keimung der Gerste und gegebenenfalls auch der Pilzsporen eingeleitet. Anschliessend sät man die vorgekeimte Gerste mit 2 × 50 Korn 3 cm tief in eine Standarderde und kultiviert sie im Gewächshaus bei einer Temperatur von ca. 180 °C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen in diesem Test z.B. die Verbindungen gemäss folgender Herstellungsbeispiele: 71, 74, 77, 98, 104.

Tabelle B

Drechsera graminea-Test (Gerste)/Saatgutbehandlung (syn. Helminthosporium gramineum)

| Wirkstoff | Wirkstoffaufwandmenge in mg/kg Saatgut | Kranke Pflanzen in % der insgesamt aufgelaufenen Pflanzen |
|---|---|---|
| unbehandelt | - | 24,0 |
| 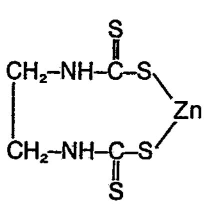 (B) (bekannt) | 600 | 19,0 |

Tabelle B (Fortsetzung)
Drechsera graminea-Test (Gerste)/Saatgutbehandlung (syn. Helminthosporium gramineum)

| Wirkstoff | Wirkstoffaufwand-menge in mg/kg Saatgut | Kranke Pflanzen in % der insgesamt aufgelaufenen Pflanzen |
|---|---|---|
| 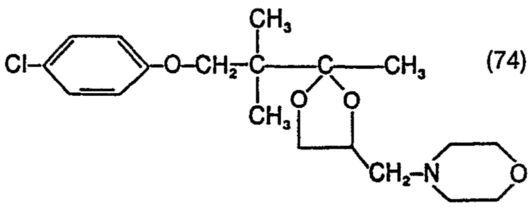 (74) | 500 | 1,1 |
| 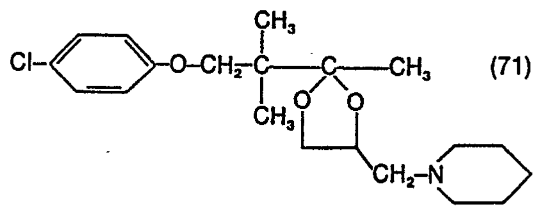 (71) × HCl | 500 | 1,2 |
| 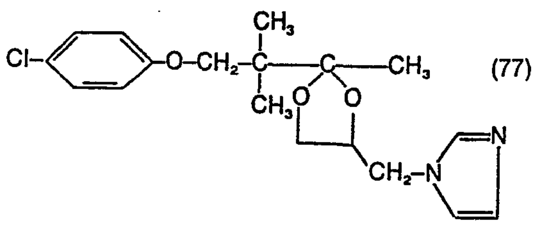 (77) | 500 | 3,5 |
| 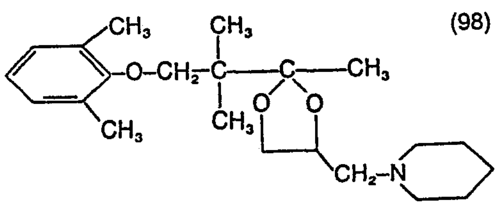 (98) | 500 | 2,5 |
| 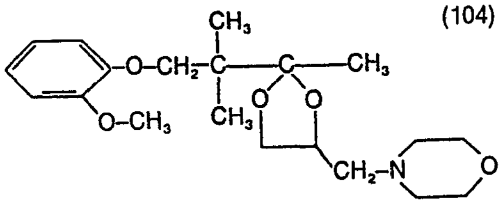 (104) | 500 | 3,6 |

Beispiel C

Erysiphe-Test (Gerste) / protektiv
Lösungsmittel: 100 Gewichtsteile Dimethylformamid
Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 29, 71, 72, 73, 74, 75, 76, 78, 89, 90, 92, 94, 95, 97, 98, 100, 102, 104, 105, 106, 107, 113.

Tabelle C
Erysiphe-Test (Gerste)/protektiv

| Wirkstoff | Wirkstoffkonzentration in der Spritzbrühe in Gew.% | Krankheitsbefall in % der unbehandelten Kontrolle |
|---|---|---|
| 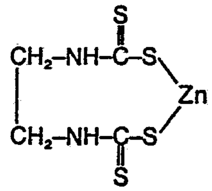 (B) (bekannt) | 0,025 | 100 |
| 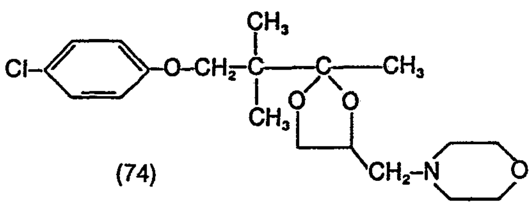 (74) | 0,025 | 3,8 |
| 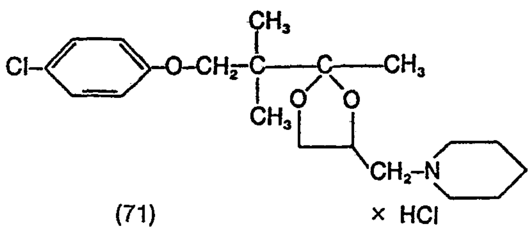 (71) × HCl | 0,025 | 3,8 |
| 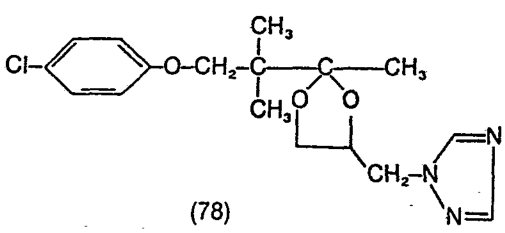 (78) | 0,025 | 25,0 |
| 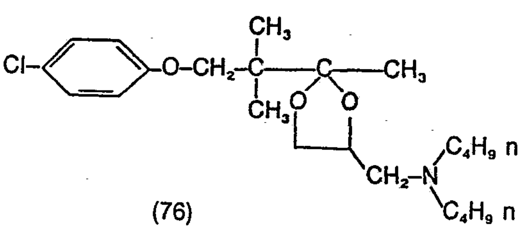 (76) | 0,025 | 12,5 |

Tabelle C (Fortsetzung)
Erysiphe-Test (Gerste)/protektiv

| Wirkstoff | Wirkstoffkonzentration in der Spritzbrühe in Gew.% | Krankheitsbefall in % der unbehandelten Kontrolle |
|---|---|---|
| 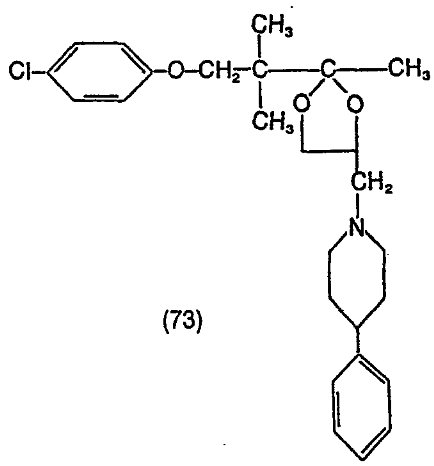 (73) | 0,025 | 12,5 |
| 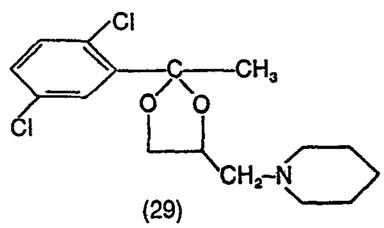 (29) | 0,025 | 25,0 |
| 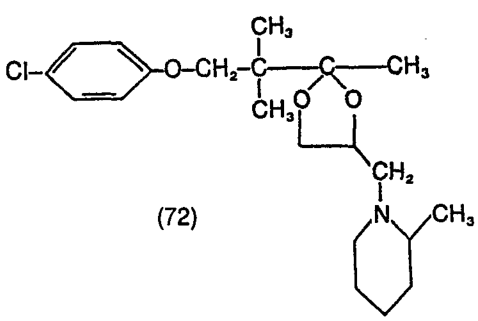 (72) | 0,025 | 12,5 |
| 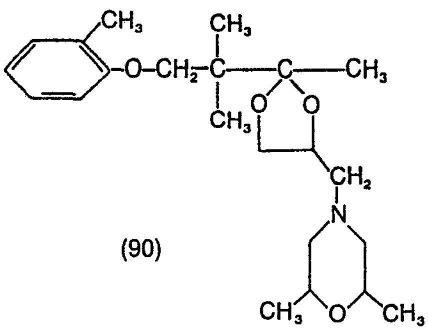 (90) | 0,025 | 12,5 |

Tabelle C (Fortsetzung)
Erysiphe-Test (Gerste)/protektiv

| Wirkstoff | Wirkstoffkonzentration in der Spritzbrühe in Gew.% | Krankheitsbefall in % der unbehandelten Kontrolle |
|---|---|---|
| 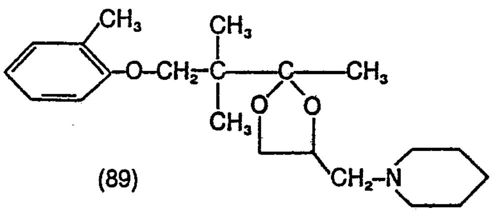 (89) | 0,025 | 0,0 |
| 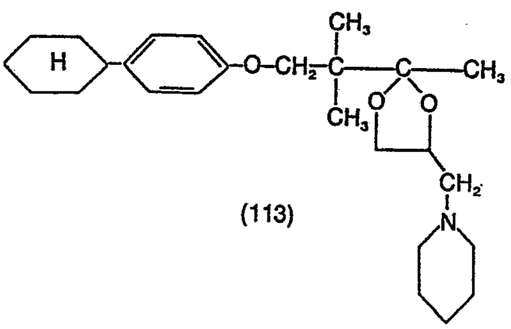 (113) | 0,025 | 25,0 |
| 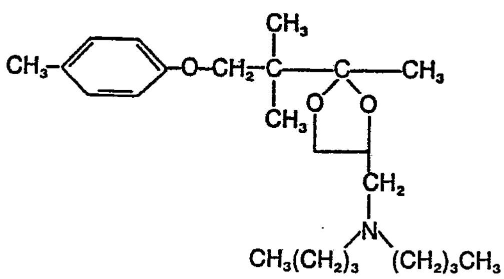 (92) | 0,025 | 12,5 |
| 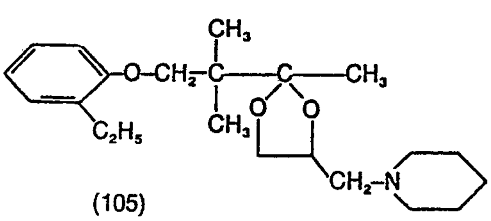 (105) | 0,025 | 8,8 |
| 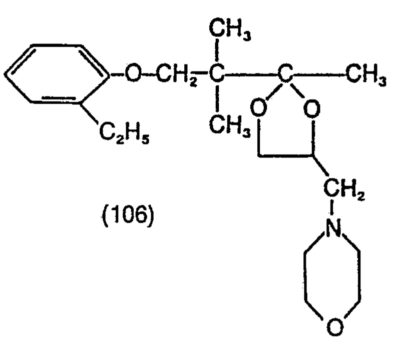 (106) | 0,025 | 12,5 |

Tabelle C (Fortsetzung)
Erysiphe-Test (Gerste)/protektiv

| Wirkstoff | Wirkstoffkonzentration in der Spritzbrühe in Gew.% | Krankheitsbefall in % der unbehandelten Kontrolle |
|---|---|---|
| 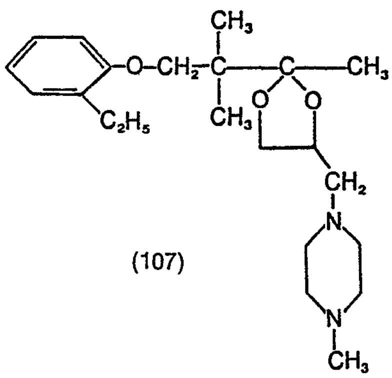 (107) | 0,025 | 16,3 |
| 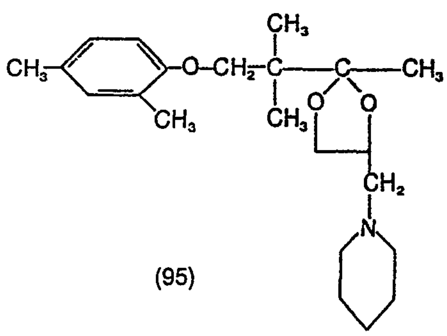 (95) | 0,025 | 10.0 |
| 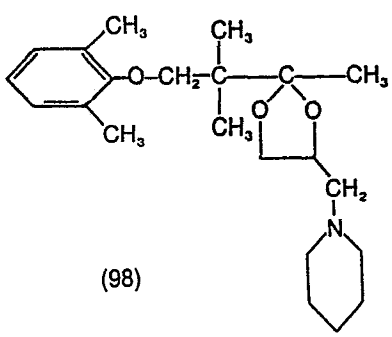 (98) | 0,025 | 3,8 |
| 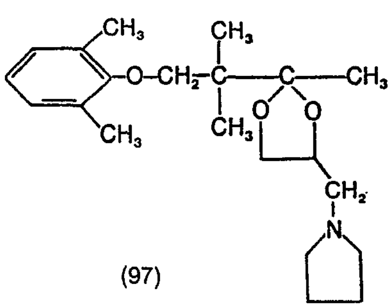 (97) | 0,025 | 12,5 |

Tabelle C (Fortsetzung)
Erysiphe-Test (Gerste)/protektiv

| Wirkstoff | Wirkstoffkonzentration in der Spritzbrühe in Gew.% | Krankheitsbefall in % der unbehandelten Kontrolle |
|---|---|---|
| 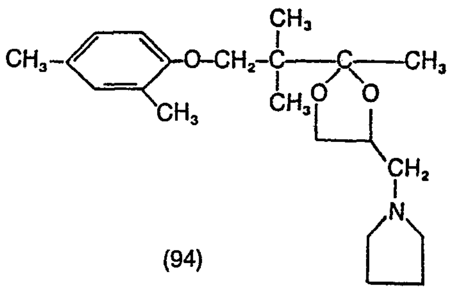 (94) | 0,025 | 12,5 |
| 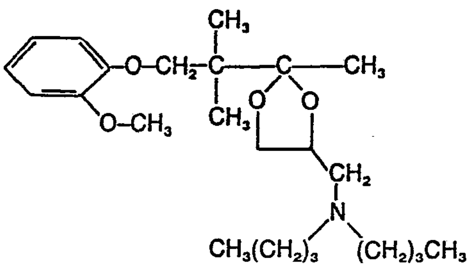 (100) | 0,025 | 33,8 |
| 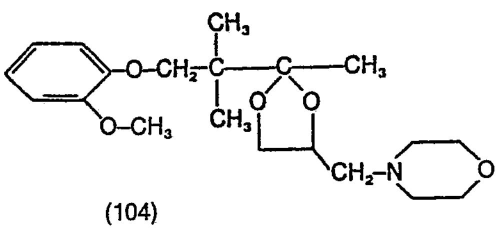 (104) | 0,025 | 21,3 |
| 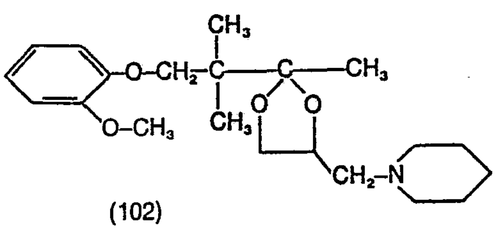 (102) | 0,025 | 12,5 |
| 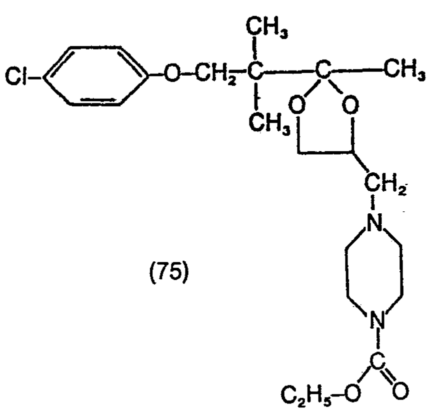 (75) | 0,025 | 33,8 |

**Patentansprüche**

1. Verwendung von substituierten Aminoketalen der Formel (I)

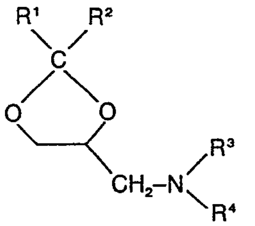

(I)

in welcher

$R^1$ für geradkettiges oder verzweigtes Alkyl mit 1 bis 18 Kohlenstoffatomen; für geradkettiges oder verzweigtes Halogenalkyl mit 1 bis 12 Kohlenstoffatomen und 1 bis 5 Halogenatomen, für geradkettiges oder verzweigtes Cyanalkyl mit 1 bis 4 Kohlenstoffatomen im Alkylteil, für gegebenenfalls ein- oder mehrfach, gleich oder verschieden substituiertes Arylalkyl, Aryloxyalkyl, Aralkyloxyalkyl, Arylthioalkyl, Arylsulfinylalkyl, Arylsulfonylalkyl mit jeweils 1 bis 6 Kohlenstoffatomen in jedem Alkylteil und 6 bis 10 Kohlenstoffatomen im Arylteil steht, wobei als Arylsubstituenten jeweils in Frage kommen:

Halogen, Cyano, Nitro; Alkyl, Alkoxy, Alkylthio mit jeweils 1 bis 4 Kohlenstoffatomen; Halogenalkyl, Halogenalkoxy und Halogenalkylthio mit jeweils 1 oder 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor oder durch Alkyl mit 1 bis 4 Kohlenstoffatomen substituiertes Phenyl oder Phenoxy; und für Cycloalkylalkyl mit 1 bis 4 Kohlenstoffatomen im Alkylteil und 3 bis 7 Kohlenstoffatomen im gegebenenfalls ein- oder mehrfach durch Alkyl mit 1 bis 4 Kohlenstoffatomen substituierten Cycloalkylteil, ferner für geradkettiges oder verzweigtes Alkenyl oder Alkinyl mit jeweils 2 bis 6 Kohlenstoffatomen, für gegebenenfalls einfach oder mehrfach, gleich oder verschieden durch Alkyl mit 1 bis 4 Kohlenstoffatomen substituiertes Cycloalkyl mit 3 bis 7 Kohlenstoffatomen, oder für jeweils gegebenenfalls einfach oder mehrfach, gleich oder verschieden substituiertes Aryl mit 6 bis 10 Kohlenstoffatomen, sowie Arylalkenyl mit 2 bis 6 Kohlenstoffatomen im Alkenylteil und 6 bis 10 Kohlenstoffatomen im Arylteil steht, wobei als Arylsubstituenten jeweils in Frage kommen:

Halogen, Cyano, Nitro; Alkyl, Alkoxy, Alkylthio mit jeweils 1 bis 4 Kohlenstoffatomen; Halogenalkyl, Halogenalkoxy und Halogenalkylthio mit jeweils 1 oder 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy;

$R^2$ die Bedeutung hat wie $R^1$, von diesem gleich oder verschieden sein kann und ausserdem für Wasserstoff steht,

$R^3$ für geradkettiges oder verzweigtes Alkyl mit 1 bis 12 Kohlenstoffatomen, für geradkettiges oder verzweigtes Alkenyl mit 3 bis 12 Kohlenstoffatomen, für geradkettiges oder verzweigtes Alkinyl mit 3 bis 7 Kohlenstoffatomen, für gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen sowie für jeweils gegebenenfalls einfach oder mehrfach, gleich oder verschieden substituiertes Aryl mit 6 bis 10 Kohlenstoffatomen oder Aralkyl mit 1 oder 2 Kohlenstoffatomen im Alkylteil und 6 bis 10 Kohlenstoffatomen im Arylteil, wobei als Arylsubstituenten jeweils in Frage kommen:

Halogen, Alkyl mit 1 bis 4 Kohlenstoffatomen sowie Halogenalkyl mit 1 oder 2 Kohlenstoffatomen und 1 bis 5 Halogenatomen;

$R^4$ für geradkettiges oder verzweigtes Alkyl mit 1 bis 12 Kohlenstoffatomen, für geradkettiges oder verzweigtes Alkenyl mit 3 bis 12 Kohlenstoffatomen, für geradkettiges oder verzweigtes Alkinyl mit 3 bis 7 Kohlenstoffatomen, für gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen sowie für jeweils gegebenenfalls einfach oder mehrfach, gleich oder verschieden substituiertes Aryl mit 6 bis 10 Kohlenstoffatomen oder Aralkyl mit 1 bis 2 Kohlenstoffatomen im Alkylteil und 6 bis 10 Kohlenstoffatomen im Arylteil steht, wobei als Arylsubstituenten jeweils in Frage kommen:

Halogen, Alkyl mit 1 bis 4 Kohlenstoffatomen, sowie Halogenalkyl mit 1 oder 2 Kohlenstoffatomen und 1 bis 5 Halogenatomen; oder

$R^3$ und $R^4$ zusammen mit dem Stickstoffatom, an das sie gebunden sind, für einen gegebenenfalls einfach oder mehrfach, gleich oder verschieden substituierten 5- bis 7-gliedrigen, gesättigten oder ungesättigten Heterocyclus mit 1 bis 3 Heteroatomen, vorzugsweise Stickstoff oder Sauerstoff stehen, wobei als Substituenten vorzugsweise genannt seien: Alkyl mit 1 bis 4 Kohlenstoffatomen, Alkoxycarbonyl mit 1 bis 4 Kohlenstoffatomen im Alkylteil und Phenyl, zur Bekämpfung von Schädlingen.

2. Verwendung gemäss Anspruch 1, wobei in Formel (I)

$R^1$ für geradkettiges oder verzweigtes Alkyl mit 1 bis 12 Kohlenstoffatomen, Cyclopropyl, Cyclopentyl, Cyclohexyl, für geradkettiges oder verzweigtes Halogenalkyl mit 1 bis 6 Kohlenstoffatomen und 1 bis 5 Halogenatomen oder für jeweils gegebenenfalls ein- bis dreifach, gleich oder verschieden substituiertes Phenyl, Phenylalkyl, Phenoxyalkyl, Benzyloxyalkyl, Phenylthioalkyl, Phenylsulfinylalkyl und Phenylsulfonylalkyl mit jeweils 1 bis 5 Kohlenstoffatomen im Alkylteil, sowie gegebenenfalls ein- bis dreifach, gleich oder verschieden substituiertes Phenylalkenyl mit 2 bis 4 Kohlenstoffatomen im Alkenylteil steht, wobei als Phenylsubstituenten in Frage kommen:

Fluor, Chlor, Brom, Cyano, Nitro, Methyl, Methoxy, Methylthio, Ethyl, Ethoxy, Ethylthio, n- und i-Propyl, Isopropoxy, n-, iso-, sec.- und t-Butyl, Trifluormethyl, Trifluormethoxy, Trifluormethylthio, Cyclohexyl, Methoxycarbonyl, Ethoxycarbonyl und gegebenenfalls durch Fluor, Chlor oder Methyl substituiertes Phenyl oder Phenoxy;

$R^2$ für geradkettiges oder verzweigtes Alkyl mit 1 bis 10 Kohlenstoffatomen, für gegebenenfalls ein- bis dreifach, gleich oder verschieden substituiertes Phenyl und Benzyl, wobei als Phenyl- bzw. Benzylsubstituenten jeweils in Frage kommen:

Fluor, Chlor, Brom, Cyano, Nitro, Trifluormethyl, Trifluormethoxy, Trifluormethylthio, Methoxycarbonyl und Ethoxycarbonyl, ferner für Cyclopropyl, Cyclopentyl und Cyclohexyl,

$R^3$ für Methyl, Ethyl, n- und i-propyl, n-, i-, sec.- und t-Butyl, n-Pentyl, n-Hexyl, n-Heptyl, n-Octyl, n-Nonyl, n-Decyl, n-Dodecyl, Allyl, Propargyl, 2-Butenyl, Cyclopentyl, Cyclohexyl sowie für jeweils gegebenenfalls ein- bis zweifach, gleich oder verschieden durch Fluor, Chlor, Methyl oder Trifluormethyl substituiertes Phenyl oder Benzyl steht und

$R^4$ für n- und i-Propyl, n-, i-, sec.- und t-Butyl, n-Pentyl, n-Hexyl, n-Heptyl, n-Octyl, n-Nonyl, n-Decyl, Allyl, Propargyl, 2-Butenyl, Cyclopentyl, Cyclohexyl sowie für jeweils gegebenenfalls ein- bis zweifach, gleich oder verschieden durch Fluor, Chlor, Methyl oder Trifluormethyl substituiertes Phenyl oder Benzyl steht; oder

$R^3$ und $R^4$ zusammen mit dem Stickstoffatom, an das sie gebunden sind, für jeweils gegebenenfalls ein- bis dreifach, gleich oder verschieden substituiertes 1-Pyrrolidinyl, 1-Piperidinyl, 1-Piperazinyl, 4-Morpholinyl, 1-Perhydroazepinyl, 1-Imidazolyl, 1-(1,2,4-Triazolyl) oder 1-Pyrazolyl stehen, wobei als Substituenten genannt seien:

Methyl, Ethyl, n- und i-Propyl, Phenyl, Methoxycarbonyl und Ethoxycarbonyl.

3. Verwendung gemäss Anspruch 1, wobei in Formel (I)

$R^1$ für geradkettiges oder verzweigtes Alkyl mit 1 bis 5 Kohlenstoffatomen sowie für jeweils gegebenenfalls ein- bis dreifach, gleich oder verschieden substituiertes Phenylalkenyl, Phenylalkyl, Phenoxyalkyl, Benzyloxyalkyl, Phenylthioalkyl, Phenylsulfinylalkyl und Phenylsulfonylalkyl mit jeweils bis zu 5 Kohlenstoffatomen im Alkylteil bzw. Alkenylteil steht, wobei als Phenylsubstituenten genannt seien:

Fluor, Chlor, Brom, Methyl, Ethyl, n- und i-Propyl, t-Butyl, Methoxy, Ethoxy, Isopropoxy, Cyclohexyl, Trifluormethyl, Trifluormethoxy, Trifluormethylthio, Methoxycarbonyl, Ethoxycarbonyl, Nitro, Cyano, Phenyl und Phenoxy;

$R^2$ für geradkettiges oder verzweigtes Alkyl mit 1 bis 4 Kohlenstoffatomen oder Wasserstoff steht;

$R^3$ für Methyl, Ethyl, n- und i-Propyl oder n-, i- und t-Butyl steht;

$R^4$ für Methyl, Ethyl, n- und i-Propyl, n- und i-Butyl, Pentyl oder Hexyl steht oder

$R^3$ und $R^4$ zusammen mit dem stickstoffatom, an das sie gebunden sind, für jeweils gegebenenfalls ein- bis dreifach, gleich oder verschieden substituiertes 1-Piperidinyl, 1-Perhydroazepinyl, 1-Pyrrolidinyl, 4-Morpholinyl, 1-Piperazinyl, 1-Imidazolyl und 1-(1,2,4-Triazolyl) stehen, wobei als Substituenten genannt seien:

Alkyl mit 1 bis 3 Kohlenstoffatomen, Methoxycarbonyl, Ethoxycarbonyl und Phenyl.

4. Verwendung gemäss Anspruch 1, wobei in Formel (I)

$R^1$ für die Gruppierungen

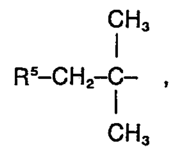 , 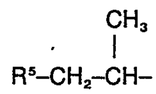 , 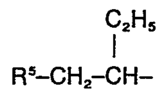 ,

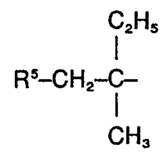 und 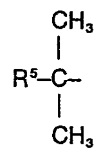 steht, wobei

$R^5$ für jeweils gegebenenfalls einfach bis dreifach, gleich oder verschieden substituiertes Phenyl, Phenoxy, Phenylthio, Phenylsulfinyl oder Phenylsulfonyl steht, wobei als Substituenten genannt seien:

Fluor, Chlor, Brom, Nitro, Cyclohexyl, geradkettiges oder verzweigtes Alkyl mit 1 bis 4 Kohlenstoffatomen, geradkettiges oder verzweigtes Alkoxy mit 1 bis 3 Kohlenstoffatomen und Alkoxycarbonyl mit 1 oder 2 Kohlenstoffatomen im Alkoxyteil,

$R^2$ für geradkettiges oder verzweigtes Alkyl mit 1 bis 4 Kohlenstoffatomen steht,

$R^3$ für geradkettiges oder verzweigtes Alkyl mit 1 bis 4 Kohlenstoffatomen steht oder für geradkettiges oder verzweigtes Alkenyl mit 3 bis 5 Kohlenstoffatomen steht,

$R^4$ für geradkettiges oder verzweigtes Alkyl mit 1 bis 4 Kohlenstoffatomen steht und

$R^3$ und $R^4$ zusammen mit dem Stickstoffatom, an das sie gebunden sind, für jeweils gegebenenfalls einfach bis dreifach, gleich oder verschieden durch Alkyl mit 1 bis 3 Kohlenstoffatomen substituierten Piperidin-, Morpholin- oder Pyrrolidinrest stehen.

5. Verwendung gemäss Anspruch 1, wobei in Formel (I)

$R^1$ für die Gruppierungen

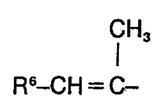 und 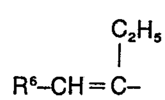 steht,

wobei

$R^6$ für jeweils gegebenenfalls einfach bis dreifach, gleich oder verschieden substituiertes Phenyl steht, wobei als Substituenten genannt seien: Fluor, Chlor, Brom, Nitro, Cyclohexyl, geradkettiges oder verzweigtes Alkyl oder Alkoxy mit je 1 bis 4 Kohlenstoffatomen und Alkoxycarbonyl mit 1 oder 2 Kohlenstoffatomen im Alkoxyteil,

$R^2$ für geradkettiges oder verzweigtes Alkyl mit 1 bis 4 Kohlenstoffatomen,

$R^3$ für geradkettiges oder verzweigtes Alkyl mit 1 bis 4 Kohlenstoffatomen oder für geradkettiges oder verzweigtes Alkenyl mit 3 bis 5 Kohlenstoffatomen steht,

$R^4$ für geradkettiges oder verzweigtes Alkyl mit 1 bis 4 Kohlenstoffatomen steht und

$R^3$ und $R^4$ zusammen mit dem Stickstoffatom, an das sie gebunden sind, für jeweils gegebenenfalls einfach bis dreifach, gleich oder verschieden durch Alkyl mit 1 bis 3 Kohlenstoffatomen substituierten Piperidin-, Morpholin- oder Pyrrolidinrest stehen.

6. Verfahren zur Bekämpfung von Schädlingen, dadurch gekennzeichnet, dass man substituierte Aminoketale der Formel (I) gemäss Anspruch 1 auf Schädlinge oder ihren Lebensraum einwirken lässt.

7. Substituierte Aminoketale der Formel (IA)

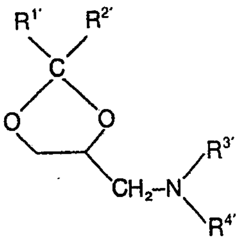

(IA)

wobei

$R^{1'}$ für geradkettiges oder verzweigtes Alkyl mit 6 bis 18 Kohlenstoffatomen; für geradkettiges oder verzweigtes Halogenalkyl mit 2 bis 12 Kohlenstoffatomen und 1 bis 5 Halogenatomen, für geradkettiges oder verzweigtes Cyanalkyl mit 1 bis 4 Kohlenstoffatomen im Alkylteil, für ein- oder mehrfach, gleich oder verschieden substituiertes Benzyl, für gegebenenfalls ein- oder mehrfach, gleich oder verschieden substituiertes Arylalkyl mit 2 bis 6 Kohlenstoffatomen im Alkylteil und 6 bis 10 Kohlenstoffatomen im Arylteil, für Aryloxyalkyl, für Aralkyloxyalkyl, für Arylthioalkyl, für Arylsulfinylalkyl, für Arylsulfonylalkyl mit jeweils 1 bis 6 Kohlenstoffatomen in jedem Alkylteil und 6 bis 10 Kohlenstoffatomen im Arylteil, wobei als Arylsubstituenten jeweils in Frage kommen:
Halogen, Cyano, Nitro; Alkyl, Alkoxy, Alkylthio mit jeweils 1 bis 4 Kohlenstoffatomen; Halogenalkyl, Halogenalkoxy und Halogenalkylthio mit jeweils 1 oder 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy; und für Cycloalkylalkyl mit 1 bis 4 Kohlenstoffatomen im Alkylteil und 3 bis 7 Kohlenstoffatomen im gegebenenfalls ein- oder mehrfach durch Alkyl mit 1 bis 4 Kohlenstoffatomen substituierten Cycloalkylteil; ferner für geradkettiges oder verzweigtes Alkenyl mit 2 bis 5 Kohlenstoffatomen, für geradkettiges oder verzweigtes Alkinyl mit 2 bis 6 Kohlenstoffatomen; für ein- bis mehrfach, gleich oder verschieden durch Alkyl mit 1 bis 4 Kohlenstoffatomen substituiertes Cycloalkyl mit 3 bis 7 Kohlenstoffatomen und für Arylalkenyl mit 2 bis 6 Kohlenstoffatomen im Alkenylteil und 6 bis 10 Kohlenstoffatomen im Arylteil steht, wobei als Arylsubstituenten jeweils in Frage kommen:
Halogen, Cyano, Nitro; Alkyl, Alkoxy, Alkylthio mit jeweils 1 bis 4 Kohlenstoffatomen; Halogenalkoxy und Halogenalkylthio mit jeweils 1 oder 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy;

$R^{2'}$ für Wasserstoff, für geradkettiges oder verzweigtes Alkyl mit 1 bis 18 Kohlenstoffatomen; für geradkettiges oder verzweigtes Halogenalkyl mit 1 bis 12 Kohlenstoffatomen und 1 bis 5 Halogenatomen, für geradkettiges oder verzweigtes Cyanalkyl mit 1 bis 4 Kohlenstoffatomen im Alkylteil, für gegebenenfalls ein- oder mehrfach, gleich oder verschieden substituiertes Arylalkyl, Aryloxyalkyl, für Aralkyloxyalkyl, Arylthioalkyl, Arylsulfinylalkyl oder für Arylsulfonylalkyl mit jeweils 1 bis 6 Kohlenstoffatomen in jedem Alkylteil und 6 bis 10 Kohlenstoffatomen im Arylteil, wobei als Arylsubstituenten jeweils in Frage kommen:
Halogen, Cyano, Nitro; Alkyl, Alkoxy, Alkylthio mit jeweils 1 bis 4 Kohlenstoffatomen; Halogenalkyl, Halogenalkoxy und Halogenalkylthio mit jeweils 1 oder 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy;
und für Cycloalkylalkyl mit 1 bis 4 Kohlenstoffatomen im Alkylteil und 3 bis 7 Kohlenstoffatomen im gegebenenfalls ein- oder mehrfach durch Alkyl mit 1 bis 4 Kohlenstoffatomen substituierten Cycloalkylteil, ferner für geradkettiges oder verzweigtes Alkenyl oder Alkinyl mit jeweils 2 bis 6 Kohlenstoffatomen für gegebenenfalls einfach oder mehrfach, gleich oder verschieden durch Alkyl mit 1 bis 4 Kohlenstoffatomen substituiertes Cycloalkyl mit 3 bis 7 Kohlenstoffatomen, oder für jeweils gegebenenfalls einfach oder mehrfach, gleich oder verschieden substituiertes Aryl mit 6 bis 10 Kohlenstoffatomen, sowie Arylalkenyl mit 2 bis 6 Kohlenstoffatomen im Alkenylteil und 6 bis 10 Kohlenstoffatomen im Arylteil steht, wobei als Arylsubstituenten jeweils in Frage kommen:
Halogen, Cyano, Nitro; Alkyl, Alkoxy, Alkylthio mit jeweils 1 bis 4 Kohlenstoffatomen; Halogenalkyl, Halogenalkoxy und Halogenalkylthio mit jeweils 1 bis 2 Kohlenstoffatomen und 1 bis 5 Halogenatomen, Cycloalkyl mit 5 bis 7 Kohlenstoffatomen, Alkoxycarbonyl mit 1 bis 4 Kohlenstoffatomen im Alkylteil und gegebenenfalls durch Halogen, insbesondere Fluor oder Chlor, oder durch Alkyl mit 1 bis 4 Kohlenstoffatomen substituiertes Phenyl oder Phenoxy;

$R^{3'}$ für geradkettiges oder verzweigtes Alkyl mit 1 bis 12 Kohlenstoffatomen, für geradkettiges oder

verzweigtes Alkenyl mit 3 bis 12 Kohlenstoffatomen, für geradkettiges oder verzweigtes Alkinyl mit 3 bis 7 Kohlenstoffatomen, für gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen sowie für jeweils gegebenenfalls einfach oder mehrfach, gleich oder verschieden substituiertes Aryl mit 6 bis 10 Kohlenstoffatomen oder Aralkyl mit 1 oder 2 Kohlenstoffatomen im Alkylteil und 6 bis 10 Kohlenstoffatomen im Arylteil steht, wobei als Arylsubstituenten jeweils in Frage kommen:
Halogen, Alkyl mit 1 bis 4 Kohlenstoffatomen sowie Halogenalkyl mit 1 oder 2 Kohlenstoffatomen und 1 bis 5 Halogenatomen;

$R^{4'}$ für geradkettiges oder verzweigtes Alkyl mit 1 bis 12 Kohlenstoffatomen, für geradkettiges oder verzweigtes Alkenyl mit 3 bis 12 Kohlenstoffatomen, für geradkettiges oder verzweigtes Alkinyl mit 3 bis 7 Kohlenstoffatomen, für gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen sowie für jeweils gegebenenfalls einfach oder mehrfach, gleich oder verschieden substituiertes Aryl mit 6 bis 10 Kohlenstoffatomen oder Aralkyl mit 1 bis 2 Kohlenstoffatomen im Alkylteil und 6 bis 10 Kohlenstoffatomen im Arylteil steht, wobei als Arylsubstituenten jeweils in Frage kommen:
Halogen, Alkyl mit 1 bis 4 Kohlenstoffatomen, sowie Halogenalkyl mit 1 oder 2 Kohlenstoffatomen und 1 bis 5 Halogenatomen; oder

$R^{3'}$ und $R^{4'}$ zusammen mit dem Stickstoffatom, an das sie gebunden sind, für einen gegebenenfalls einfach oder mehrfach, gleich oder verschieden substituierten 5- bis 7-gliedrigen, gesättigten oder ungesättigten Heterocyclus mit 1 bis 3 Stickstoff- und/oder Sauerstoffatomen stehen, wobei als Substituenten genannt seien:
Alkyl mit 1 bis 4 Kohlenstoffatomen, Alkoxycarbonyl mit 1 bis 4 Kohlenstoffatomen im Alkylteil und Phenyl, ausgenommen Verbindungen, in welchen
(a) $R^{1'}$ für Ethylbutylcarbinol (3-Heptyl), $R^{2'}$ für Phenyl und $R^{3'}$ und $R^{4'}$ für Ethyl stehen und
(b) $R^{1'}$ für Methyl, $R^{2'}$ für $n-C_9H_{19}-$ und $R^{3'}$ und $R^{4'}$ für Ethyl stehen.

8. Substituierte Aminoketale gemäss Anspruch 7, wobei

$R^{1'}$ für geradkettiges oder verzweigtes Alkyl mit 6 bis 12 Kohlenstoffatomen; für geradkettiges oder verzweigtes Halogenalkyl mit 2 bis 6 Kohlenstoffatomen und 1 bis 5 Halogenatomen, für Cyanmethyl oder Cyanethyl, für ein- bis dreifach, gleich oder verschieden substituiertes Benzyl, gegebenenfalls ein- bis dreifach, gleich oder verschieden substituiertes Phenylalkyl mit 2 bis 6 Kohlenstoffatomen im Alkylteil, für Phenyloxyalkyl, für Phenylalkyloxyalkyl, für Phenylthioalkyl, für Phenylsulfinylalkyl, oder für Phenylsulfonylalkyl mit jeweils 1 bis 4 Kohlenstoffatomen in jedem Alkylteil steht, wobei als Phenyl- bzw. Benzylsubstituenten jeweils in Frage kommen:
Fluor, Chlor, Brom, Cyano, Nitro, Methyl, Methoxy, Methylthio, Ethyl, Ethoxy, Ethylthio, n- und i-Propyl, Isopropoxy, n-, iso-, sec.- und t-Butyl, Trifluormethyl, Trifluormethoxy, Trifluormethylthio, Cyclohexyl, Methoxycarbonyl, Ethoxycarbonyl und gegebenenfalls durch Fluor, Chlor oder Methyl substituiertes Phenyl oder Phenoxy;
und ferner für Cycloalkylalkyl mit 1 bis 4 Kohlenstoffatomen im Alkylteil und 5 bis 7 Kohlenstoffatomen im gegebenenfalls ein- oder mehrfach durch Alkyl mit 1 bis 4 Kohlenstoffatomen substituierten Cycloalkylteil; ferner für geradkettiges oder verzweigtes Alkenyl mit 3 bis 5 Kohlenstoffatomen, für geradkettiges oder verzweigtes Alkinyl mit 3 bis 6 Kohlenstoffatomen; für ein- bis dreifach, gleich oder verschieden durch Methyl, Ethyl oder Propyl substituiertes Cycloalkyl mit 5 bis 7 Kohlenstoffatomen und für Phenylalkenyl mit 2 bis 5 Kohlenstoffatomen im Alkenylteil steht, wobei als Phenylsubstituenten jeweils in Frage kommen:
Fluor, Chlor, Brom, Cyano, Nitro, Methyl, Methoxy, Methylthio, Ethyl, Ethoxy, Ethylthio, n- und i-Propyl, Isopropoxy, n-, iso-, sec.- und t-Butyl, Trifluormethyl, Trifluormethoxy, Trifluormethylthio, Cyclohexyl, Methoxycarbonyl, Ethoxycarbonyl und gegebenenfalls durch Fluor, Chlor oder Methyl substituiertes Phenyl oder Phenoxy;

$R^{2'}$ für Wasserstoff, für geradkettiges oder verzweigtes Alkyl mit 1 bis 10 Kohlenstoffatomen; für gegebenenfalls ein- bis dreifach, gleich oder verschieden substituiertes Phenyl oder Benzyl steht, wobei als Phenyl- bzw. Benzylsubstituenten jeweils in Frage kommen:
Fluor, Chlor, Brom, Cyano, Nitro, Trifluormethyl, Trifluormethoxy, Trifluormethylthio, Methoxycarbonyl und Ethoxycarbonyl,

$R^{3'}$ für Methyl, Ethyl, n- und i-Propyl, n-, i-, sec.- und t-Butyl, n-Pentyl, n-Hexyl, n-Heptyl, n-Octyl, n-Nonyl, n-Decyl, n-Dodecyl, Allyl, Propargyl, 2-Butenyl, Cyclopentyl, Cyclohexyl sowie für jeweils gegebenenfalls ein- bis zweifach, gleich oder verschieden durch Fluor, Chlor, Methyl oder Trifluormethyl substituiertes Phenyl oder Benzyl steht und

$R^{4'}$ für n- und i-Propyl, n-, i-, sec.- und t-Butyl, n-Pentyl, n-Hexyl, n-Heptyl, n-Octyl, n-Nonyl, n-Decyl, Allyl, Propargyl, 2-Butenyl, Cyclopentyl, Cyclohexyl sowie für jeweils gegebenenfalls ein- bis zweifach, gleich oder verschieden durch Fluor, Chlor, Methyl oder Trifluormethyl substituiertes Phenyl oder Benzyl steht; oder

$R^{3'}$ und $R^{4'}$ zusammen mit dem Stickstoffatom, an das sie gebunden sind, für jeweils gegebenenfalls ein- bis dreifach, gleich oder verschieden substituiertes 1-Pyrrolidinyl, 1-Piperidinyl, 1-Piperazinyl, 4-Morpholinyl, 1-Perhydroazepinyl, 1-Imidazolyl, 1-(1,2,4-Triazolyl) oder 1-Pyrazolyl stehen, wobei als Substituenten genannt seien:
Methyl, Ethyl, n- und i-Propyl, Phenyl, Methoxycarbonyl und Ethoxycarbonyl.

9. Substituierte Aminoketale gemäss Anspruch 7, wobei

$R^{1'}$ für die Gruppierungen

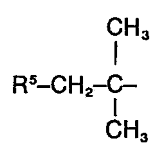 , 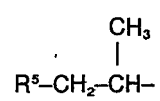 , 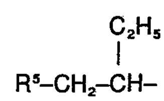 ,

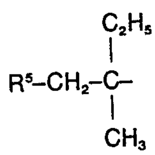 und 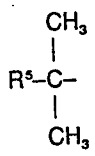 steht, wobei

$R^5$ für jeweils gegebenenfalls einfach bis dreifach, gleich oder verschieden substituiertes Phenyl, Phenoxy, Phenylthio, Phenylsulfinyl oder Phenylsulfonyl steht, wobei als Substituenten genannt seien:

Fluor, Chlor, Brom, Nitro, Cyclohexyl, geradkettiges oder verzweigtes Alkyl mit 1 bis 4 Kohlenstoffatomen, geradkettiges oder verzweigtes Alkoxy mit 1 bis 3 Kohlenstoffatomen und Alkoxycarbonyl mit 1 oder 2 Kohlenstoffatomen im Alkoxyteil,

$R^{2'}$ für geradkettiges oder verzweigtes Alkyl mit 1 bis 4 Kohlenstoffatomen steht,

$R^{3'}$ für geradkettiges oder verzweigtes Alkyl mit 1 bis 4 Kohlenstoffatomen oder für geradkettiges oder verzweigtes Alkenyl mit 3 bis 5 Kohlenstoffatomen steht,

$R^{4'}$ für geradkettiges oder verzweigtes Alkyl mit 1 bis 4 Kohlenstoffatomen steht und

$R^{3'}$ und $R^{4'}$ zusammen mit dem Stickstoffatom, an das sie gebunden sind, für einen jeweils gegebenenfalls einfach bis dreifach, gleich oder verschieden durch Alkyl mit 1 bis 3 Kohlenstoffatomen substituierten Piperidin-, Morpholin- oder Pyrrolidinrest stehen.

10. Substituierte Aminoketale gemäss Anspruch 7, wobei

$R^{1'}$ für die Gruppierungen

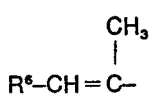 und 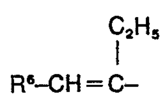

steht, wobei

$R^6$ für jeweils gegebenenfalls einfach bis dreifach, gleich oder verschieden substituiertes Phenyl steht, wobei als Substituenten genannt seien: Fluor, Chlor, Brom, Nitro, Cyclohexyl, geradkettiges oder verzweigtes Alkyl oder Alkoxy mit je 1 bis 4 Kohlenstoffatomen und Alkoxycarbonyl mit 1 oder 2 Kohlenstoffatomen im Alkoxyteil,

$R^{2'}$ für geradkettiges oder verzweigtes Alkyl mit 1 bis 4 Kohlenstoffatomen steht,

$R^{3'}$ für geradkettiges oder verzweigtes Alkyl mit 1 bis 4 Kohlenstoffatomen oder für geradkettiges oder verzweigtes Alkenyl mit 3 bis 5 Kohlenstoffatomen steht,

$R^{4'}$ für geradkettiges oder verzweigtes Alkyl mit 1 bis 4 Kohlenstoffatomen steht und

$R^{3'}$ und $R^{4'}$ zusammen mit dem Stickstoffatom, an das sie gebunden sind, für jeweils gegebenenfalls einfach bis dreifach, gleich oder verschieden, durch Alkyl mit 1 bis 3 Kohlenstoffatomen substituierten Piperidin-, Morpholin- oder Pyrrolidinrest stehen.

11. Schädlingsbekämpfungsmittel, gekennzeichnet durch einen Gehalt an mindestens einem substituierten Aminoketal gemäss Anspruch 7.

12. Verfahren zur Herstellung von Schädlingsbekämpfungsmitteln, dadurch gekennzeichnet, dass man wenigstens ein substituiertes Aminoketal gemäss Anspruch 7 mit Streckmitteln und/oder oberflächenaktiven Mitteln vermischt.

## Claims

1. Use of substituted aminoketals of the formula (I)

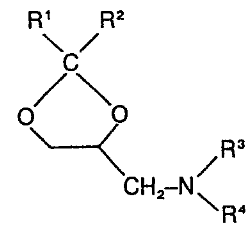

(I)

in which

$R^1$ represents straight-chain or branched alkyl having 1 to 18 carbon atoms; straight-chain or branched halogenoalkyl having 1 to 12 carbon atoms and 1 to 5 halogen atoms, straight-chain or branched cyanoalkyl having 1 to 4 carbon atoms in the alkyl part, arylalkyl, aryloxyalkyl, aralkyloxyalkyl, arylthioalkyl, arylsulphinylalkyl and arylsulphonylalkyl, each having 1 to 6 carbon atoms in each alkyl part and 6 to 10 carbon atoms in the aryl part, these radicals being optionally monosubstituted or polysubstituted by identical or different substituents, the following being suitable aryl substituents in each case: halogen, cyano, nitro; alkyl, alkoxy and alkylthio each having 1 to 4 carbon atoms; halogenoalkyl, halogenoalkoxy and halogenoalkylthio, each having 1 or 2 carbon atoms and 1 to 5 halogen atoms, cycloalkyl having 5 to 7 carbon atoms, alkoxycarbonyl having 1 to 4 carbon atoms in the alkyl part, and phenoxy or phenyl which is optionally substituted by halogen, in particular fluorine or chlorine, or by alkyl having 1 to 4 carbon atoms; and represents cycloalkylalkyl having 1 to 4 carbon atoms in the alkyl part and 3 to 7 carbon atoms in the cycloalkyl part which is optionally monosubstituted or polysubstituted by alkyl having 1 to 4 carbon atoms, and also represents straight-chain or branched alkenyl or alkinyl, each having 2 to 6 carbon atoms, cycloalkyl which has 3 to 7 carbon atoms and is optionally monosubstituted or polysubstituted by identical or different alkyl radicals having 1 to 4 carbon atoms, or represents aryl having 6 to 10 carbon atoms and arylalkenyl having 2 to 6 carbon atoms in the alkenyl part and 6 to 10 carbon atoms in the aryl part, each of which is optionally monosubstituted or polysubstituted by identical or different substituents, suitable aryl substituents in each case being: halogen, cyano, nitro; alkyl, alkoxy, alkylthio, each having 1 to 4 carbon atoms; halogenoalkyl, halogenoalkoxy and halogenoalkylthio, each having 1 or 2 carbon atoms and 1 to 5 halogen atoms, cycloalkyl having 5 to 7 carbon atoms, alkoxycarbonyl having 1 to 4 carbon atoms in the alkyl part, and phenoxy or phenyl which is optionally substituted by halogen, in particular fluorine or chlorine, or by alkyl having 1 to 4 carbon atoms;

$R^2$ has the meaning of $R^1$ and may be identical or different to this radical, and additionally represents hydrogen,

$R^3$ represents straight-chain or branched alkyl having 1 to 12 carbon atoms, straight-chain or branched alkenyl having 3 to 12 carbon atoms, straight-chain or branched alkinyl having 3 to 7 carbon atoms, cycloalkyl which has 5 to 7 carbon atoms and is optionally monosubstituted or polysubstituted by identical or different alkyl groups having 1 to 4 carbon atoms, and aryl having 6 to 10 carbon atoms or aralkyl having 1 or 2 carbon atoms in the alkyl part and 6 to 10 carbon atoms in the aryl part, each of which radicals is optionally monosubstituted or polysubstituted by identical or different substituents, suitable aryl substituents in each case being:
halogen, alkyl having 1 to 4 carbon atoms and halogenoalkyl having 1 or 2 carbon atoms and 1 to 5 halogen atoms;

$R^4$ represents straight-chain or branched alkyl having 1 to 12 carbon atoms, straight-chain or branched alkenyl having 3 to 12 carbon atoms, straight-chain or branched alkinyl having 3 to 7 carbon atoms, cycloalkyl which has 5 to 7 carbon atoms and is optionally monosubstituted or polysubstituted by identical or different alkyl groups having 1 to 4 carbon atoms, and aryl having 6 to 10 carbon atoms or aralkyl having 1 to 2 carbon atoms in the alkyl part and 6 to 10 carbon atoms in the aryl part, each of which radicals is optionally monosubstituted or polysubstituted by identical or different substituents, suitable aryl substituents in each case being:
halogen, alkyl having 1 to 4 carbon atoms and halogenoalkyl having 1 or 2 carbon atoms and 1 to 5 halogen atoms; or

$R^3$ and $R^4$, together with the nitrogen atom to which they are bonded, represent a 5-membered to 7-membered, saturated or unsaturated heterocyclic structure, which has 1 to 3 heteroatoms, preferably nitrogen or oxygen, and is optionally monosubstituted or polysubstituted by identical or different substituents, the following being preferably mentioned as substituents: alkyl having 1 to 4 carbon atoms, alkoxycarbonyl having 1 to 4 carbon atoms in the alkyl part and phenyl, for combating pests.

2. Use according to Claim 1, wherein in formula (I)

$R^1$ represents straight-chain or branched alkyl having 1 to 12 carbon atoms, cyclopropyl, cyclopentyl, cyclohexyl, straight-chain or branched halogenoalkyl having 1 to 6 carbon atoms and 1 to 5 halogen atoms, or phenyl, phenylalkyl, phenoxyalkyl, benzyloxyalkyl, phenylthioalkyl, phenylsulphinylalkyl and phenylsulphonylalkyl, each having 1 to 5 carbon atoms in the alkyl part, these radicals in each case being optionally monosubstituted to trisubstituted by identical or different substituents, and phenylalkenyl which has 2 to 4 carbon atoms in the alkenyl part and is optionally monosubstituted to trisubstituted by identical or different substituents, suitable phenyl substituents being:
fluorine, chlorine, bromine, cyano, nitro, methyl, methoxy, methylthio, ethyl, ethoxy, ethylthio, n- and i-propyl, isopropoxy, n-, iso-, sec.- and t-butyl, trifluoromethyl, trifluoromethoxy, trifluoromethylthio, cyclohexyl, methoxycarbonyl, ethoxycarbonyl and phenoxy or phenyl which is optionally substituted by fluorine, chlorine or methyl;

$R^2$ represents straight-chain or branched alkyl having 1 to 10 carbon atoms, benzyl and phenyl which is optionally monosubstituted to trisubstituted by identical or different substituents, suitable phenyl or benzyl substituents in each case being: fluorine, chlorine, bromine, cyano, nitro, trifluoromethyl, trifluoromethoxy, trifluoromethylthio, methoxycarbonyl and ethoxycarbonyl, or represents cyclopropyl, cyclopentyl and cyclohexyl;

$R^3$ represents methyl, ethyl, n- and i-propyl, n-, i-, sec.- and t-butyl, n-pentyl, n-hexyl, n-heptyl, n-octyl, n-nonyl, n-decyl, n-dodecyl, allyl, propargyl, 2-butenyl, cyclopentyl or cyclohexyl, and phenyl or benzyl, each of which is optionally monosubstituted to disubstituted by identical or different substituents from the group comprising fluorine, chlorine, methyl and trifluoromethyl, and

$R^4$ represents n- and i-propyl, n-, i-, sec.- and t-butyl, n-pentyl, n-hexyl, n-heptyl, n-octyl, n-nonyl, n-decyl, allyl, propargyl, but-2-enyl, cyclopentyl or cyclohexyl, and phenyl or benzyl, each of which is optionally monosubstituted to disubstituted by identical or different substituents from the group comprising fluorine, chlorine, methyl and trifluoromethyl; or

$R^3$ and $R^4$, together with the nitrogen atom to which they are bonded, represent pyrrolidin-1-yl, piperidin-1-yl, piperazin-1-yl, morpholin-4-yl, perhydroazepin-1-yl, imidazol-1-yl, 1,2,4-triazol-1-yl or pyrazol-1-yl, each of which is optionally monosubstituted to trisubstituted by identical or different substituents, the following being mentioned as substituents:
methyl, ethyl, n- and i-propyl, phenyl, methoxycarbonyl and ethoxycarbonyl.

3. Use according to Claim 1, wherein in formula (I)

$R^1$ represents straight-chain or branched alkyl having 1 to 5 carbon atoms, and phenylalkenyl, phenylalkyl, phenoxyalkyl, benzyloxyalkyl, phenylthioalkyl, phenylsulphinylalkyl and phenylsulphonylalkyl, each of which has up to 5 carbon atoms in the alkyl part or alkenyl part and is optionally monosubstituted to trisubstituted by identical or different substituents, the following being mentioned as phenyl substituents:
fluorine, chlorine, bromine, methyl, ethyl, n- and i-propyl, t-butyl, methoxy, ethoxy, isopropoxy, cyclohexyl, trifluoromethyl, trifluoromethoxy, trifluoromethylthio, methoxycarbonyl, ethoxycarbonyl, nitro, cyano, phenyl and phenoxy;

$R^2$ represents straight-chain or branched alkyl having 1 to 4 carbon atoms or hydrogen;

$R^3$ represents methyl, ethyl, n- and i-propyl or n-, i- and t-butyl;

$R^4$ represents methyl, ethyl, n- and i-propyl, n- and i-butyl, pentyl or hexyl, or

$R^3$ and $R^4$, together with the nitrogen atom to which they are bonded, represent piperidin-1-yl, perhydroazepin-1-yl, pyrrolidin-1-yl, morpholin-4-yl, piperazin-1-yl, imidazol-1-yl and 1,2,4-triazol-1-yl, each of which is optionally monosubstituted to trisubstituted by identical or different substituents, the following being mentioned as substituents: alkyl having 1 to 3 carbon atoms, methoxycarbonyl, ethoxycarbonyl and phenyl.

4. Use according to Claim 1, wherein in formula (I)

$R^1$ represents the groupings

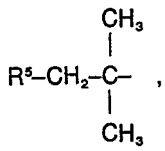 , 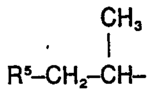 , 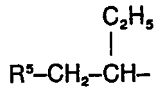 ,

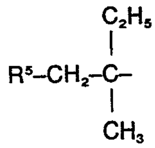 and 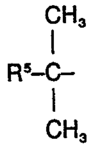

wherein

$R^5$ represents phenyl, phenoxy, phenylthio, phenylsulphinyl or phenylsulphonyl, each of which is optionally monosubstituted to trisubstituted by identical or different substituents, the following being mentioned as substituents: fluorine, chlorine, bromine, nitro, cyclohexyl, straight-chain or branched alkyl having 1 to 4 carbon atoms, straight-chain or branched alkoxy having 1 to 3 carbon atoms and alkoxycarbonyl having 1 or 2 carbon atoms in the alkoxy part,

$R^2$ represents straight-chain or branched alkyl having 1 to 4 carbon atoms,

$R^3$ represents a straight-chain or branched alkyl having 1 to 4 carbon atoms or straight-chain or branched alkenyl having 3 to 5 carbon atoms,

$R^4$ represents straight-chain or branched alkyl having 1 to 4 carbon atoms, and

$R^3$ and $R^4$, together with the nitrogen atom to which they are bonded, represent a piperidine, morpholine or pyrrolidine radical which is optionally monosubstituted to trisubstituted by identical or different alkyl radicals having 1 to 3 carbon atoms.

5. Use according to Claim 1, wherein in formula (I)

$R^1$ represents the groupings

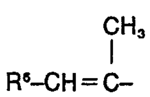 and 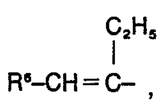 ,

wherein

$R^6$ represents phenyl which is in each case optionally monosubstituted to trisubstituted by identical or different substituents, the following being mentioned as substituents: fluorine, chlorine, bromine, nitro, cyclohexyl, straight-chain or branched alkyl or alkoxy, each having 1 to 4 carbon atoms and alkoxycarbonyl having 1 or 2 carbon atoms in the alkoxy part,

$R^2$ represents straight-chain or branched alkyl having 1 to 4 carbon atoms,

$R^3$ represents straight-chain or branched alkyl having 1 to 4 carbon atoms or straight-chain or branched alkenyl having 3 to 5 carbon atoms,

$R^4$ represents straight-chain or branched alkyl having 1 to 4 carbon atoms, and

$R^3$ and $R^4$, together with the nitrogen atom to which they are bonded, represent a piperidine, morpholine or pyrrolidine radical which is optionally monosubstituted to trisubstituted by identical or different alkyl radicals having 1 to 3 carbon atoms.

6. Process for combating pests, characterised in that substituted aminoketals of the formula (I) according to Claim 1 are allowed to act on pests or their habitat.

7. Substituted aminoketals of the formula (IA)

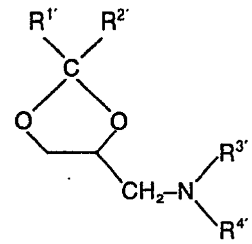

(IA)

wherein

$R^{1'}$ represents straight-chain or branched alkyl having 6 to 18 carbon atoms; straight-chain or branched halogenoalkyl having 2 to 12 carbon atoms and 1 to 5 halogen atoms, straight-chain or branched cyanoalkyl having 1 to 4 carbon atoms in the alkyl part, benzyl which is monosubstituted or polysubstituted by identical or different substituents, arylalkyl which has 2 to 6 carbon atoms in the alkyl part and 6 to 10 carbon atoms in the aryl part and is optionally monosubstituted or polysubstituted by identical or different substituents, and aryloxyalkyl, aralkyloxyalkyl, arylthioalkyl, arylsulphinylalkyl and arylsulphonylalkyl, each of which has 1 to 6 carbon atoms in each alkyl part and 6 to 10 carbon atoms in the aryl part, suitable aryl substituents in each case being: halogen, cyano, nitro; alkyl, alkoxy and alkylthio, each having 1 to 4 carbon atoms, halogenoalkyl, halogenoalkoxy and halogenoalkylthio, each having 1 or 2 carbon atoms and 1 to 5 halogen atoms, cycloalkyl having 5 to 7 carbon atoms, alkoxycarbonyl having 1 to 4 carbon atoms in the alkyl part, and phenoxy or phenyl which is optionally substituted by halogen, in particular fluorine or chlorine, or by alkyl having 1 to 4 carbon atoms; cycloalkylalkyl having 1 to 4 carbon atoms in the alkyl part and 3 to 7 carbon atoms in the cycloalkyl part which is optionally monosubstituted or polysubstituted by alkyl having 1 to 4 carbon atoms; and also represents straight-chain or branched alkenyl having 2 to 5 carbon atoms, straight-chain or branched alkinyl having 2 to 6 carbon atoms; cycloalkyl which has 3 to 7 carbon atoms and is monosubstituted to polysubstituted by identical or

different alkyl radicals having 1 to 4 carbon atoms, and represents arylalkenyl having 2 to 6 carbon atoms in the alkenyl part and 6 to 10 carbon atoms in the aryl part, suitable aryl substituents in each case being:
halogen, cyano, nitro; alkyl, alkoxy and alkylthio, each having 1 to 4 carbon atoms, halogenoalkoxy and halogenoalkylthio, each having 1 or 2 carbon atoms and 1 to 5 halogen atoms, cycloalkyl having 5 to 7 carbon atoms, alkoxycarbonyl having 1 to 4 carbon atoms in the alkyl part, and phenoxy or phenyl which is optionally substituted by halogen, in particular fluorine or chlorine, or by alkyl having 1 to 4 carbon atoms;

$R^{2'}$ represents hydrogen, straight-chain or branched alkyl having 1 to 18 carbon aroms, straight-chain or branched halogenoalkyl having 1 to 12 carbon atoms and 1 to 5 halogen atoms, straight-chain or branched cyanoalkyl having 1 to 4 carbon atoms in the alkyl part, arylalkyl, aryloxyalkyl, aralkyloxyalkyl, arylthioalkyl, arylsulphinylalkyl or arylsulphonylalkyl, each of which has 1 to 6 carbon atoms in each alkyl part and 6 to 10 carbon atoms in the aryl part and is optionally monosubstituted or polysubstituted by identical or different substituents, suitable aryl substituents in each case being:
halogen, cyano, nitro; alkyl, alkoxy and alkylthio, each having 1 to 4 carbon atoms; halogenoalkyl, halogenoalkoxy and halogenoalkylthio, each having 1 or 2 carbon atoms and 1 to 5 halogen atoms, cycloalkyl having 5 to 7 carbon atoms, alkoxycarbonyl having 1 to 4 carbon atoms in the alkyl part, and phenoxy or phenyl which is optionally substituted by halogen, in particular fluorine or chlorine, or by alkyl having 1 to 4 carbon atoms; and cycloalkylalkyl having 1 to 4 carbon atoms in the alkyl part and 3 to 7 carbon atoms in the cycloalkyl part which is optionally monosubstituted or polysubstituted by alkyl having 1 to 4 carbon atoms, and also represents straight-chain or branched alkenyl or alkinyl, each having 2 to 6 carbon atoms, cycloalkyl which has 3 to 7 carbon atoms and is optionally monosubstituted or polysubstituted by identical or different alkyl radicals having 1 to 4 carbon atoms, or represents aryl having 6 to 10 carbon atoms and arylalkenyl having 2 to 6 carbon atoms in the alkenyl part and 6 to 10 carbon atoms in the aryl part, each of which is optionally monosubstituted or polysubstituted by identical or different substituents, suitable aryl substituents in each case being: halogen, cyano, nitro; alkyl, alkoxy and alkylthio, each having 1 to 4 carbon atoms; halogenoalkyl, halogenoalkoxy and halogenoalkylthio, each having 1 to 2 carbon atoms and 1 to 5 halogen atoms, cycloalkyl having 5 to 7 carbon atoms, alkoxycarbonyl having 1 to 4 carbon atoms in the alkyl part, and phenoxy or phenyl which is optionally substituted by halogen, in particular fluorine or chlorine, or by alkyl having 1 to 4 carbon atoms;

$R^{3'}$ represents straight-chain or branched alkyl having 1 to 12 carbon atoms, straight-chain or branched alkenyl having 3 to 12 carbon atoms, straight-chain or branched alkinyl having 3 to 7 carbon atoms, cycloalkyl which has 5 to 7 carbon atoms and is optionally monosubstituted or polysubstituted by identical or different alkyl radicals having 1 to 4 carbon atoms, and represents aryl having 6 to 10 carbon atoms or aralkyl having 1 or 2 carbon atoms in the alkyl part and 6 to 10 carbon atoms in the aryl part, each of which is optionally monosubstituted or polysubstituted by identical or different substituents, suitable aryl substituents in each case being:
halogen, alkyl having 1 to 4 carbon atoms and halogenoalkyl having 1 or 2 carbon atoms and 1 to 5 halogen atoms;

$R^{4'}$ represents straight-chain or branched alkyl having 1 to 12 carbon atoms, straight-chain or branched alkenyl having 3 to 12 carbon atoms, straight-chain or branched alkinyl having 3 to 7 carbon atoms, cycloalkyl which has 5 to 7 carbon atoms and is optionally monosubstituted or polysubstituted by identical or different alkyl radicals having 1 to 4 carbon atoms, and aryl having 6 to 10 carbon atoms or aralkyl having 1 to 2 carbon atoms in the alkyl part and 6 to 10 carbon atoms in the aryl part, each of which is optionally monosubstituted or polysubstituted by identical or different substituents, suitable aryl substituents in each case being:
halogen, alkyl having 1 to 4 carbon atoms, and halogenoalkyl having 1 or 2 carbon atoms and 1 to 5 halogen atoms; or

$R^{3'}$ and $R^{4'}$, together with the nitrogen atom to which they are bonded, represent a 5-membered to 7-membered, saturated or unsaturated heterocyclic structure which has 1 to 3 nitrogen and/or oxygen atoms and is optionally monosubstituted or polysubstituted by identical or different substituents, the following being mentioned as substituents: alkyl having 1 to 4 carbon atoms, alkoxycarbonyl having 1 to 4 carbon atoms in the alkyl part, and phenyl, with the exception of compounds in which

(a) $R^{1'}$ represents ethylbutylcarbinol (3-heptyl),
$R^{2'}$ represents phenyl and
$R^{3'}$ and $R^{4'}$ represent ethyl and

(b) $R^{1'}$ represents methyl,
$R^{2'}$ represents n–$C_9H_{19}$– and
$R^{3'}$ and $R^{4'}$ represent ethyl.

8. Substituted aminoketals according to Claim 7, wherein

$R^{1'}$ represents straight-chain or branched alkyl having 6 to 12 carbon atoms; straight-chain or branched halogenoalkyl having 2 to 6 carbon atoms and 1 to 5 halogen atoms, cyanomethyl or cyanoethyl, benzyl which is monosubstituted to trisubstituted by identical or different substituents, phenylalkyl which has 2 to 6 carbon atoms in the alkyl part and is optionally monosubstituted to trisubstituted by identical or different substituents, and phenyloxyalkyl, phenylalkyloxyalkyl, phenylthioalkyl, phenylsulphinylalkyl or phenylsulphonylalkyl, each of which has 1 to 4 carbon atoms in each alkyl part, suitable phenyl or benzyl substituents in each case being:
fluorine, chlorine, bromine, cyano, nitro, methyl, methoxy, methylthio, ethyl, ethoxy, ethylthio, n-and i-propyl, isopropoxy, n-, iso-, sec.- and t-butyl, trifluoromethyl, trifluoromethoxy, trifluoromethylthio, cyclonexyl, methoxycarbonyl and ethoxycarbonyl, and phenoxy or phenyl which is optionally substituted by fluorine, chlorine or methyl; and also represents cycloalkylalkyl having 1 to 4 carbon atoms in the alkyl part and 5 to 7 carbon atoms in the cycloalkyl part which is optionally monosubstituted or polysubstituted by alkyl having 1 to 4 carbon atoms; and also represents straight-chain or branched alkenyl having 3 to 5 carbon atoms, straight-chain or branched alkinyl having 3 to 6 carbon atoms; cycloalkyl which has 5 to 7 carbon atoms and is monosubstituted to trisubstituted by identical or different substituents from amongst methyl, ethyl and propyl, and represents phenylalkenyl having 2 to 5 carbon atoms in the alkenyl part, suitable phenyl substituents in each case being:

fluorine, chlorine, bromine, cyano, nitro, methyl, methoxy, methylthio, ethyl, ethoxy, ethylthio, n- and i-propyl, isopropoxy, n-, iso-, sec.- and t-butyl, trifluoromethyl, trifluoromethoxy, trifluoromethylthio, cyclohexyl, methoxycarbonyl, ethoxycarbonyl, and phenoxy or phenyl which is optionally substituted by fluorine, chlorine or methyl;

$R^{2'}$ represents hydrogen, straight-chain or branched alkyl having 1 to 10 carbon atoms; and benzyl or phenyl which is optionally monosubstituted to trisubstituted by identical or different substituents, suitable phenyl or benzyl substituents in each case being:

fluorine, chlorine, bromine, cyano, nitro, trifluoromethyl, trifluoromethoxy, trifluoromethylthio, methoxycarbonyl and ethoxycarbonyl;

$R^{3'}$ represents methyl, ethyl, n- and i-propyl, n-, i-, sec.- and t-butyl, n-pentyl, n-hexyl, n-heptyl, n-octyl, n-nonyl, n-decyl, n-dodecyl, allyl, propargyl, but-2-enyl, cyclopentyl and cyclohexyl, and benzyl or phenyl, each of which is optionally monosubstituted to disubstituted by identical or different substituents from amongst fluorine, chlorine, methyl and trifluoromethyl and

$R^{4'}$ represents n- and i-propyl, n-, i-, sec.- and t-butyl, n-pentyl, n-hexyl, n-heptyl, n-octyl, n-nonyl, n-decyl, allyl, propargyl, but-2-enyl, cyclopentyl and cyclohexyl, and phenyl or benzyl, each of which is optionally monosubstituted or disubstituted by identical or different substituents from amongst fluorine, chlorine, methyl and trifluoromethyl; or

$R^{3'}$ and $R^{4'}$, together with the nitrogen atom to which they are bonded, represent pyrrolidin-1-yl, piperidin-1-yl, piperazin-1-yl, morpholin-4-yl, perhydroazepin-1-yl, imidazol-1-yl, 1,2,4-triazol-1-yl or pyrazol-1-yl, each of which is optionally monosubstituted to trisubstituted by identical or different substituents, the following being mentioned as substituents:

methyl, ethyl, n- and i-propyl, phenyl, methoxycarbonyl and ethoxycarbonyl.

9. Substituted aminoketals according to Claim 7, wherein

$R^{1'}$ represents the groupings

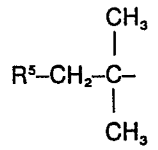 , 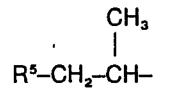 , 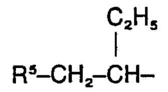 ,

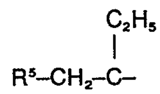 and 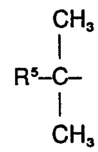

wherein

$R^5$ represents phenyl, phenoxy, phenylthio, phenylsulphinyl or phenylsulphonyl, each of which is optionally monosubstituted to trisubstituted by identical or different substituents, the following being mentioned as substituents: fluorine, chlorine, bromine, nitro, cyclohexyl, straight-chain or branched alkyl having 1 to 4 carbon atoms, straight-chain or branched alkoxy having 1 to 3 carbon atoms and alkoxycarbonyl having 1 or 2 carbon atoms in the alkoxy part,

$R^{2'}$ represents straight-chain or branched alkyl having 1 to 4 carbon atoms,

$R^{3'}$ represents straight-chain or branched alkyl having 1 to 4 carbon atoms or straight-chain or branched alkenyl having 3 to 5 carbon atoms,

$R^{4'}$ represents straight-chain or branched alkyl having 1 to 4 carbon atoms, and

$R^{3'}$ and $R^{4'}$, together with the nitrogen atom to which they are bonded, represent a piperidine, morpholine or pyrrolidine radical, each of which is optionally monosubstituted to trisubstituted by identical or different alkyl radicals having 1 to 3 carbon atoms.

10. Substituted aminoketals according to Claim 7, wherein

$R^{1'}$ represents the groupings

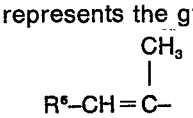 and 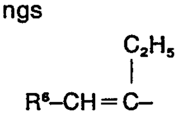

wherein

$R^6$ represents phenyl which in each case is optionally monosubstituted to trisubstituted by identical or different substituents, the following being mentioned as substituents: fluorine, chlorine, bromine, nitro, cyclohexyl, straight-chain or branched alkyl or alkoxy, each having 1 to 4 carbon atoms, and alkoxycarbonyl having 1 or 2 carbon atoms in the alkoxy part,

$R^{2'}$ represents straight-chain or branched alkyl having 1 to 4 carbon atoms,

$R^{3'}$ represents straight-chain or branched alkyl having 1 to 4 carbon atoms or straight-chain or branched alkenyl having 3 to 5 carbon atoms,

$R^{4'}$ represents straight-chain or branched alkyl having 1 to 4 carbon atoms, and

$R^{3'}$ and $R^{4'}$, together with the nitrogen atom to which they are bonded, represent a piperidine, morpholine or pyrrolidine radical, each of which is optionally monosubstituted to trisubstituted by identical or different alkyl radicals having 1 to 3 carbon atoms.

11. Agents for combating pests, characterised by a content of at least one substituted aminoketal according to Claim 7.

12. Process for the preparation of agents for combating pests, characterised in that at least one substituted aminoketal according to Claim 7 is mixed with extenders and/or surface-active agents.

**Revendications**

1. Utilisation d'aminocétals substitués de formule (I)

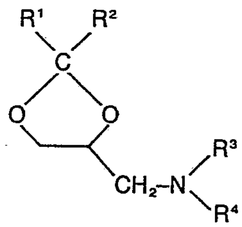

(I)

dans laquelle

$R^1$ représente un alcoyle à chaîne droite ou ramifiée ayant 1 à 18 atomes de carbone, un halogénoalcoyle à chaîne droite ou ramifiée ayant 1 à 12 atomes de carbone et 1 à 5 atomes d'halogène, un cyanalcoyle à chaîne droite ou ramifiée ayant 1 à 4 atomes de carbone dans la portion alcoyle, un arylalcoyle, aryloxyalcoyle, aralcoyloxyalcoyle, arylthioalcoyle, arylsulfinylalcoyle, arylsulfonylalcoyle qui sont éventuellement substitués une ou plusieurs fois, de manière identique ou différente, et qui ont chacun 1 à 6 atomes de carbone dans chaque portion alcoyle et 6 à 10 atomes de carbone dans la portion aryle, en envisageant comme substituants de l'aryle:
halogène, cyano, nitro; alcoyle, alcoxy, alcoylthio ayant chacun 1 à 4 atomes de carbone; halogénoalcoyle, halogénoalcoxy et halogénoalcoylthio ayant chacun 1 ou 2 atomes de carbone et 1 à 5 atomes d'halogène, cycloalcoyle ayant 5 à 7 atomes de carbone, alcoxycarbonyle ayant 1 à 4 atomes de carbone dans la portion alcoyle et un phényle ou phénoxy éventuellement substitués par de l'halogène, en particulier par du fluor ou du chlore ou par un alcoyle ayant 1 à 4 atomes de carbone; et un cycloalcoylalcoyle ayant 1 à 4 atomes de carbone dans la portion alcoyle et 3 à 7 atomes de carbone dans la portion cycloalcoyle qui est éventuellement substituée une ou plusieurs fois par un alcoyle ayant 1 à 4 atomes de carbone, en outre un alcényle ou alcinyle à chaîne droite ou ramifiée ayant chacun 2 à 6 atomes de carbone, un cycloalcoyle éventuellement substitué une ou plusieurs fois, de manière identique ou différente par un alcoyle ayant 1 à 4 atomes de carbone et ayant 3 à 7 atomes de carbone, ou un aryle ayant 6 à 10 atomes de carbone de même qu'un arylalcényle ayant 2 à 6 atomes de carbone dans la portion alcényle et 6 à 10 atomes de carbone dans la portion aryle, qui sont chacun éventuellement substitués une ou plusieurs fois, de manière identique ou différente, en envisageant chaque fois comme substituants de l'aryle:
halogène, cyano, nitro; alcoyle, alcoxy, alcoylthio ayant chacun 1 à 4 atomes de carbone; halogénoalcoyle, halogénoalcoxy et halogénoalcoylthio ayant chacun 1 ou 2 atomes de carbone et 1 à 5 atomes d'halogène, cycloalcoyle ayant 5 à 7 atomes de carbone, alcoxycarbonyle ayant 1 à 4 atomes de carbone dans la portion alcoyle, et phényle ou phénoxy éventuellement substitués par de l'halogène, en particulier par du fluor ou du chlore ou par un alcoyle ayant 1 à 4 atomes de carbone,

$R^2$ a la signification de $R^1$, il peut être identique ou non identique à celui-ci et il représente en outre de l'hydrogène,

$R^3$ représente un alcoyle à chaîne droite ou ramifiée ayant 1 à 12 atomes de carbone, un alcényle à chaîne droite ou ramifiée ayant 3 à 12 atomes de carbone, un alcinyle à chaîne droite ou ramifiée ayant 3 à 7 atomes de carbone, un cycloalcoyle ayant 5 à 7 atomes de carbone éventuellement substitué une ou plusieurs fois, de manière identique ou différente, par un alcoyle ayant 1 à 4 atomes de carbone, de même qu'un aryle ayant 6 à 10 atomes de carbone ou aralcoyle ayant 1 ou 2 atomes de carbone dans la portion alcoyle et 6 à 10 atomes de carbone dans la portion aryle et qui sont éventuellement substitués chacun une ou plusieurs fois, de manière identique ou différente, en envisageant comme substituants de l'aryle:
halogène, alcoyle ayant 1 à 4 atomes de carbone de même qu'halogénoalcoyle ayant 1 ou 2 atomes de carbone et 1 à 5 atomes d'halogène;

$R^4$ représente un alcoyle à chaîne droite ou ramifiée ayant 1 à 12 atomes de carbone, un alcényle à chaîne droite ou ramifiée ayant 3 à 12 atomes de carbone, un alcinyle à chaîne droite ou ramifiée ayant 3 à 7 atomes de carbone, un cycloalcoyle ayant 5 à 7 atomes de carbone éventuellement substitué une ou plusieurs fois, de manière identique ou différente, par un alcoyle ayant 1 à 4 atomes de carbone, de même qu'un aryle ayant 6 à 10 atomes de carbone ou aralcoyle ayant 1 à 2 atomes de carbone dans la portion alcoyle et 6 à 10 atomes de carbone dans la portion aryle et qui sont substitués chacun éventuellement une ou plusieurs fois, de manière identique ou différente, en envisageant comme substituants de l'aryle:
halogène, alcoyle ayant 1 à 4 atomes de carbone de même qu'halogénoalcoyle ayant 1 à 2 atomes de carbone et 1 à 5 atomes d'halogène; ou bien

$R^3$ et $R^4$, conjointement avec l'atome d'azote auquel ils sont attachés, représentent un hétérocycle saturé ou insaturé à 5 à 7 chaînons, éventuellement substitué une ou plusieurs fois, de manière identique ou différente et qui comporte 1 à 3 hétéroatomes, de préférence de l'azote ou de l'oxygène, en citant de préférence comme substituants: alcoyle ayant 1 à 4 atomes de carbone, alcoxycarbonyle ayant 1 à 4 atomes de carbone dans la portion alcoyle, et phényle, pour combattre les parasites.

2. Utilisation selon la revendication 1, dans la formule (I)

$R^1$ signifiant un alcoyle à chaîne droite ou ramifiée ayant 1 à 12 atomes de carbone, cyclopropyle, cyclopentyle, cyclohexyle, un halogénoalcoyle à

chaîne droite ou ramifiée ayant 1 à 6 atomes de carbone et 1 à 5 atomes d'halogène, ou un phényle, phénylalcoyle, phénoxyalcoyle, benzyloxyalcoyle, phénylthioalcoyle, phénylsulfinylalcoyle et phénylsulfonylalcoyle éventuellement substitués chacun une à trois fois, de manière identique ou différente et qui possèdent chacun 1 à 5 atomes de carbone dans la portion alcoyle ainsi qu'un phénylalcényle éventuellement substitué une à trois fois, de manière identique ou différente et qui contient 2 à 4 atomes de carbone dans la portion alcényle, en envisageant en tant que substituants du phényle:

fluor, chlore, brome, cyano, nitro, méthyle, méthoxy, méthylthio, éthyle, éthoxy, éthylthio, n- et i-propyle, isopropoxy, n-, iso-, sec- et t-butyle, trifluorométhyle, trifluorométhoxy, trifluorométhylthio, cyclohexyle, méthoxycarbonyle, éthoxycarbonyle et phényle ou phénoxy éventuellement substitués par du fluor, du chlore ou méthyle,

$R^2$ un alcoyle à chaîne droite ou ramifiée ayant 1 à 10 atomes de carbone, un phényle et benzyle éventuellement substitués une à trois fois, de manière identique ou différente, en envisageant chaque fois comme substituants du phényle ou du benzyle:

fluor, chlore, brome, cyano, nitro, trifluorométhyle, trifluorométhoxy, trifluorométhylthio, méthoxycarbonyle et éthoxycarbonyle, en outre cyclopropyle, cyclopentyle et cyclohexyle,

$R^3$ un méthyle, éthyle, n- et i-propyle, n-, i-, sec- et t-butyle, n-pentyle, n-hexyle, n-heptyle, n-octyle, n-nonyle, n-décyle, n-dodécyle, allyle, propargyle, 2-butényle, cyclopentyle, cyclohexyle de même qu'un phényle ou benzyle éventuellement substitués chacun une à deux fois, de manière identique ou différente, par du fluor, du chlore, un méthyle ou un trifluorométhyle, et

$R^4$ un n- et i-propyle, n-, i-, sec- et t-butyle, n-pentyle, n-hexyle, n-heptyle, n-octyle, n-nonyle, n-décyle, allyle, propargyle, 2-butényle, cyclopentyle, cyclohexyle de même qu'un phényle ou benzyle éventuellement substitués chacun une à deux fois, de manière identique ou différente, par du fluor, chlore, méthyle ou trifluorométhyle; ou bien

$R^3$ et $R^4$, conjointement avec l'atome d'azote auquel ils sont attachés, représentent un 1-pyrrolidinyle, 1-pipéridinyle, 1-pipérazinyle, 4-morpholinyle, 1-perhydroazépinyle, 1-imidazolyle, 1-(1,2,4-triazolyle) ou 1-pyrazolyle éventuellement substitués chacun une à trois fois, de manière identique ou différente, en citant en tant que substituants: méthyle, éthyle, n- et i-propyle, phényle, méthoxycarbonyle et éthoxycarbonyle.

3. Utilisation selon la revendication 1 où, dans la formule (I):

$R^1$ représente un alcoyle à chaîne droite ou ramifiée ayant 1 à 5 atomes de carbone de même qu'un phénylalcényle, phénylalcoyle, phènoxyalcoyle, benzyloxyalcoyle, phénylthioalcoyle, phénylsulfinylalcoyle et phénylsulfonylalcoyle éventuellement substitués chacun une à trois fois, de manière identique ou différente, et ayant chacun jusqu'à 5 atomes de carbone dans la portion alcoyle ou alcényle, en citant comme substituants du phényle:

fluor, chlore, brome, méthyle, éthyle, n- et i-propyle, t-butyle, méthoxy, éthoxy, isopropoxy, cyclohexyle, trifluorométhyle, trifluorométhoxy, trifluorométhylthio, méthoxycarbonyle, éthoxycarbonyle, nitro, cyano, phényle et phénoxy;

$R^2$ un alcoyle à chaîne droite ou ramifiée ayant 1 à 4 atomes de carbone ou de l'hydrogène,

$R^3$ un méthyle, éthyle, n- et i-propyle ou n-, i- et t-butyle,

$R^4$ un méthyle, éthyle, n- et i-propyle, n- et i-butyle, pentyle ou hexyle, ou bien

$R^3$ et $R^4$, conjointement avec l'atome d'azote auquel ils sont attachés, représentent un 1-pipéridinyle, 1-perhydroazépinyle, 1-pyrrolidinyle, 4-morpholinyle, 1-pipérazinyle, 1-imidazolyle et 1-(1,2,4-triazolyle) éventuellement substitués chacun une à trois fois, de manière identique ou différente, en citant comme substituants:

alcoyle ayant 1 à 3 atomes de carbone, méthoxycarbonyle, éthoxycarbonyle et phényle.

4. Utilisation selon la revendication 1 où, dans la formule (I):

$R^1$ représente les groupements:

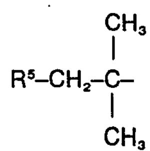 , 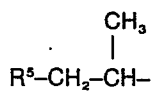 , 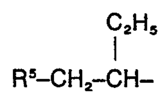 ,

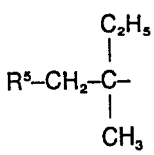 et 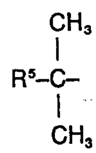

$R^5$ un phényle, phénoxy, phénylthio, phénylsulfinyle ou phénylsulfonyle éventuellement substitués chacun une à trois fois, de manière identique ou différente, en citant comme substituants:

fluor, chlore, brome, nitro, cyclohexyle, alcoyle à chaîne droite ou ramifiée ayant 1 à 4 atomes de carbone, alcoxy à chaîne droite ou ramifiée ayant 1 à 3 atomes de carbone et alcoxycarbonyle ayant 1 ou 2 atomes de carbone dans la portion alcoxy,

$R^2$ un alcoyle à chaîne droite ou ramifiée ayant 1 à 4 atomes de carbone,

$R^3$ un alcoyle à chaîne droite ou ramifiée ayant 1 à 4 atomes de carbone ou un alcényle à chaîne droite ou ramifiée ayant 3 à 5 atomes de carbone,

$R^4$ un alcoyle à chaîne droite ou ramifiée ayant 1 à 4 atomes de carbone et

$R^3$ et $R^4$, conjointement avec l'atome d'azote auquel ils sont attachés, représentent un radical pipéridine, morpholine ou pyrrolidine éventuellement substitués chacun une à trois fois, de manière identique ou différente, par un alcoyle ayant 1 à 3 atomes de carbone.

5. Utilisation selon la revendication 1 où, dans la formule (I):

$R^1$ représente les groupements

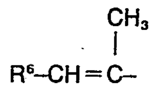 et 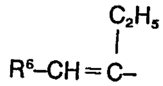

et où

$R^6$ représente un phényle éventuellement substitué chaque fois une à trois fois, de manière identique ou différente, en citant en tant que substituants:
fluor, chlore, brome, nitro, cyclohexyle, alcoyle ou alcoxy à chaîne droite ou ramifiée ayant chacun 1 à 4 atomes de carbone et alcoxycarbonyle ayant 1 ou 2 atomes de carbone dans la portion alcoxy,

$R^2$ un alcoyle à chaîne droite ou ramifiée ayant 1 à 4 atomes de carbone,

$R^3$ un alcoyle à chaîne droite ou ramifiée ayant 1 à 4 atomes de carbone ou un alcényle à chaîne droite ou ramifiée ayant 3 à 5 atomes de carbone,

$R^4$ un alcoyle à chaîne droite ou ramifiée ayant 1 à 4 atomes de carbone et

$R^3$ et $R^4$, conjointement avec l'atome d'azote auquel ils sont attachés, représentent un radical pipéridine, morpholine ou pyrrolidine éventuellement substitués chacun une à trois fois, de manière identique ou différente, par un alcoyle ayant 1 à 3 atomes de carbone.

6. Procédé pour combattre les parasites, caractérisé en ce qu'on fait agir des aminocétals substitués de formule (I) selon la revendication 1 sur les parasites ou sur leur espace vital.

7. Aminocétals substitués de formule (IA):

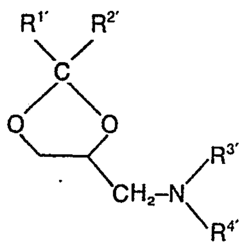 (IA)

dans laquelle

$R^{1'}$ représente un alcoyle à chaîne droite ou ramifiée ayant 6 à 18 atomes de carbone, un halogénoalcoyle à chaîne droite ou ramifiée ayant 2 à 12 atomes de carbone et 1 à 5 atomes d'halogène, un cyanalcoyle à chaîne droite ou ramifiée ayant 1 à 4 atomes de carbone dans la portion alcoyle, un benzyle substitué une ou plusieurs fois, de manière identique ou différente, un arylalcoyle éventuellement substitué une ou plusieurs fois, de manière identique ou différente et ayant 2 à 6 atomes de carbone dans la portion alcoyle et 6 à 10 atomes de carbone dans la portion aryle, un aryloxyalcoyle, un aralcoyloxyalcoyle, un arylthioalcoyle, un arylsulfinylalcoyle, un arylsulfonylalcoyle qui ont chacun 1 à 6 atomes de carbone dans chaque portion alcoyle et 6 à 10 atomes de carbone dans la portion aryle, en envisageant chaque fois comme substituants de l'aryle:
halogène, cyano, nitro; alcoyle, alcoxy, alcoylthio ayant chacun 1 à 4 atomes de carbone, halogénoalcoyle, halogénoalcoxy et halogénoalcoylthio ayant chacun 1 ou 2 atomes de carbone et 1 à 5 atomes d'halogène, cycloalcoyle ayant 5 à 7 atomes de carbone, alcoxycarbonyle ayant 1 à 4 atomes de carbone dans la portion alcoyle et un phényle ou phénoxy éventuellement substitués par de l'halogène, en particulier par du fluor ou du chlore, ou par un alcoyle ayant 1 à 4 atomes de carbone et un cycloalcoylalcoyle ayant 1 à 4 atomes de carbone dans la portion alcoyle et 3 à 7 atomes de carbone dans la portion cycloalcoyle éventuellement substitué une ou plusieurs fois par un alcoyle ayant 1 à 4 atomes de carbone; en outre un alcényle à chaîne droite ou ramifiée ayant 2 à 5 atomes de carbone, un alcinyle à chaîne droite ou ramifiée ayant 2 à 6 atomes de carbone; un cycloalcoyle ayant 3 à 7 atomes de carbone éventuellement substitué une à plusieurs fois, de manière identique ou différente, par un alcoyle ayant 1 à 4 atomes de carbone et un arylalcényle ayant 2 à 6 atomes de carbone dans la portion alcényle et 6 à 10 atomes de carbone dans la portion aryle, en envisageant chaque fois comme substituants de l'aryle:
halogène, cyano, nitro, alcoyle, alcoxy, alcoylthio ayant chaque fois 1 à 4 atomes de carbone; halogénoalcoxy et halogénoalcoylthio ayant chacun 1 ou 2 atomes de carbone et 1 à 5 atomes d'halogène, cycloalcoyle ayant 5 à 7 atomes de carbone, alcoxycarbonyle ayant 1 à 4 atomes de carbone dans la portion alcoyle et un phényle ou phénoxy éventuellement substitués par de l'halogène, en particulier par du fluor ou du chlore, ou par un alcoyle ayant 1 à 4 atomes de carbone;

$R^{2'}$ représente de l'hydrogène, un alcoyle à chaîne droite ou ramifiée ayant 1 à 18 atomes de carbone; un halogénoalcoyle à chaîne droite ou ramifiée ayant 1 à 12 atomes de carbone et 1 à 5 atomes d'halogène, un cyanalcoyle à chaîne droite ou ramifiée ayant 1 à 4 atomes de carbone dans la portion alcoyle, un arylalcoyle, aryloxyalcoyle, un aralcoyloxyalcoyle, un arylthioalcoyle, un arylsulfinylalcoyle ou un arylsulfonylalcoyle éventuellement substitués chacun une ou plusieurs fois, de manière identique ou différente et ayant chacun 1 à 6 atomes de carbone dans chaque portion alcoyle et 6 à 10 atomes de carbone dans la portion aryle, en envisageant chaque fois comme substituants de l'aryle:
halogène, cyano, nitro; alcoyle, alcoxy, alcoylthio ayant chacun 1 à 4 atomes carbone; halogénoalcoyle, halogénoalcoxy et halogénoalcoylthio ayant chacun 1 ou 2 atomes de carbone et 1 à 5 atomes d'halogène, cycloalcoyle ayant 5 à 7 atomes de carbone, alcoxycarbonyle ayant 1 à 4 atomes de carbone dans la portion alcoyle, et un phényle ou phénoxy éventuellement substitués par de l'halogène, en particulier par du fluor ou du chlore, ou par un alcoyle ayant 1 à 4 atomes de carbone; et un cycloalcoylalcoyle ayant 1 à 4 atomes de carbone dans la portion alcoyle et 3 à 7 atomes de carbone dans la portion cycloalcoyle qui éventuellement est substituée une ou plusieurs fois par un alcoyle ayant 1 à 4 atomes de carbone, en outre un alcényle ou alcinyle à chaîne

droite ou ramifiée ayant chacun 2 à 6 atomes de carbone, un cycloalcoyle éventuellement substitué une ou plusieurs fois, de manière identique ou différente par un alcoyle ayant 1 à 4 atomes de carbone et qui possède 3 à 7 atomes de carbone, ou un aryle ayant 6 à 10 atomes de carbone, de même qu'un arylalcényle ayant 2 à 6 atomes de carbone dans la portion alcényle et 6 à 10 atomes de carbone dans la portion aryle, qui sont éventuellement substitués chacun une à plusieurs fois, de manière identique ou différente, en envisageant chaque fois comme substituants de l'aryle: halogène, cyano, nitro; alcoyle, alcoxy, alcoylthio ayant chacun 1 à 4 atomes de carbone; halogénoalcoyle, halogénoalcoxy et halogénoalcoylthio ayant chacun 1 à 2 atomes de carbone et 1 à 5 atomes d'halogène, cycloalcoyle ayant 5 à 7 atomes de carbone, alcoxycarbonyle ayant 1 à 4 atomes de carbone dans la portion alcoyle et un phényle ou phénoxy éventuellement substitués par de l'halogène, en particulier par du fluor ou du chlore, ou par un alcoyle ayant 1 à 4 atomes de carbone;

$R^{3'}$ représente un alcoyle à chaîne droite ou ramifiée ayant 1 à 12 atomes de carbone, un alcényle à chaîne droite ou ramifiée ayant 3 à 12 atomes de carbone, un alcinyle à chaîne droite ou ramifiée ayant 3 à 7 atomes de carbone, un cycloalcoyle éventuellement substitué une ou plusieurs fois, de manière identique ou différente par un alcoyle ayant 1 à 4 atomes de carbone et qui possède 5 à 7 atomes de carbone, de même qu'un aryle ayant 6 à 10 atomes de carbone ou aralcoyle ayant 1 ou 2 atomes de carbone dans la portion alcoyle et 6 à 10 atomes de carbone dans la portion aryle, qui sont éventuellement substitués chacun une ou plusieurs fois, de manière identique ou différente, en envisageant chaque fois comme substituants de l'aryle:

halogène, alcoyle ayant 1 à 4 atomes de carbone de même qu'halogènoalcoyle ayant 1 ou 2 atomes de carbone et 1 à 5 atomes d'halogène;

$R^{4'}$ représente un alcoyle à chaîne droite ou ramifiée ayant 1 à 12 atomes de carbone, un alcényle à chaîne droite ou ramifiée ayant 3 à 12 atomes de carbone, un alcinyle à chaîne droite ou ramifiée ayant 3 à 7 atomes de carbone, un cycloalcoyle éventuellement substitué une ou plusieurs fois, de manière identique ou différente par un alcoyle ayant 1 à 4 atomes de carbone et qui possède 5 à 7 atomes de carbone, de même qu'un aryle ayant 6 à 10 atomes de carbone ou aralcoyle ayant 1 à 2 atomes de carbone dans la portion alcoyle et 6 à 10 atomes de carbone dans la portion aryle et qui sont chacun substitués éventuellement une ou plusieurs fois, de manière identique ou différente, en envisageant chaque fois comme substituants de l'aryle:

halogène, alcoyle ayant 1 à 4 atomes de carbone de même que halogénoalcoyle ayant 1 ou 2 atomes de carbone et 1 à 5 atomes d'halogène; ou bien

$R^{3'}$ et $R^{4'}$, conjointement avec l'atome d'azote auquel ils sont attachés, représentent un hétérocycle saturé ou insaturé à 5 à 7 chaînons, éventuellement substitué une ou plusieurs fois, de manière identique ou différente et qui contient 1 à 3 atomes d'azote et/ou d'oxygène, en citant comme substituants:

alcoyle ayant 1 à 4 atomes de carbone, alcoxycarbonyle ayant 1 à 4 atomes de carbone dans la portion alcoyle et phényle, à l'exception des composés dans lesquels

$R^{1'}$ représente de l'éthylbutylcarbinol (3-heptyle), $R^{2'}$ un phényle et $R^{3'}$ et $R^{4'}$ un éthyle.

$R^{1'}$ représente de methyle, $R^{2'}$ n–$C_9H_{19}$– et $R^{3'}$ et $R^{4'}$ un éthyle.

8. Aminocétals substitués selon la revendication 7, dans lesquels

$R^{1'}$ représente un alcoyle à chaîne droite ou ramifiée ayant 6 à 12 atomes de carbone, un halogénoalcoyle à chaîne droite ou ramifiée ayant 2 à 6 atomes de carbone et 1 à 5 atomes d'halogéne, un cyanométhyle ou cyanoéthyle, un benzyle substitué une à trois fois, de manière identique ou différente, un phénylalcoyle ayant 2 à 6 atomes de carbone dans la portion alcoyle, qui est éventuellement substitué une à trois fois, de manière identique ou différente, un phényloxyalcoyle, un phénylalcoyloxyalcoyle, un phénylthioalcoyle, un phénylsulfinylalcoyle ou un phénylsulfonylalcoyle ayant chacun 1 à 4 atomes de carbone dans chaque portion alcoyle, en envisageant comme substituants du phényle ou du benzyle:

fluor, chlore, brome, cyano, nitro, méthyle, méthoxy, méthylthio, éthyle, éthoxy, éthylthio, n- et i-propyle, isopropoxy, n-, iso-, sec- et t-butyle, trifluorométhyle, trifluorométhoxy, trifluorométhlylthio, cyclohexyle, méthoxycarbonyle, éthoxycarbonyle et phényle ou phénoxy éventuellement substitués par du fluor, du chlore ou méthyle;

en outre un cycloalcoylalcoyle ayant 1 à 4 atomes de carbone dans la portion alcoyle et 5 à 7 atomes de carbone dans la portion cycloalcoyle et qui est éventuellement substitué une à plusieurs fois par un alcoyle ayant 1 à 4 atomes de carbone; en outre un alcényle à chaîne droite ou ramifiée ayant 3 à 5 atomes de carbone, un alcinyle à chaîne droite ou ramifiée ayant 3 à 6 atomes de carbone; un cycloalcoyle ayant 5 à 7 atomes de carbone et substitué une à trois fois, de manière identique ou différente, par un méthyle, éthyle ou propyle, et un phénylalcényle ayant 2 à 5 atomes de carbone dans la portion alcényle, en envisageant chaque fois comme substituants du phényle:

fluor, chlore, brome, cyano, nitro, méthyle, méthoxy, méthylthio, éthyle, éthoxy, éthylthio, n- et i-propyle, isopropoxy, n-, iso-, sec- et t-butyle, trifluorométhyle, trifluorométhoxy, trifluorométhylthio, cyclohexyle, méthoxycarbonyle, éthoxycarbonyle et un phényle ou phénoxy éventuellement substitués par du fluor, du chlore ou méthyle;

$R^{2'}$ de l'hydrogène, un alcoyle à chaîne droite ou ramifiée ayant 1 à 10 atomes de carbone, un phényle ou benzyle éventuellement substitués une à trois fois, de manière identique ou différente, en envisageant comme substituants du phényle ou benzyle:

fluor, chlore, brome, cyano,nitro, trifluorométhyle, trifluorométhoxy, trifluorométhylthio, méthoxycarbonyle et éthoxycarbonyle,

$R^{3'}$ un méthyle, éthyle, n- et i-propyle, n-, i-, sec- et t-butyle, n-pentyle, n-hexyle, n-heptyle, n-octyle, n-nonyle, n-décyle, n-dodécyle, allyle, propargyle, 2-buténylе, cyclopentyle, cyclohexyle de même qu'un phényle ou benzyle éventuellement substitués chacun une à deux fois, de manière identique ou différente, par du fluor, du chlore, un méthyle ou trifluorométhyle et

$R^{4'}$ un n- et i-propyle, n-, i-, sec- et t-butyle, n-pentyle, n-hexyle, n-heptyle, n-octyle, n-nonyle, n-décyle, allyle, propargyle, 2-buténylе, cyclopentyle, cyclohexyle, de même qu'un phényle ou benzyle éventuellement substitués chacun une à deux fois, de manière identique ou différente, par du fluor, du chlore, un méthyle ou un trifluorométhyle, ou bien

$R^{3'}$ et $R^{4'}$, conjointement avec l'atome d'azote auquel ils sont attachés, représentent un 1-pyrrolidinyle, 1-pipéridinyle, 1-pipérazinyle, 4-morpholinyle, 1-perhydroazépinyle, 1-imidazolyle, 1-(1,2,4-triazolyle) ou 1-pyrazolyle éventuellement substitués chacun une à trois fois, de manière identique ou différente, en citant comme substituants:
méthyle, éthyle, n- et i-propyle, phényle, méthoxycarbonyle et éthoxycarbonyle.

9. Aminocétals substitués selon la revendication 7, dans lesquels

$R^{1'}$ représente les groupements

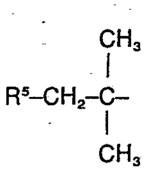 , 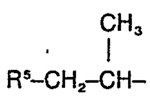 , 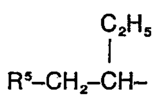 ,

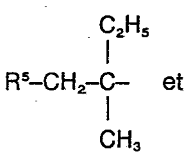 et 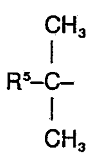

$R^5$ un phényle, phénoxy, phénylthio, phénylsulfinyle ou phénylsulfonyle éventuellement substitués une à trois fois, de manière identique ou différente, en citant comme substituants:
fluor, chlore, brome, nitro, cyclohexyle, alcoyle à chaîne droite ou ramifiée ayant 1 à 4 atomes de carbone, alcoxy à chaîne droite ou ramifiée ayant 1 à 3 atomes de carbone et alcoxycarbonyle ayant 1 ou 2 atomes de carbone dans la portion alcoxy,

$R^{2'}$ un alcoyle à chaîne droite ou ramifiée ayant 1 à 4 atomes de carbone,

$R^{3'}$ un alcoyle à chaîne droite ou ramifiée ayant 1 à 4 atomes de carbone ou un alcényle à chaîne droite ou ramifiée ayant 3 à 5 atomes de carbone,

$R^{4'}$ un alcoyle à chaîne droite ou ramifiée ayant 1 à 4 atomes de carbone et

$R^{3'}$ et $R^{4'}$, conjointement avec l'atome d'azote auquel ils sont attachés, représentent un radical pipéridine, morpholine ou pyrrolidine éventuellement substitués chacun une à trois fois, de manière identique ou différente, par un alcoyle ayant 1 à 3 atomes de carbone.

10. Aminocétals substitués selon la revendication 7, dans lesquels

$R^{1'}$ représente les groupements

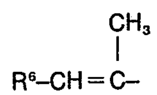 et 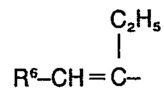

$R^6$ représentant un phényle éventuellement substitué chaque fois une à trois fois, de manière identique ou différente, en citant comme substituants:
fluor, chlore, brome, nitro, cyclohexyle, alcoyle ou alcoxy à chaîne droite ou ramifiée ayant chacun 1 à 4 atomes de carbone et alcoxycarbonyle ayant 1 ou 2 atomes de carbone dans la portion alcoxy,

$R^{2'}$ un alcoyle à chaîne droite ou ramifiée ayant 1 à 4 atomes de carbone,

$R^{3'}$ un alcoyle à chaîne droite ou ramifiée ayant 1 à 4 atomes de carbone ou un alcényle à chaîne droite ou ramifiée ayant 3 à 5 atomes de carbone,

$R^{4'}$ un alcoyle à chaîne droite ou ramifiée ayant 1 à 4 atomes de carbone et

$R^{3'}$ et $R^{4'}$, conjointement avec l'atome d'azote auquel ils sont attachés, représentant un radical pipéridine, morpholine ou pyrrolidine éventuellement substitués chacun une à trois fois, de manière identique ou différente, par un alcoyle ayant 1 à 3 atomes de carbone.

11. Agents pour combattre les parasites, caractérisés par une teneur en au moins un aminocétal substitué selon la revendication 7.

12. Procédé de fabrication d'agents pour combattre les parasites, caractérisé en ce qu'on mélange au moins un aminocétal substitué selon la revendication 7 avec des diluants et/ou des agents tensioactifs.